(19) Europäisches Patentamt / European Patent Office / Office européen des brevets

(11) **EP 2 773 704 B1**

(12) **EUROPÄISCHE PATENTSCHRIFT**

(45) Veröffentlichungstag und Bekanntmachung des Hinweises auf die Patenterteilung:
**24.06.2020   Patentblatt 2020/26**

(51) Int Cl.:
*C09C 1/64* *(2006.01)*       *C09D 5/36* *(2006.01)*
*C08K 9/02* *(2006.01)*       *A61Q 1/02* *(2006.01)*
*A61K 8/26* *(2006.01)*       *A61Q 1/06* *(2006.01)*
*A61Q 1/10* *(2006.01)*       *A61Q 3/02* *(2006.01)*
*A61Q 5/06* *(2006.01)*       *A61K 8/02* *(2006.01)*
*C09D 5/03* *(2006.01)*       *C08L 27/16* *(2006.01)*

(21) Anmeldenummer: **12783964.5**

(22) Anmeldetag: **02.11.2012**

(86) Internationale Anmeldenummer:
**PCT/EP2012/071735**

(87) Internationale Veröffentlichungsnummer:
**WO 2013/064643 (10.05.2013 Gazette 2013/19)**

(54) **BESCHICHTETE, NASSCHEMISCH OXIDIERTE ALUMINIUMEFFEKTPIGMENTE, VERFAHREN ZU DEREN HERSTELLUNG, BESCHICHTUNGSMITTEL UND BESCHICHTETER GEGENSTAND**

COATED, WET-CHEMICALLY OXIDIZED ALUMINIUM EFFECT PIGMENTS, METHOD FOR THE PRODUCTION THEREOF, COATING AGENT AND COATED OBJECT

PIGMENTS À EFFET ENROBÉS À BASE D'ALUMINIUM OXYDÉ PAR VOIE CHIMIQUE HUMIDE, PROCÉDÉ DE PRODUCTION DESDITS PIGMENTS, AGENT D'ENROBAGE ET OBJET ENROBÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.11.2011   DE 102011055072**

(43) Veröffentlichungstag der Anmeldung:
**10.09.2014   Patentblatt 2014/37**

(73) Patentinhaber: **Eckart GmbH**
**91235 Hartenstein (DE)**

(72) Erfinder:
• **SCHUMACHER, Dirk**
**91257 Pegnitz (DE)**
• **GRÜNER, Michael**
**91275 Auerbach (DE)**
• **HÖFENER, Sebastian**
**90482 Nürnberg (DE)**
• **STRUCK, Oliver**
**91239 Henfenfeld (DE)**

(74) Vertreter: **Louis Pöhlau Lohrentz**
**Patentanwälte**
**Postfach 30 55**
**90014 Nürnberg (DE)**

(56) Entgegenhaltungen:
WO-A1-2006/041658       DE-A1- 3 334 598
US-A1- 2003 209 169     US-A1- 2007 104 663
US-A1- 2008 115 693     US-A1- 2009 264 575

EP 2 773 704 B1

**Beschreibung**

Die Erfindung betrifft farbige oxidierte Aluminiumeffektpigmente, ein Verfahren zu ihrer Herstellung sowie deren Verwendung. Die Erfindung betrifft weiterhin ein Beschichtungsmittel sowie einen beschichteten Gegenstand.

Aluminiumeffektpigmente bieten eine große Anzahl an Einsatzmöglichkeiten und sind in vielfältigen Einsatzgebieten etabliert. Vielfach anzutreffen sind sie beispielsweise bei Kosmetika, im Automobilsektor, bei der Kunststoffherstellung, bei Anstrichmitteln oder der Druckindustrie. Hierbei bieten sie neben optischen auch beispielsweise spezielle technische Eigenschaften. Aluminiumeffektpigmente zeigen durch ihre plättchenförmige Ausformung interessante optische Effekte, was auf deren größere Reflexionsfläche zurückzuführen ist. Neben der Grundform der Effektpigmente an sich wurden in der Vergangenheit ferner verschiedene Oberflächenmodifikationen entwickelt, um neue Eigenschaften zu kreieren oder die bestehenden Eigenschaften gezielt zu beeinflussen.

Beispielsweise beschreibt die US 5,964,936 A eine kontrollierte nasschemische Oxidation der Oberfläche von Aluminiumeffektpigmenten, welche von der Firma Eckart unter dem Namen Aloxal vertrieben werden. Hierbei bildet sich eine charakteristische Oberflächenstruktur, die infolge von Interferenzeffekten eine Färbung der Effektpigmente hervorruft. Zwar bieten die beispielsweise hiermit erzielbaren Champagnerfarbtöne ein hochinteressantes Farbspektrum für viele Anwendungen, jedoch kann das in der Aluminiumoxidschicht enthaltene Wasser eine nachträgliche Oxidation hervorrufen, so dass eine Langzeitfarbstabilität nur schwer zu erzielen ist.

Die US 5,931,996 A beschreibt ein Verfahren zur Herstellung farbiger Aluminiumeffektpigmente, wobei Farbpigmente in eine Metalloxidbeschichtung der Aluminiumeffektpigmente eingebracht werden.

Ferner beschreibt die US 2007/0104663 A1 die Beschichtung von nasschemisch oxidierten Aluminiumpartikeln mit einer Metallchalkogenidschicht. Hierbei erhaltene Partikel weisen eine minimale Gesamtdicke, festhaftende Oxidschichten und sicherheitstechnische Unbedenklichkeit auf.

Zudem wird in der US 2008/0249209 A1 die Herstellung von anorganisch/organischen Mischschichten mit einem anorganischen Netzwerk und wenigstens einer organischen Komponente beschrieben. Es hat sich herausgestellt, dass die Herstellung derartiger anorganisch/organischer Mischschichten prozesstechnisch aufwendig ist.

Aus der US 2009/0264575 A1 sind Metalleffektpigmente mit einer Beschichtung bekannt, welche chemisch vernetzbare und/oder unter Einwirkung von Wärme, IR-Strahlung, UV-Strahlung und/oder Elektronenstrahlen vernetzbare oligomere und/oder polymere Bindemittel enthält.

Die US 6,761,762 B1 offenbart mit reaktiven Orientierungshilfsmitteln beschichtete Effektpigmente. Gegenstand dieses Patentes sind im wesentlichen Aluminiumeffektpigmente, die mit einer Beschichtung aus Metalloxiden oder Polymeren beschichtet sein können. Auf dieser Beschichtung sind sodann Orientierungshilfsmittel angeordnet, die eine kovalente Anbindung an das Bindemittel einer Farbe oder eines Lackes ermöglichen.

Schließlich sind aus der US 5,332,767 A kunstharzbeschichtete Metalleffektpigmente, insbesondere Aluminiumeffektpigmente, bekannt.

In der US 2009/0117281 A1 werden Aluminiumeffektpigmente, umfassend eine Haftvermittlungsschicht und eine Polymerschicht, beschrieben. Ziel hierbei soll eine verbesserte Wasserstabilität und Chemikalienstabilität der Pigmente sein.

Ferner werden in der US 2010/0152355 A1 Metalleffektpigmente beschrieben, die mit einer Kunstharzbeschichtung versehen sind, wobei die Kunstharzbeschichtung ein Polymer und ein organofunktionelles Silan umfasst.

Keines der vorgenannten Dokumente betrifft das Problem einer Nachoxidation bei den nasschemisch oxidierten Aluminiumpigmenten. Der Begriff "Nachoxidation" bezeichnet dabei vorliegend einen der nasschemischen Oxidation nachfolgenden unkontrollierten Oxidationsprozess, der vermutlich auf das auf oder in der nasschemisch erzeugten Aluminiumoxidschicht gebundene Wasser zurückzuführen ist.

Es hat sich ferner gezeigt, dass bei Verwendung von nasschemisch oxidierten Aluminiumeffektpigmente insbesondere in Autolack-Formulierungen beim Einbrennen der entsprechenden Lackbeschichtungen Blasen oder ähnliche Unregelmäßigkeiten, sogenannte Kochblasen oder auch Kocher, auftreten können, wodurch die optischen und/oder funktionalen Eigenschaften des Lacks drastisch beeinträchtigt werden. Ein ähnlicher nachteiliger Effekt tritt auch bei der Verwendung von nasschemisch oxidierten Aluminiumeffektpigmenten in Kunststoffmaterialien auf. So wurde beispielsweise bei der Extrusion von Thermoplasten, die nasschemisch oxidierte Aluminiumeffektpigmente enthalten, eine Trübung beobachtet, die die optischen Eigenschaften des Kunststoffs verschlechtert. Es wird vermutet, dass auch diese Trübung auf das Entstehen von Gasblasen in dem Kunststoff zurückzuführen ist.

Insbesondere geben die vorgenannten Dokumente keine Hinweise, welche Maßnahmen ergriffen werden müssten, damit die optischen Eigenschaften von nasschemisch oxidierten Aluminiumpigmenten über die Zeit keinen Veränderungen unterliegen.

US 2003/0209169 A1 betrifft Mehrschichtpigmente, die auf beschichteten Metallplättchen basieren.

Aufgabe der Erfindung ist es mithin, nasschemisch oxidierte Aluminiumeffektpigmente bereitzustellen, deren optische Eigenschaften sich über die Zeit nicht ändern, die insbesondere eine Farbstabilität aufweisen, eine einfache und vielseitige Applikation ermöglichen und gleichzeitig keine merklichen Einbußen bezüglich der optischen Qualität aufweisen.

Ferner ist es eine Aufgabe der vorliegenden Erfindung, ein Verfahren zur Herstellung derartiger Pigmente bereitzustellen.

Ferner ist es eine Aufgabe der vorliegenden Erfindung, Verwendungen bereitzustellen, bei denen die erfindungsgemäßen Pigmente besonders vorteilhaft eingesetzt werden können.

Die der Erfindung zugrunde liegende Aufgabe wird durch die Bereitstellung von nasschemisch oxidierten Aluminiumeffektpigmenten gemäß Anspruch 1 gelöst.

Bei weiteren Ausführungsformen der vorgenannten Pigmente liegt die Summe der Gehalte der wenigstens einen Metalloxidschicht und der wenigstens einen organischen Polymerschicht in einem Bereich von 10 bis 50 Gew.-%, bezogen auf das Gewicht des unbeschichteten, nasschemisch oxidierten Aluminiumeffektpigments, und das Gewichtsverhältnis der wenigstens einen von Aluminiumoxid verschiedenen Metalloxidschicht zur wenigstens einen

Polymerschicht liegt in einem Bereich von 1:2 bis 1:20. Insbesondere ist es bei bevorzugten der vorgenannten Ausführungsformen bevorzugt, dass die Summe der Gehalte der wenigstens einen Metalloxidschicht und der wenigstens einen organischen Polymerschicht in einem Bereich von 13 bis 40 Gew.-%, bezogen auf das Gewicht des unbeschichteten, nasschemisch oxidierten Aluminiumeffektpigments, und das Gewichtsverhältnis der wenigstens einen von Aluminiumoxid verschiedenen Metalloxidschicht zur wenigstens einen Polymerschicht in einem Bereich von 1:2,2 bis 1:17 liegt.

Bei weiteren Ausführungsformen der vorgenannten Pigmente beträgt der Gehalt an elementarem Aluminium höchstens 87 Gew.-%, bezogen auf das Gewicht der unbeschichteten, nasschemisch oxidierten Aluminiumeffektpigmente.

Bei weiteren Ausführungsformen der vorgenannten Pigmente beträgt der Gewichtsanteil der wenigstens einen von Aluminiumoxid verschiedenen Metalloxidschicht mindestens 0,8 Gew.-%, bezogen auf das Gewicht der unbeschichteten, nasschemisch oxidierten Aluminiumeffektpigmente. Ferner ist es bei weiteren Ausführungsformen der vorgenannten Pigmente bevorzugt, dass der Gewichtsanteil der wenigstens einen von Aluminiumoxid verschiedenen Metalloxidschicht höchstens 20 Gew.-%, bezogen auf das Gewicht der unbeschichteten, nasschemisch oxidierten Aluminiumeffektpigmente beträgt. Bei weiteren der vorgenannten Ausführungsformen der vorgenannten Pigmente liegt der Gewichtsanteil der wenigstens einen von Aluminiumoxid verschiedenen Metalloxidschicht in einem Bereich von 0,8 bis 20 Gew.-%, vorzugsweise in einem Bereich von 1,6 bis 16 Gew.-% und noch mehr bevorzugt in einem Bereich von 2,1 bis 14 Gew.-%, bezogen auf das Gewicht der unbeschichteten, nasschemisch oxidierten Aluminiumeffektpigmente.

Bei weiteren Ausführungsformen der vorgenannten Pigmente besteht die wenigstens eine von Aluminiumoxid verschiedene Metalloxidschicht im Wesentlichen aus Metalloxid, das ausgewählt wird aus der Gruppe bestehend aus Siliziumoxid, Boroxid, Zirkoniumoxid, Ceroxid, Eisenoxid, Titanoxid, Chromoxid, Zinnoxid, Molybdänoxid, deren Oxidhydraten, deren Hydroxiden sowie Mischungen davon. Insbesondere besteht die wenigstens eine von Aluminiumoxid verschiedene Metalloxidschicht der vorgenannten Pigmente im Wesentlichen aus Siliziumoxid, dessen Hydroxiden oder Mischungen davon.

Bei weiteren Ausführungsformen der vorgenannten Pigmente beträgt der Gewichtsanteil der wenigstens einen organischen Polymerschicht mindestens 8 Gew.-%, bezogen auf das Gewicht des unbeschichteten, nasschemisch oxidierten Aluminiumeffektpigments. Bei weiteren Ausführungsformen der vorgenannten Pigmente beträgt der Gewichtsanteil der wenigstens einen organischen Polymerschicht höchstens 40 Gew.-%, bezogen auf das Gewicht des unbeschichteten, nasschemisch oxidierten Aluminiumeffektpigments. Bei weiteren der vorgenannten Ausführungsformen liegt der Gewichtsanteil der wenigstens einen organischen Polymerschicht in einem Bereich von 8 bis 40 Gew.-%, bezogen auf das Gewicht des unbeschichteten, nasschemisch oxidierten Aluminiumeffektpigments.

Bei weiteren Ausführungsformen der vorgenannten Pigmente wird das wenigstens eine organische Polymer der wenigstens einen organischen Polymerschicht ausgewählt aus der Gruppe bestehend aus Polyacrylat, Polymethacrylat, Polyacrylamid, Polyacrylnitril, Polyvinylchlorid, Polyvinylacetat, Polyamid, Polyalken, Polydien, Polyalkin, Polyalkylenglykol, Epoxidharz, Polyester, Polyether, Polyol, Polyurethan, Polycarbonat, Polyethylenterephthalat und Mischungen davon, insbesondere aus der Gruppe bestehend aus Polyacrylat, Polymethacrylat und Mischungen davon.

Bei weiteren Ausführungsformen der vorgenannten Pigmente ist die wenigstens eine von Aluminiumoxid verschiedene Metalloxidschicht keine anorganisch/organische Mischschicht.

Bei weiteren Ausführungsformen der vorgenannten Pigmente weist das nichtoxidierte Aluminium der erfindungsgemäßen Aluminiumeffektpigmente eine Reinheit von mindestens 99,5 Gew.-% auf. Bei anderen Ausführungsformen der vorliegenden Erfindung besteht das nichtoxidierte Aluminium der erfindungsgemäßen Aluminiumeffektpigmente hingegen aus einer Aluminiumlegierung, die neben Aluminium mindestens 5 Gew.-% andere Metalle enthält. Hierbei werden die anderen Metalle vorzugsweise ausgewählt aus der Gruppe bestehend aus Eisen, Mangan, Kupfer, Vanadium, Chrom, Nickel, Cobalt, Silizium, Magnesium, Zink und Titan.

Bei weiteren Ausführungsformen weisen die vorgenannten Pigmente zusätzlich zur wenigstens einen von Aluminiumoxid verschiedenen Metalloxidschicht eine hochbrechende Metallchalkogenidschicht, vorzugsweise eine hochbrechende Metalloxidschicht wie beispielsweise Eisenoxid, auf.

Ferner betrifft die vorliegende Erfindung die Bereitstellung eines Verfahrens zur Herstellung von beschichteten naßchemisch oxidierten Aluminiumeffektpigmenten gemäß Anspruch 11.

Insbesondere ist es bei weiteren Ausführungsformen bevorzugt, dass bei Schritt 2 das Metalloxid mit einem Sol-Gel-Prozess aufgebracht wird.

Bei weiteren Ausführungsformen ist es bevorzugt, dass das nasschemische Oxidieren in Schritt 1 bei pH 7 bis 12 mit einem Gemisch von Wasser und einem oder mehreren wassermischbaren Lösungsmitteln durchgeführt wird. Beispiele derartiger wassermischbarer Lösungsmittel sind Ethanol, n-Propanol, i-Propanol, n-Butanol, i-Butanol, Methoxypropanol, Aceton und Butylglykol.

Ferner betrifft die vorliegende Erfindung Pigmente, die nach den erfindungsgemäßen Verfahren hergestellt wurden.

Ferner betrifft die vorliegende Erfindung die Verwendung der erfindungsgemäßen Pigmente in Kunststoff, Kometik oder in einem Beschichtungsmittel, insbesondere einer Farbe, einem Lack, einem Pulverlack oder einer Druckfarbe.

Das erfindungsgemäße Pigment kann einem Medium auch zugesetzt werden, um einen spezifischen technischen Effekt zu erzielen, wobei optische Effekte nicht umfasst sind. Beispiele hierfür ist die Verwendung des erfindungsgemäßen Pigmentes in einem Kunststoff als Lasermarkierungsadditiv. Selbstverständlich kann das erfindungsgemäße Pigment auch verwendet werden, um die optischen Eigenschaften eines Mediums zu beeinflussen oder zu gestalten. Beispiele hierfür sind Pigmente, die infolge ihrer Eigenfarbe oder basierend auf Interferenzeffekten ein Farbveränderung bewirken.

Ferner betrifft die vorliegende Erfindung ein Beschichtungsmittel, das die die erfindunsgemäßen Pigmente enthält.

Zudem betrifft die vorliegende Erfindung beschichtete Gegenstände, die die erfindungsgemäßen Pigmente und/oder das erfindungsgemäße Beschichtungsmittel enthalten und/oder aufweisen.

## Abbildungen

Abbildung 1 zeigt eine REM-Analyse eines Aluminiumeffektpigments wie beispielsweise in Beispiel 2 eingesetzt vor der nasschemischen Oxidation. An der gleichmäßig ausgeformten Oberfläche sind lediglich Abriebspuren und feinste Teilchen zu finden.

Abbildung 2 zeigt eine REM-Analyse der Oberfläche eines nasschemisch oxidierten Aluminiumeffektpigmentes des gleichen Typs wie in Abbildung 1 bei identischer Vergrößerung. Die gleichförmig ausgebildeten Oberflächenstrukturen sind gut zu erkennen.

Abbildung 3 zeigt eine REM-Analyse weiterer Aluminiumeffektpigmente vor der nasschemischen Oxidation.

Abbildung 4 zeigt eine REM-Analyse eines Aluminiumeffektpigments des Typs aus Abbildung 3 nach der nasschemischen Oxidation bei identischer Vergrößerung.

Abbildung 5 zeigt eine REM-Analyse weiterer Aluminiumeffektpigmente vor der nasschemischen Oxidation.

Abbildung 6 zeigt eine REM-Analyse eines Aluminiumeffektpigments des Typs aus Abbildung 5 nach der nasschemischen Oxidation bei identischer Vergrößerung.

Abbildungen 7 und 8 zeigen REM-Analysen von Querschliffen nasschemisch oxidierter Aluminiumeffektpigmente. Deutlich zu erkennen ist der nichtoxidierte Kern der Pigmente, sowie die nasschemisch erzeugte, umhüllende Aluminiumoxidschicht.

Abbildung 9 zeigt eine REM-Analyse eines Querschliffs eines nasschemisch oxidierten Aluminiumeffektpigments bei 2,5-fach höherer Vergrößerung, verglichen mit der Vergrößerung der Abbildungen 7 oder 8. Die entstandene gleichmäßig ausgebildete, poröse Oxidbeschichtung ist hierbei gut zu erkennen.

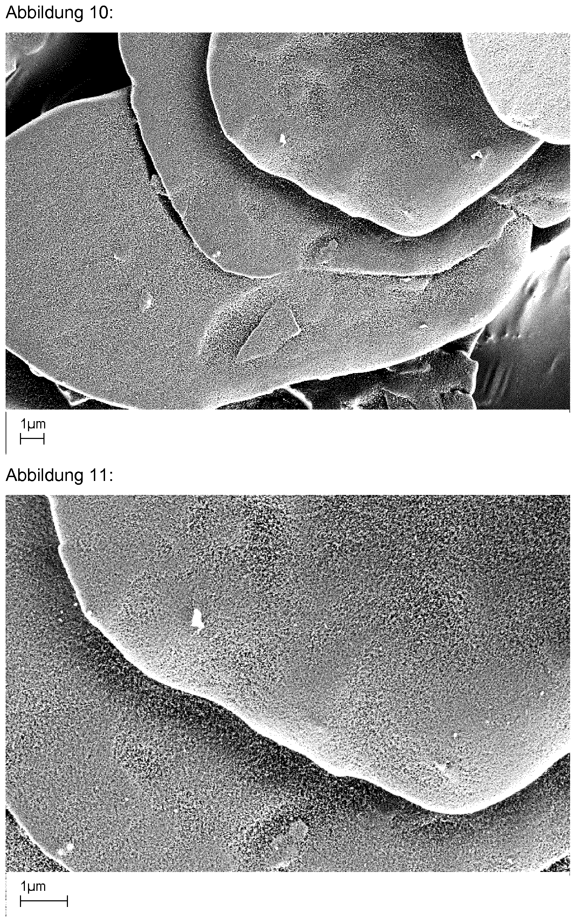

Abbildungen 10 und 11 zeigen REM-Analysen unbeschichteter nasschemisch oxidierter Aluminiumpigmente als Vergleichsbeispiel.

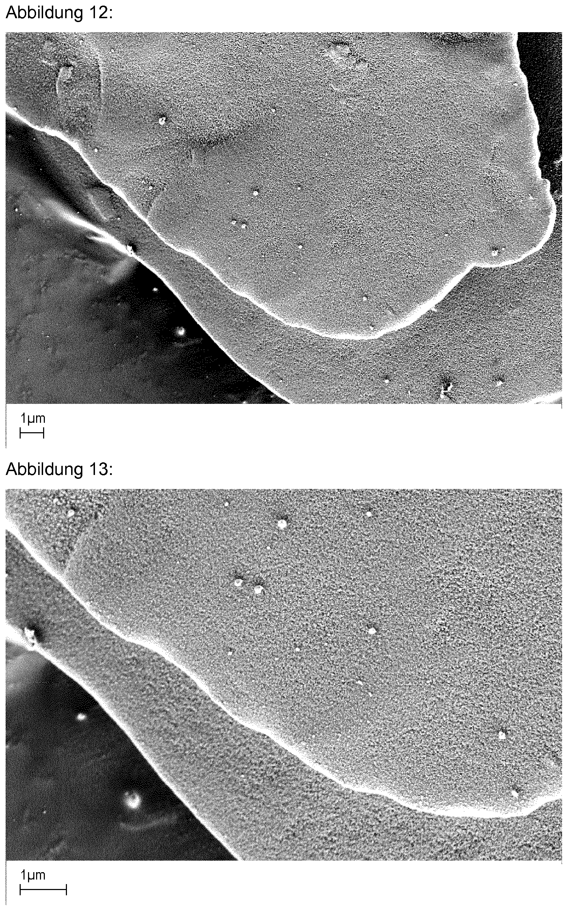

Abbildungen 12 und 13 zeigen REM-Analysen von nasschemisch oxidierten Aluminiumpigmenten, wie in den Abbildungen 10 und 11, nach der Aufbringung einer Siliziumoxidschicht.

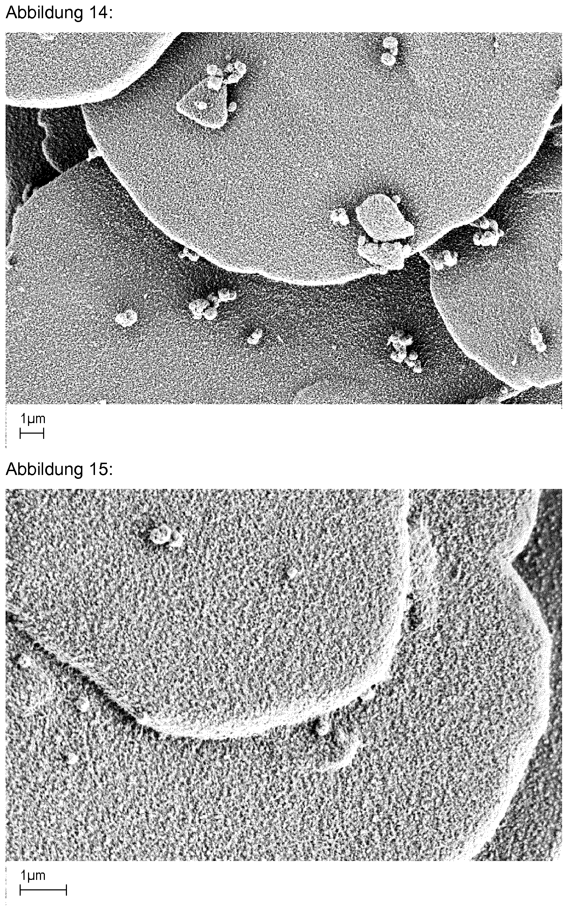

Abbildungen 14 und 15 zeigen die REM-Analysen von naßchemisch oxidierten und mit einer Siliziumoxidschicht versehenen Aluminiumpigmenten, wie in den Abbildungen 12 und 13 gezeigt, bei denen zusätzlich eine organische Polymerschicht aufgebracht ist.

Die Herstellung farbiger Aluminiumeffektpigmente durch nasschemische Oxidation von Aluminiumeffektpigmenten ist bereits aus der US 5,964,936 A bekannt. Die hierbei erhaltenen Pigmente können das vorgenannte Problem aufweisen, dass sie einer Nachoxidation unterliegen, infolge der eine Farbveränderung der betreffenden Pigmente auftritt. Eine derartige Farbveränderung ist jedoch in vielen Bereichen inakzeptabel. Beispielsweise sind in Reparaturlacksystemen der Automobilindustrie bereits geringfügige Farbänderungen inakzeptabel. Da nasschemisch oxidierte Aluminiumpigmente bei vielen Anwendungen Störungen, beispielsweise durch Bildung von Blasen in einem Beschichtungsmittel, wie einem Lack oder einer Farbe, hervorrufen, waren die nasschemisch oxidierten Aluminiumeffektpigmente in der Vergangenheit in vielen Bereichen nicht einsetzbar. Das Nachahmen der spezifischen Farbtöne und der besonderen optischen Qualitäten der nasschemisch oxidierten Aluminiumeffektpigmente war prozeßtechnisch sehr schwierig.

Überraschenderweise wurde gefunden, dass durch das Aufbringen wenigstens einer Metalloxidschicht und wenigstens einer organischen Polymerschicht dauerhaft farbstabile Aluminiumeffektpigmente prozeßtechnisch einfach hergestellt und damit neue Anwendungsgebiete für nasschemisch oxidierte Aluminiumeffektpigmente erschlossen werden können.

Ferner wurde überraschenderweise gefunden, dass besonders vorteilhafte Aluminiumeffektpigmente durch die Aufbringung wenigstens einer, vorzugsweise einer einzigen, von Aluminiumoxid verschiedenen Metalloxidschicht auf die nasschemisch oxidierten Aluminiumeffektpigmente erhalten werden. Es wird vermutet, dass die Probleme einer nachträglichen Oxidation im Wesentlichen auf das auf oder in der nasschemisch erzeugten Aluminiumoxidschicht gebundene Wasser zurückzuführen ist. So zeigten Messungen, dass beispielsweise auf den bei der nasschemischen Oxidation eingesetzten Aluminiumeffektpigmenten durch Lagerung unter Raumatmosphäre vor der nasschemischen Oxidation ca. 0,4 Gew.-% Wasser vorhanden ist. Nach der nasschemischen Oxidation findet sich auf diesen Aluminiumeffektpigmenten hingegen ein Wassergehalt von 8,1 Gew.-%., jeweils bezogen auf das Gesamtgewicht der jeweiligen Aluminiumeffektpigmente.

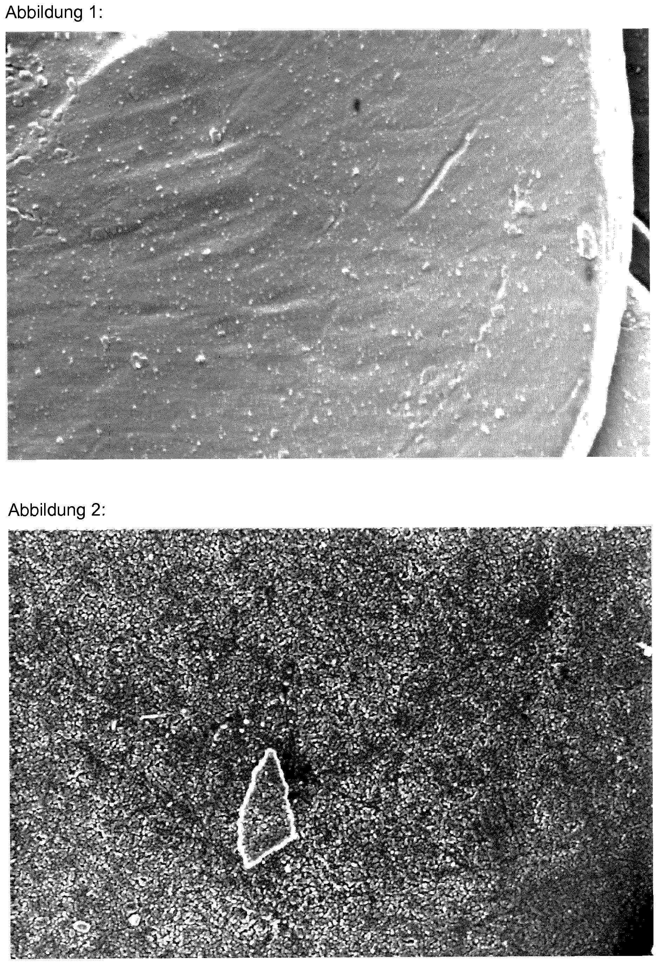

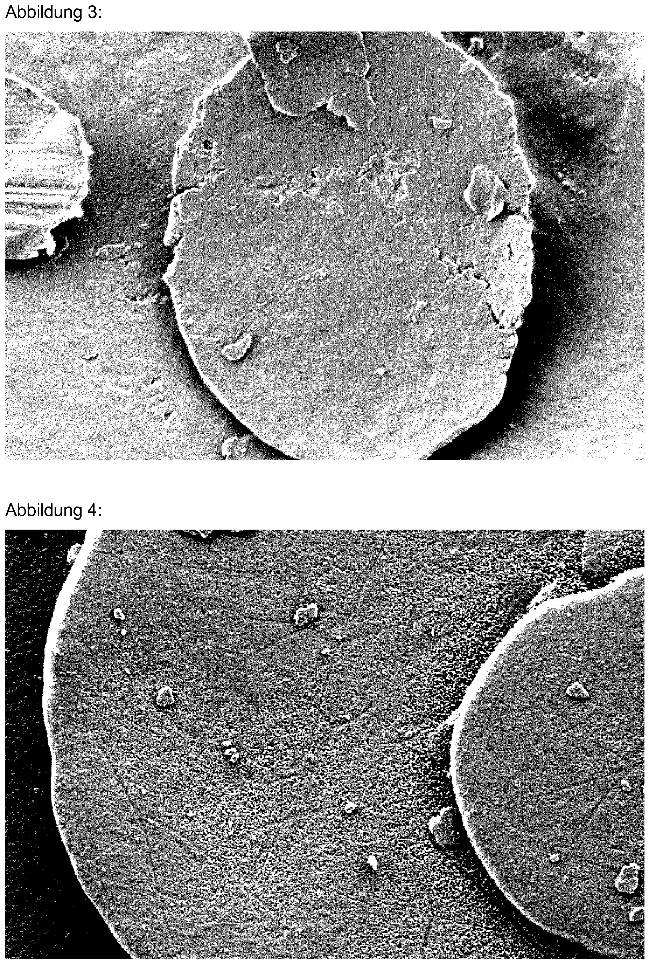

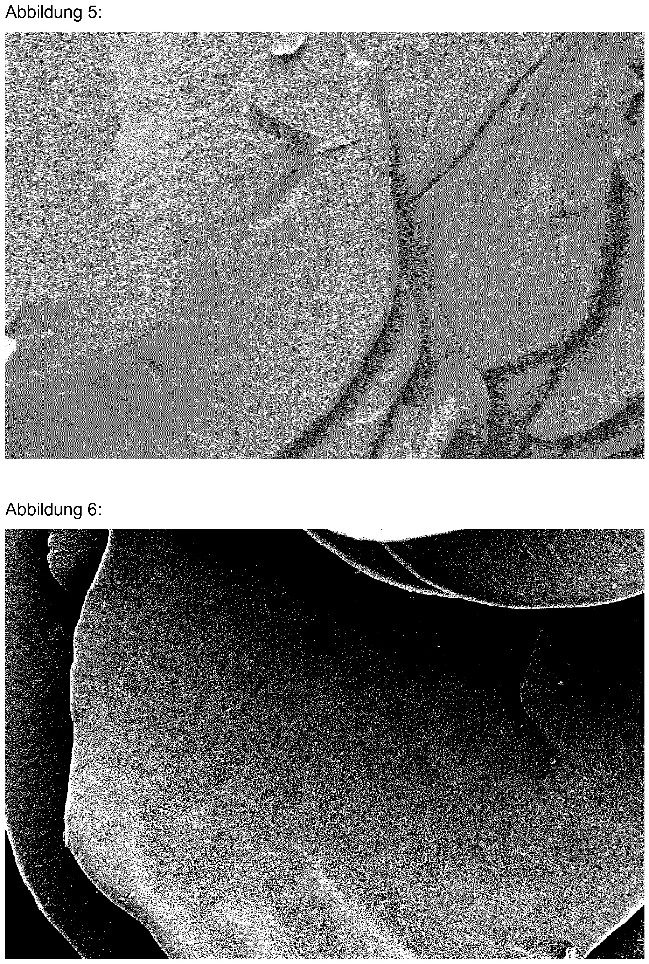

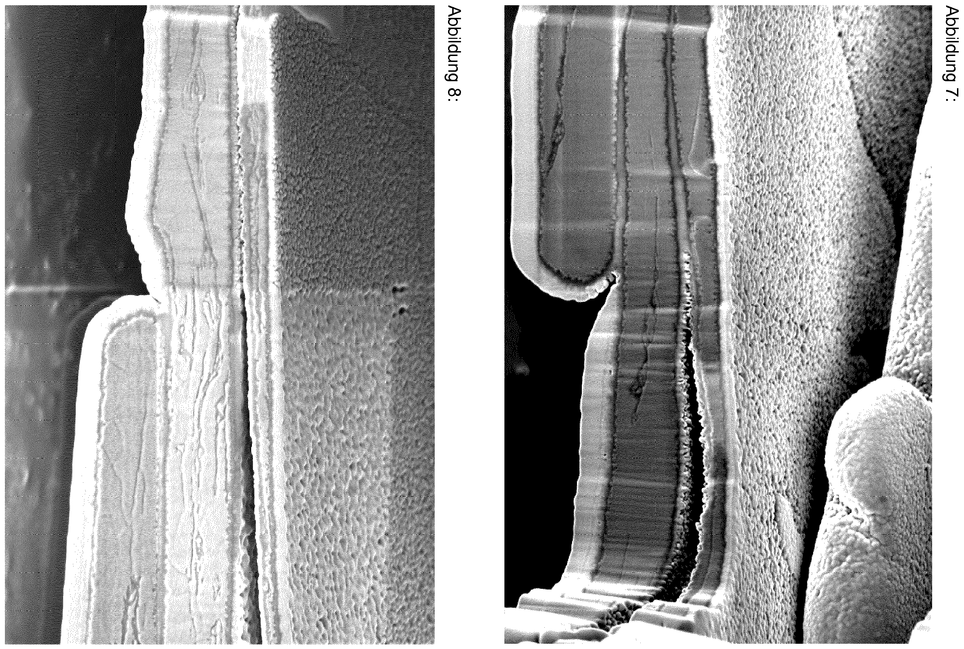

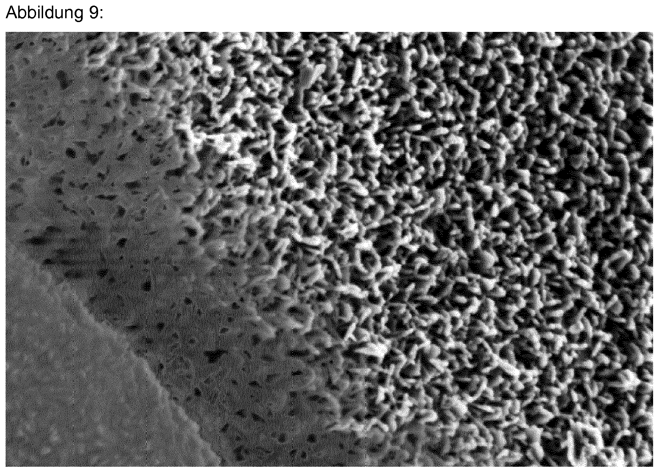

Die nasschemisch erzeugte Aluminiumoxidschicht bildet keine dichte und geschlossene Struktur, wie dies von einer natürlichen Aluminiumoxidschicht bekannt ist. Wie beispielsweise aus Abbildung 9 ersichtlich ist, bildet die nasschemisch erzeugte Aluminiumoxidschicht eine poröse Struktur, die zudem von Kanälen durchzogen ist. Dies wird auch beispielsweise durch Messungen der BET-Oberfläche belegt, wonach die BET-Oberfläche gebräuchlicher Aluminiumeffektpigmente des in Abbildung 1 dargestellten Typs infolge der nasschemischen Oxidation von 2,6 m$^2$/g auf 35,4 m$^2$/g ansteigt. Die Abbildungen 2, 4, 6, 7, 8 und 9 zeigen REM-Aufnahmen von nasschemisch oxidierten Aluminiumeffektpigmenten.

Demgemäß sollte das Aufbringen einer Metalloxidschicht oder Polymerschicht auf die Oberfläche der nasschemisch oxidierten Aluminiumeffektpigmente lediglich dazu führen, dass das enthaltene Wasser eingeschlossen wird und die vorgenannte Nachoxidation weiterhin unkontrolliert abläuft.

Ohne dass es als Einschränkung der Erfindung verstanden werden darf, vermuten die Erfinder, dass die infolge der porösen Aluminiumoxidschicht unzureichend geschützte Aluminiumoberfläche die Entstehung der nachträglich aufgebrachten, von Aluminiumoxid verschiedenen Metalloxidschicht an den Orten, an den Wasser gebunden oder eingelagert ist, begünstigt und hierdurch ein erster Schutz gegenüber einer nachfolgenden Oxidation entsteht. Ferner erwiesen sich insbesondere Beschichtungsverfahren zur Aufbringung der Metalloxidschicht als besonders vorteilhaft, bei denen die von Aluminiumoxid verschiedene Metalloxidschicht mittels einer Hydrolysereaktion aufgebracht wird, insbesondere unter Verwendung des Sol-Gel-Prozesses. Unter dem Begriff "Hydrolysereaktion" im Sinne der vorliegenden Erfindung wird verstanden, dass die Metalloxidquelle unter Verbrauchen von Wasser zersetzt wird. Beispiele für derartige Hydrolysereaktionen sind die Spaltung von Alkoholaten in Alkohol und Metallhydroxid oder die Spaltung von Metallhalogenid in Halogenwasserstoff und Metallhydroxid. Insbesondere wird unter einer "Hydrolysereaktion" im Sinne der Erfindung die Spaltung von Alkoholaten in Alkohol und Metallhydroxid verstanden.

Ohne dass es als Einschränkung der Erfindung verstanden werden soll, vermuten die Erfinder, dass durch die vorgenannten Hydrolysereaktionen das auf oder in der nasschemisch erzeugten Aluminiumoxidschicht gebundene Wasser zumindest teilweise, vorzugsweise überwiegend, weiter bevorzugt vollständig, verbraucht und somit eine Nachoxidation wirksam behindert oder verhindert wird. Überraschenderweise sind hierfür bereits geringe Mengen einer Metalloxidquelle ausreichend. Der Begriff "Metalloxidquelle" bezeichnet im Sinne der vorliegenden Erfindung metallhaltige Ausgangsstoffe, aus denen die Metalloxidbeschichtung erzeugt wird. Beispiele hierfür sind insbesondere Metallalkoxylate, wobei die Alkoxylgruppe ein bis sechs Kohlenstoffatome, vorzugsweise ein bis drei Kohlenstoffatome, enthält. Als sehr geeignet haben sich Tetramethoxysilan und/oder Tetraethoxysilan erwiesen. Ohne dass es als Einschränkung der Erfindung verstanden werden darf, vermuten die Erfinder, dass das nasschemisch erzeugte Aluminiumoxid die Bildung der von Aluminiumoxid verschiedenen Metalloxidschicht möglicherweise basierend auf pH-Wert-Effekten oder elektronischen Effekten begünstigt und daher bevorzugt das auf und/oder in der nasschemisch erzeugten Aluminiumoxidschicht gebundene Wasser verbraucht wird. Somit ermöglicht der bevorzugte Einsatz von Beschichtungsverfahren unter Verwendung einer Hydrolysereaktion zur Aufbringung der Metalloxidschicht, vorzugsweise das Sol-Gel-Verfahren, eine besonders schonende chemische Trocknung der nasschemisch erzeugten Aluminiumoxidschicht, die auf anderem Wege nicht zu erreichen gewesen wäre.

Eine möglichst schonende Entfernung des Wassers ist insbesondere bei den erfindungsgemäß eingesetzten nasschemisch oxidierten Aluminiumeffektpigmenten erforderlich. So neigen nasschemisch oxidierte Aluminiumeffektpigmente aufgrund ihrer rauen und dadurch großen Oberfläche deutlich stärker zur Agglomeration als nichtoxidierte Aluminiumeffektpigmente. Im Gegensatz zu konventionellen Verfahren wie beispielsweise einer Trocknung im Unterdruck wird bei dem erfindungsgemäßen Verfahren lediglich die im organischen Lösungsmittel enthaltene, geringe Wassermenge verbraucht und die Lösungsmittelmenge insgesamt mithin nur minimal verringert. Somit tritt keine merkliche Konzentrierung der Pigmente ein.

Ferner wird bei dem erfindungsgemäßen Verfahren die feine Oberflächenstruktur nicht geschädigt, wie dies beispielsweise bei herkömmlichen Trocknungsverfahren aufgrund von hohen thermischen Belastungen erfolgen kann. Ein Schädigung oder gar Zerstörung der feinen Oberflächenstruktur beeinträchtigt jedoch den durch nasschemische Oxidation der Aluminiumeffektpigmente erzeugten optischen Effekt, da die Eigenfarbe dieser Pigmentpartikel durch Interferenzeffekte an eben diesen feinen Oberflächenstrukturen hervorgerufen wird. Zudem sind die konventionellen Trocknungsverfahren sehr zeit- und arbeitsaufwendig. Auch binden konventionell getrocknete durch nasschemische Oxidation hergestellte Aluminiumeffektpigmente bei Luftkontakt Wasser, so dass eine Lagerung und Verarbeitung unter Luft- und Feuchtigkeitsausschluss notwendig wäre, um eine Nachoxidation zu vermeiden. Alternativ könnten derartige Pigmente über einen längeren Zeitraum gelagert werden, bis infolge eine nachfolgend ausgebildeten natürlichen Aluminiumoxidschicht eine ausreichende Farbkonstanz erreicht wird. Dies würde jedoch die Herstellungszeit drastisch erhöhen und zusätzliche Lagerkosten verursachen. Zudem würde eine derart unkontrollierte und unkontrollierbare Nachoxidation in einem schwer vorhersagbaren Farbton resultieren und könnte infolge einer ungleichmäßigen Nachoxidation der Pigmente die Farbbrillanz erniedrigen.

Ferner zeigte sich überraschenderweise, dass auch eine nachfolgende Freisetzung des Wassers in einem Kondensationsschritt, beispielsweise beim Sol-Gel-Prozess, unschädlich ist. Ohne dass es als Einschränkung der Erfindung verstanden werden soll, vermuten die Erfinder, dass das beispielsweise bei einer nachfolgenden Kondensation freigesetzte Wasser feinverteilt in der entstehenden und entstandenen von Aluminiumoxid verschiedenen Metalloxidschicht vorliegt. Aufgrund der Wasserundurchlässigkeit der von Aluminiumoxid verschiedenen Metalloxidschicht verursachen die feinverteilten Wassermoleküle jedoch keine Oxidation des darunterliegenden Aluminiums. Ferner liegt offenbar keine ausreichend große Ansammlung von Wassermolekülen vor, die beim Erhitzen der Partikel Gasblasen ausbilden könnten, wodurch die optische Qualität eines Applikationsmedium, beispielsweise einer Farbe oder eines Lackes, beeinträchtigt würde.

Zudem hat sich überraschenderweise gezeigt, dass die erfindungsgemäßen Aluminiumeffektpigmente weitere anwendungstechnische Vorteile aufweisen. Beispielsweise weisen die erfindungsgemäßen Aluminiumeffektpigmente eine hervorragende Applizierbarkeit in Form eines Pulverlackes auf.

Durch die oben beschriebene nasschemische Oxidation von Aluminiumeffektpigmenten können insbesondere gelbliche Farbtöne wie champagnerfarbene Farbtöne erhalten werden. Die erfindungsgemäßen Pigmente weisen einen ausgezeichneten Glanz auf. Durch Erhöhung des Oxidationsgrades können intensiver gefärbte und dunklere erfindungsgemäße Pigmente erhalten werden. Bei weiteren Ausführungsformen beträgt der Metallgehalt der erfindungsgemäßen Pigmente daher vorzugsweise höchstens 87 Gew.-%, weiter vorzugsweise 85 Gew.-%, noch weiter vorzugsweise höchstens 81 Gew.-%, mehr bevorzugt höchstens 77 Gew.-%, noch mehr bevorzugt höchstens 74 Gew.-% und am meisten bevorzugt höchstens 71 Gew.-%, jeweils bezogen auf das Gesamtgewicht der unbeschichteten, nasschemisch oxidierten Aluminiumeffektpigmente. Die Bestimmung des Metallgehalts erfolgt beispielsweise durch Auflösen der unbeschichteten Pigmente in 15%-iger wässeriger Natronlauge und Quantifizierung der freiwerdenden Wasserstoffmenge.

Es hat sich jedoch ferner gezeigt, dass bei zu dicken Aluminiumoxidschichten qualitativ minderwertige Farbtöne erzielt werden. Ohne dass es als Einschränkung der Erfindung verstanden werden soll, ist es die Ansicht der Erfinder, dass eine zu dicke, poröse Aluminiumoxidschicht keine ausreichende Stabilität aufweist und die bevorzugte regelmäßige Oberflächenstruktur immer mehr Schadstellen aufweist. Dies führt wiederum zu einer Verschlechterung der Pigmentfarbe. Bei weiteren Ausführungsformen, bei denen beispielsweise eine hellere Farbe zur Erzeugung einer subtilen Farbnuance gewünscht ist, ist es bevorzugt, dass der Gehalt an elementarem Aluminium bei den erfindungsgemäßen Pigmenten mindestens 25 Gew.-%, vorzugsweise mindestens 28 Gew.-%, mehr bevorzugt mindestens 31 Gew.-%, noch mehr bevorzugt mindestens 34 Gew.-% und am meisten bevorzugt mindestens 36 Gew.-% beträgt, jeweils bezogen auf das Gesamtgewicht der unbeschichteten, nasschemisch oxidierten Aluminiumeffektpigmente.

Bei weiteren der vorgenannten Ausführungsformen liegt der Gehalt an elementarem Aluminium bei den erfindungsgemäß eingesetzten nasschemisch oxidierten Aluminiumeffektpigmente in einem Bereich von 25 bis 87 Gew.-%, bezogen auf das Gewicht der nasschemisch oxidierten Aluminiumeffektpigmente. Vorzugsweise liegt der Gehalt an elementarem Aluminium bei den erfindungsgemäßen Pigmente in einem Bereich von 28 bis 81 Gew.-%, mehr bevorzugt in einem Bereich von 31 bis 77 Gew.-%, noch mehr bevorzugt in einem Bereich von 34 bis 74 Gew.-% und am meisten bevorzugt in einem Bereich von 36 bis 71 Gew.-%, jeweils bezogen auf das Gesamtgewicht der unbeschichteten, nasschemisch oxidierten Aluminiumeffektpigmente.

Obgleich nasschemisch oxidierte Aluminiumeffektpigmente eine sehr große Oberfläche aufweisen, an und/oder in der große Mengen Wasser gebunden und/oder enthalten sind, hat sich überraschenderweise gezeigt, dass im Verhältnis zum nasschemisch erzeugten Aluminiumoxid bereits sehr geringe Mengen an einer von Aluminiumoxid verschiedenen Metalloxidschicht wirkungsvoll eine Nachoxidation vermindern oder vollständig unterbinden. Ferner zeigte sich überraschenderweise, dass in Verbindung mit der erfindungsgemäß über, vorzugsweise auf, der von Aluminiumoxid verschiedenen Metalloxidschicht aufgebrachten organischen Polymerschicht eine sehr geringe Menge an der Metalloxidschicht im Verhältnis zur nasschemisch erzeugten Aluminiumoxidschicht eine überraschend hohe Stabilität gegenüber beispielsweise oxidierenden Bedingungen erreicht wird. Da eine dicke Metalloxidschicht die optische Qualität der Pigmente beeinträchtigt und insbesondere die Deckungsfähigkeit, d.h. die pro Gewichtseinheit an Pigment abgedeckte Fläche, der Pigmente mindert, ermöglicht die vorliegende Erfindung die Bereitstellung von farbigen Aluminiumeffektpigmenten von besonders hoher optischer Qualität bei hoher Stabilität, insbesondere von langanhaltender Farbstabiltät.

Das Verhältnis des in Form der Metalloxidschicht aufgebrachten von Aluminiumoxid verschiedenen Metalloxids, insbesondere Siliziumoxid, zu dem in der nasschemisch erzeugten Aluminiumoxidschicht enthaltenen Aluminiumoxids liegt in einem Bereich von 1:1 bis 1:40, vorzugsweise in einem Bereich von 1:1,2 bis 1:36, mehr bevorzugt in einem Bereich von 1:1,5 bis 1:32 und noch mehr bevorzugt in einem Bereich von 1:1,8 bis 1:28. Insbesondere ist es bei weiteren der vorgenannten Ausführungsformen bevorzugt, dass das Verhältnis des in Form der Metalloxidschicht aufgebrachten von Aluminiumoxid verschiedenen Metalloxids, insbesondere Siliziumoxid, zu dem in der nasschemisch erzeugten Aluminiumoxidschicht enthaltenen Aluminiumoxids in einem Bereich von 1:2 bis 1:25, vorzugsweise in einem Bereich von 1:2,2 bis 1:21, mehr bevorzugt in einem Bereich von 1:2,4 bis 1:18 und noch mehr bevorzugt in einem Bereich von 1:2,5 bis 1:15 liegt. Die vorgenannten Verhältnisse stellen das Verhältnis des Gewichts des aufgebrachten von Aluminiumoxid verschiedenen Metalloxids in Bezug auf das Gewicht der durch nasschemische Oxidation erzeugten Aluminiumoxids dar.

Die Menge des in der nasschemisch erzeugten Aluminiumoxidschicht enthaltenen Aluminiumoxids kann beispielsweise durch Bestimmung der im Substrat enthaltenen Menge an elementarem Aluminium, Bestimmung der Gesamtmenge des im Substrat enthaltenen Aluminiums in Form von Aluminium und Aluminiumoxid und Rückrechnung des in der nasschemisch erzeugten Aluminiumoxidschicht enthaltenen $Al_2O_3$ erfolgen. Die Bestimmung des elementaren Aluminiumgehalts kann beispielsweise gasvolumetrisch erfolgen. Hierzu kann es erforderlich sein die Pigmente zunächst in geeigneten Lösungsmitteln, beispielsweise organischen Lösungsmitteln, teilweise aufzulösen.

Die gasvolumetrische Bestimmung des im Substrat enthaltenen Aluminiums kann beispielsweise mit der folgenden Methode erfolgen: 0,3 g der Probe werden in einem Porzellantiegel eingewogen. Es werden 50 ml 5%-ige Natronlauge in einen 250 ml Erlenmeyer-Kolben vorgelegt und der Porzellantiegel wird so auf die NaOH-Lösung aufgebracht, dass er aufschwimmt. Anschließend wird der Erlenmeyer-Kolben auf 20 °C temperiert. Es wird eine Gasbürette mit Ausgleichsgefäß gasdicht an den Erlenmeyer-Kolben angeschlossen und auf 0 eingestellt. Danach wird der Erlenmeyer-Kolben geschüttelt, bis der Tiegel umkippt und das Pigment vollständig mit der NaOH-Lösung reagieren kann. Der entstehende Wasserstoff verdrängt das Wasser in der Gasbürette.

$$p = p_{abgel.} + c - d \text{ (in Torr)}$$

$p_{ebgel.}$ = abgelesener Druck (in Torr)
c = Korrektur des abgelesenen Barometerstandes: -2,6 Torr (für 760 Torr und 20 °C)
d = Sättigungsdruck Wasserdampf für 5 %ige Natronlauge: 16,7 Torr (760 Torr 20 °C)
p = korrigierter Druck (in Torr)

Der erhaltene Wert p (in Torr) wird dann in die untere Gleichung eingesetzt, so dass der Aluminiumgehalt in % ermittelt werden kann.

$$\%\text{Metallgehalt} = \frac{p\,[\text{Torr}] * V\,[\text{ml}]}{\text{Einw. [mg]}} * \left( \frac{273\,[\text{K}] * 100}{293[\text{K}] * 760\,[\text{torr}]} \right) * \left( \frac{2 * 26,98\,[\text{mg/mMol}]}{3 * 22,442[\text{ml/mMol}]} \right)$$

V = abgelesenes Volumen in ml

Einw. = Einwaage in mg
Normaldruck in Torr: 760
Normaldruck in Pascal: 1013 $10^2$ Pa
Normtemperatur: 0 °C = 273 K
Arbeitstemperatur: 20 °C = 293 K

Zur Erzielung besonders ansprechender Farbtöne hat es sich ferner als vorteilhaft erwiesen, dass die nasschemisch erzeugte Aluminiumoxidschicht eine gewisse Mindestdicke aufweist. Bei weiteren Ausführungsformen weist die nasschemisch erzeugte Aluminiumoxidschicht daher eine Dicke von mindestens 30 nm, vorzugsweise von mindestens 35 nm, mehr bevorzugt von mindestens 45 nm, noch mehr bevorzugt von mindestens 55 nm auf. Die Dicke der nasschemisch erzeugten Aluminiumoxidschicht wird hierbei als Mittelwert mittels REM an 20 zufällig ausgewählten Pigmenten bestimmt.

Da die nasschemisch erzeugte Aluminiumoxidschicht jedoch im Vergleich zum ursprünglichen Aluminium deutlich weniger duktil ist, weist ein entsprechendes Pigment eine deutlich verringerte Verformbarkeit auf. Daher beträgt die Dicke der nasschemisch erzeugten Aluminiumoxidschicht höchstens 300 nm, vorzugsweise höchstens 250 nm, mehr bevorzugt höchstens 210 nm und noch mehr bevorzugt höchstens 160 nm.

Erfindungsgemäß liegt die Dicke der nasschemisch erzeugten Aluminiumoxidschicht in einem Bereich von 30 nm bis 300 nm, vorzugsweise in einem Bereich von 35 nm bis 250 nm, mehr bevorzugt in einem Bereich von 45 nm bis 210 nm und noch mehr bevorzugt in einem Bereich von 55 nm bis 160 nm. Beispielsweise ist es bei weiteren besonders bevorzugten Ausführungsformen bevorzugt, dass die Dicke der nasschemisch erzeugten Aluminiumoxidschicht in einem Bereich von 60 bis 120 nm liegt.

Ohne dass es als Einschränkung der Erfindung verstanden werden soll, vermuten die Erfinder, dass die mittels der Hydrolysereaktion, vorzugsweise dem Sol-Gel-Prozess, aufgebrachte Metalloxidschicht nicht nur das in oder auf der nasschemisch erzeugten Aluminiumoxidschicht gebundene Wasser verbraucht. So zeigten BET-Messungen, dass die BET-Oberfläche eines nasschemisch oxidierten Alumiumeffektpigmentes, wie in Abbildung 2 dargestellt, durch die Aufbringung einer Siliziumoxidbeschichtung von 35,4 $m^2$/g auf 5,2 $m^2$/g sinkt. Ohne dass es als Einschränkung der Erfindung verstanden werden soll, vermuten die Erfinder, dass die erfindungsgemäß aufgebrachte von Aluminiumoxid verschiedene Metalloxidbeschichtung die Hohlräume zwischen den mittels der nasschemischen Oxidation gebildeten Strukturen ausfüllt und somit die BET-Oberfläche drastisch reduziert. Bei Aluminiumeffektpigmenten ähnlicher Größe ohne nasschemische Oxidation wird hingegen gefunden, dass die BET-Oberfläche bei Pigmenten mit höherem $D_{50}$ annähernd gleich bleibt (1,4 $m^2$/g vor und nach der Aufbringung einer von Aluminiumoxid verschiedenen Metalloxidbeschichtung) oder bei Pigmenten mit kleinerem $D_{50}$ nur leicht von beispielsweise 4,2 $m^2$/g auf 3,7 $m^2$/g sinkt. Die leichte Verringerung der BET-Oberfläche der Pigmente mit kleinem $D_{50}$-Wert wird darauf zurückgeführt, dass diese Pigmente eine größere Menge an Feinanteil haben und diese sehr kleinen Partikel zumindest partiell in die Metalloxidbeschichtung eingebaut werden. Gleichzeitig wird die BET-Oberfläche jedoch nicht auf die ursprüngliche BET-Größe der Pigmente vor der nasschemischen Oxidation verringert (2,6 $m^2$/g), was auch die entsprechenden REM-Aufnahmen zeigen.

Die mittels nasschemischer Oxidation erzeugten Oberflächenstrukturen sind auch nach der Aufbringung der von Aluminiumoxid verschiedenen Metalloxidschicht noch deutlich erkennbar. Da der gewünschte Farbeffekt auf Interferenzeffekten an den mittels der nasschemischen Oxidation erzeugten Strukturen beruht, bietet das Ausfüllen dieser Hohlräume eine mechanische Stabilisierung, die für die erfindungsgemäß eingesetzten nasschemisch oxidierten Aluminiumeffektpigmente von besonders großer Bedeutung ist. Bei weiteren Ausführungsformen ist es daher bevorzugt, dass die Menge der auf die nasschemisch oxidierte Aluminiumeffektpigmentoberfläche aufgebrachten wenigstens einen von Aluminiumoxid verschiedenen Metalloxidschicht mindestens 0,8 Gew.-%, vorzugsweise mindestens 1,2 Gew.-%, mehr bevorzugt mindestens 1,6 Gew.-%, noch mehr bevorzugt mindestens 1,9 Gew.-% und am meisten bevorzugt mindestens 2,1 Gew.-% beträgt, jeweils bezogen auf das Gesamtgewicht der unbeschichteten, nasschemisch oxidierten Aluminiumeffektpigmente. Insbesondere ist es bei weiteren Ausführungsformen bevorzugt, dass die BET-Oberfläche der erfindungsgemäßen Pigmente nach Aufbringung der Metalloxidschicht höchstens 30 %, vorzugsweise höchstens 25 %, mehr bevorzugt höchstens 22 %, noch mehr bevorzugt höchstens 19 % und am meisten bevorzugt höchstens 16 % beträgt, jeweils bezogen auf die BET-Oberfläche der nasschemisch oxidierten Aluminiumeffektpigmente vor der Aufbringung der von Aluminiumoxid verschiedenen Metalloxidschicht. Die Bestimmung der BET-Oberfläche kann beispielsweise mittels eines BELsorp mini II der Firma BEL Inc. mit Sitz in Japan erfolgen.

Eine zu dicke von Aluminiumoxid verschiedene Metalloxidschicht beeinträchtigt jedoch die optischen Eigenschaften und vermindert die Deckungsfähigkeit der erfindungsgemäßen Pigmente. Bei weiteren Ausführungsformen ist es daher bevorzugt, dass die Menge der wenigstens einen von Aluminiumoxid verschiedenen Metalloxidschicht höchstens 20 Gew.-%, vorzugsweise höchstens 17 Gew.-%, mehr bevorzugt höchstens 16 Gew.-%, noch mehr bevorzugt höchstens 15 Gew.-%, und am meisten bevorzugt höchstens 14 Gew.-% beträgt, jeweils bezogen auf das Gesamtgewicht der unbeschichteten, nasschemisch oxidierten Aluminiumeffektpigmente. Insbesondere ist es bei weiteren Ausführungsformen bevorzugt, dass die Menge der wenigstens einen von Aluminiumoxid verschiedenen Metalloxidschicht in einem Bereich von 0,8 Gew.-% bis 20 Gew.-%, vorzugsweise von 1,2 Gew.-% bis 17 Gew.-%, mehr bevorzugt von 1,6 Gew.-% bis 16 Gew.-%, noch mehr bevorzugt von 1,9 Gew.-% bis 15 Gew.-% und am meisten bevorzugt von 2,1 Gew.-% bis 14 Gew.-% beträgt, jeweils bezogen auf das Gesamtgewicht der unbeschichteten, nasschemisch oxidierten Aluminiumeffektpigmente.

Der Begriff "nasschemisch erzeugte Aluminiumoxidschicht" im Sinne der vorliegenden Erfindung bezeichnet die infolge der nasschemischen Oxidation auf der Aluminiumeffektpigmentoberfläche gebildete Oxidschicht. In dieser kann Wasser gebunden sein, wodurch die Schicht teilweise oder vollständig der formalen Zusammensetzung von Aluminiumhydroxid ($Al(OH)_3$) entsprechen kann. Insbesondere ist jedoch bevorzugt, dass die nasschemisch erzeugte Aluminiumoxidschicht zu mindestens 30 mol-%, vorzugsweise zu mindestens 40 mol-%, mehr bevorzugt zu mindestens 60 mol-% und noch mehr bevorzugt zu mindestens 80 mol-% aus $Al_2O_3$ besteht, jeweils bezogen auf die Gesamtmolmenge des in der nasschemisch erzeugten Aluminiumoxidschicht enthaltenen Aluminiums. Bei der Verwendung von Aluminiumeffektpigmenten, die nicht aus reinem Aluminium, mithin aus einer Aluminiumlegierung, bestehen, kann die entsprechend nasschemisch erzeugte Oxidschicht naturgemäß auch andere Metalle und/oder Metalloxide enthalten. Auch im Falle einer Aluminiumlegierung beziehen sich die vorstehenden Angaben in mol-% nur auf die in der nasschemisch erzeugten Aluminiumlegierungsoxidschicht enthaltene Gesamtmolmenge an Aluminium.

Das erfindungsgemäß auf die nasschemisch oxidierten Aluminiumeffektpigmente aufzutragende Metalloxid ist kein Aluminiumoxid. Metalloxide, die erfindungsgemäß auf die nasschemisch oxidierten Aluminiumeffektpigmente aufgebracht werden können, werden beispielsweise ausgewählt aus der Gruppe bestehend aus Siliziumoxid, Boroxid, Zirkoniumoxid, Ceroxid, Eisenoxid, Titanoxid, Chromoxid, Zinnoxid, Molybdänoxid, deren Oxidhydraten, deren Hydroxiden und Mischungen davon. Insbesondere ist es bei weiteren Ausführungsformen bevorzugt, dass die als Metalloxidschicht aufgebrachten von Aluminiumoxid verschiedenen Metalloxide keine merkliche Eigenfarbe aufweisen, wie beispielsweise Siliziumoxid, Boroxid, Zirkoniumoxid, Titanoxid, deren Oxidhydraten, deren Hydroxiden und Mischungen davon. Ein besonders zur Erzeugung der erfindungsgemäß aufzubringenden wenigstens einen Metalloxidschicht geeignetes Metalloxid ist Siliziumoxid. Bei weiteren, besonders bevorzugten Ausführungsformen umfasst die wenigstens eine Metalloxidschicht daher Siliziumoxid. Gemäß einer weiteren bevorzugten Ausführungsform besteht die wenigstens eine Metalloxidschicht im Wesentlichen aus Siliziumoxid oder besteht noch mehr bevorzugt aus Siliziumoxid, dessen Hydroxiden oder Mischungen hiervon. Bei weiteren der vorgenannten Ausführungsformen besteht die wenigstens eine Metalloxidschicht im Wesentlichen aus Siliziumoxid, vorzugsweise $SiO_2$.

Bei weiteren Ausführungsformen ist es bevorzugt, dass die erfindungsgemäßen Pigmente zusätzlich zur wenigstens einen von Aluminiumoxid verschiedenen Metalloxidschicht eine hochbrechende Metallchalkogenidschicht, vorzugsweise eine hochbrechende Metalloxidschicht, aufweisen. Ein Beispiel derartiger hochbrechender Metalloxidschichten sind Eisenoxidschichten.

Der Begriff "besteht im Wesentlichen aus" im Sinne der vorliegenden Erfindung bedeutet, dass mindestens 90 Gew.-%, mehr bevorzugt mindestens 95 Gew.-%, noch mehr bevorzugt mindestens 99 Gew.-% und am meisten bevorzugt mindestens 99,9 Gew.-% aus dem betreffenden Stoff bestehen, soweit dies nicht im Einzelfall anders definiert ist. Im Rahmen dieser Erfindung wird beispielsweise unter dem Begriff "im Wesentlichen bestehend aus Siliziumoxid" verstanden, dass die Schicht überwiegend aus Siliziumoxid, bevorzugt $SiO_2$, besteht, jedoch auch bis zu 20 Gew.-% Wasser, bezogen auf die Siliziumoxidschicht, enthalten kann. Ferner kann das Siliziumoxid, hergestellt beispielsweise mittels des Sol-Gel-Prozesses aus Tetraalkoxysilanen, bis zu 5 Gew.-% Alkoxygruppen, die nicht hydrolysiert und kondensiert wurden, enthalten. Entsprechendes gilt für andere von Aluminiumoxid verschiedene Metalloxide, die als Metalloxidschicht aufgebracht werden oder sind.

Vorzugsweise besteht die wenigstens eine, bevorzugt eine (Zahl: 1), Metalloxidschicht im Wesentlichen, vorzugsweise vollständig, aus Siliziumoxid, besonders bevorzugt aus $SiO_2$. Im Sinne der Erfindung wird unter einer (Zahl: 1) Metalloxidschicht auch ein Aufbau von mehreren stoffidentischen Schichten verstanden, die prozeßtechnisch nacheinander unmittelbar aufeinander aufgebracht sind. Bei weiteren besonders bevorzugten Ausführungsformen ist die wenigstens eine umhüllende Metalloxidschicht eine (Zahl: 1) Siliziumoxidschicht, vorzugsweise eine (Zahl: 1) Si02-Schicht.

Der Begriff "Metalloxidschicht" im Sinne der vorliegenden Erfindung bezieht sich auf eine, vorzugsweise mittels einer Hydrolysereaktion, insbesondere mittels des Sol-Gel-Prozesses, hergestellten von Aluminiumoxid verschiedenen Metalloxidschicht, die auf die durch nasschemische Oxidation auf dem Aluminiumeffektpigment erzeugte Aluminiumoxidschicht aufgebracht ist. Eine besonders bevorzugte Art der Metalloxidschicht im Sinne der vorliegenden Erfindung umfasst oder besteht aus Siliziumoxid, bevorzugt $SiO_2$. Insbesondere ist es bevorzugt, dass die vorgenannte Beschichtung im Wesentlichen aus Siliziumoxid, vorzugsweise $SiO_2$, besteht. Der Begriff "Metalloxidschicht" im Sinne der vorliegenden Erfindung umfasst keine anorganisch/organische Mischschichten, wie sie in der US 2008/0249209 A1 beschrieben sind.

Insbesondere ist es bei weiteren Ausführungsformen bevorzugt, dass die erfindungsgemäßen, beschichteten Pigmente eine (Zahl: 1) einzige erfindungsgemäße von Aluminiumoxid verschiedene Metalloxidschicht aufweisen.

Die "organische Polymerschicht" im Sinne der vorliegenden Erfindung ist eine nichtreaktive organische Polymerschicht. Eine nichtreaktive organische Polymerschicht im Sinne der vorliegenden Erfindung ist im Wesentlichen vollständig, vorzugsweise vollständig, ausgehärtet. Eine derart ausgehärtete organische Polymerschicht reagiert mithin beispielsweise nicht wesentlich oder gar nicht mit dem Bindemittel eines Beschichtungsmittels, wie zum Beispiel eines Lackes, beispielsweise eines Pulverlackes, oder einer Farbe. Gemäß einer bevorzugten Variante findet keine Reaktion zwischen der ausgehärteten organischen Polymerschicht und dem Bindemittel eines Beschichtungsmittels statt. Als organische Polymere können eine große Zahl an dem Fachmann bekannten Polymeren eingesetzt werden. Insbesondere handelt es sich bei der "organischen Polymerschicht" um keine Beschichtung aus noch nicht ausgehärtetem Bindemittel, wie sie in der WO 2005/063897 A2 offenbart ist. Ein Bindemittel ist dadurch charakterisiert, dass es erst später in der Anwendung, beispielsweise als Harz/Härtersystem oder durch radikalische Polymerisation, aushärtet. Beispiele organischer Polymere, die insbesondere zur Verbesserung der chemischen Beständigkeit bei der vorliegenden Erfindung eingesetzt werden können, sind Polyacrylate, Polymethacrylate, Polyacrylamide, Polyacrylnitrile, Polyvinylchloride, Polyvinylacetate, Polyamide, Polyalkene, Polydiene, Polyalkine, Polyalkylenglycole, Epoxidharze, Polyester, Polyether, Polyole, Polyurethane, Polycarbonate, Polyethylenterephthalate, Kunststofflegierungen und Mischungen davon. Insbesondere ist es bei weiteren Ausführungsformen bevorzugt, dass die organische Polymerschicht direkt auf der von Aluminiumoxid verschiedenen Metalloxidschicht aufgebracht wird. Hierbei muss verstanden werden, dass in begrenztem Umfang auch ein Kontakt zwischen der nasschemisch erzeugten Aluminiumoxidschicht und der organischen Polymerschicht vorhanden sein kann. Gemäß einer bevorzugten Variante der Erfindung liegt kein Kontakt zwischen der durch die nasschemische Oxidation erzeugten Aluminiumoxidschicht der organischen Polymerschicht vor.

Zur Erzielung der gewünschten hohen Stabilität oder Beständigkeit, beispielsweise gegenüber korrosiven Medien, hat es sich als erforderlich erwiesen, dass eine gewisse Mindestmenge der organischen Polymerschicht aufgebracht ist, so dass eine im Wesentlichen vollständige, vorzugsweise vollständige, Umhüllung der von Aluminiumoxid verschiedenen Metalloxidschicht vorliegt. Bei weiteren Ausführungsformen ist es insbesondere bevorzugt, dass der Anteil der wenigstens einen organischen Polymerschicht mindestens 8 Gew.-%, vorzugsweise mindestens 9 Gew.-%, mehr bevorzugt mindestens 9,5 Gew.-%, noch mehr bevorzugt mindestens 10 Gew.-% und am meisten bevorzugt mindestens 11 Gew.-% beträgt, jeweils bezogen auf das Gesamtgewicht der unbeschichteten, nasschemisch oxidierten Aluminiumeffektpigmente.

Die Aufbringung sehr dicker organischer Polymerschichten hat sich jedoch als wenig vorteilhaft erwiesen, da nur geringfügige weitere Verbesserungen der chemischen Beständigkeit erreicht werden, jedoch beispielsweise zusätzliche Kosten entstehen und sich die optische Qualität der Pigmente, insbesondere die Deckungsfähigkeit, verschlechtert. Bei weiteren Ausführungsformen beträgt die Menge der wenigstens einen organischen Polymerschicht daher höchstens 40 Gew.-%, vorzugsweise höchstens 35 Gew.-%, mehr bevorzugt höchstens 30 Gew.-%, noch mehr bevorzugt höchstens 23 Gew.-% und am meisten bevorzugt höchstens 18 Gew.-%, jeweils bezogen auf das Gesamtgewicht der unbeschichteten, nasschemisch oxidierten Aluminiumeffektpigmente.

Bei weiteren der vorgenannten Ausführungsformen liegt die Menge der wenigstens einen organischen Polymerschicht in einem Bereich von 8 bis 40 Gew.-%, vorzugsweise in einem Bereich von 9 bis 35 Gew.-%, mehr bevorzugt in einem Bereich von 9,5 bis 30 Gew.-%, noch mehr bevorzugt in einem Bereich von 10 bis 23 Gew.-%, und am meisten bevorzugt in einem Bereich von 11 bis 18 Gew.-%, jeweils bezogen auf das Gesamtgewicht der unbeschichteten, nasschemisch oxidierten Aluminiumeffektpigmente.

Bei komplexen Schichtaufbauten von organischen und/oder anorganischen Schichten empfehlen sich zur Analyse derartiger Schichten Sputtertechniken in Kombination mit oberflächensensitiven analytischen Methoden wie ESCA und/oder SIMS, insbesondere TOF-SIMS.

Es wurde gefunden, dass es bei weiteren Ausführungsformen bevorzugt ist, dass die erfindungsgemäßen Pigmente eine mittlere Dicke der organischen Polymerschicht in einem Bereich von 80 nm bis 300 nm, bevorzugt von 100 nm bis 250 nm und besonders bevorzugt von 120 nm bis 230 nm aufweisen. Die vorgenannte mittlere Dicke der organischen Polymerschicht kann durch REM als arithmetisches Mittel der mittleren Dicke von mindestens 20 zufällig ausgewählten Pigmenten bestimmt werden. So zeigte sich, dass bei einer mittleren Dicke der organischen Polymerschicht unter 80 nm die vorteilhaften Eigenschaften der erfindungsgemäßen Pigmente für bestimmte Anwendungen nicht ausreichend ausgeprägt sind. Ferner steht der Verlust an Deckfähigkeit und/oder Glanz bei dicken organischen Polymerschichten der Aufgabenstellung der vorliegenden Erfindung zur Bereitstellung von Aluminiumeffektpigmenten mit ansprechenden optischen Eigenschaften entgegen, so dass die Dicke der Beschichtung möglichst niedrig gewählt werden sollte.

Gemäß weiteren bevorzugten Ausführungsformen besteht die wenigstens eine organische Polymerschicht im Wesentlichen aus einem Polymer, das ausgewählt wird aus der Gruppe bestehend aus Polyacrylat, Polymethacrylat, Polyacrylamid, Polyacrylnitril, Polyvinylchlorid, Polyvinylacetat, Polyamid, Polyalken, Polydien, Polyalkin, Polyalkylenglycol, Epoxidharz, Polyester, Polyether, Polyol, Polyurethan, Polycarbonat, Polyethylenterephthalat, Polymerlegierungen und Mischungen hiervon.

Bei einer Variante der Erfindung ist bevorzugt, dass die organische Polymerschicht höchstens 10 Gew.-%, vorzugsweise höchstens 5 Gew.-%, mehr bevorzugt höchstens 1 Gew.-% und noch mehr bevorzugt höchstens 0,1 Gew.-% Fluorpolymere umfasst, jeweils bezogen auf das Gesamtgewicht der Polymerschicht. Insbesondere ist es bevorzugt, dass keine Fluorpolymere in der organischen Polymerschicht enthalten sind.

Gemäß weiteren der vorgenannten Ausführungsformen besteht die wenigstens eine organische Polymerschicht im Wesentlichen aus einem Polymer, das ausgewählt wird aus der Gruppe bestehend aus Polyacrylat, Polymethacrylat, Polyurethan, Polyester und Mischungen davon. Erfindungsgemäße Pigmente mit mindestens einer derartigen organischen Polymerschicht zeichnen sich beispielsweise durch eine erhöhte UV-Beständigkeit aus. Eine erhöhte UV-Stabilität ist dann erwünscht, wenn die erfindungsgemäßen Aluminiumeffektpigmente bei Außenanwendungen verwendet werden, wie beispielsweise in einem Automobillack, einem Fassadenlack, etc.. Als besonders geeignete Polymere zur Herstellung von organischen Polymerschichten mit erhöhter UV-Beständigkeit haben sich beispielsweise Polyacrylate, Polymethacrylate oder deren Mischungen erwiesen. Bei weiteren Ausführungsformen der Erfindung besteht die mindestens eine organische Polymerschicht daher im Wesentlichen aus Polyacrylaten, Polymethacrylaten oder Mischungen davon.

Als Monomere für die Herstellung von Polyacrylaten und Polymethacrylaten werden beispielsweise Isoamylacrylat, Laurylacrylat, Stearylacrylat, Butoxyethylacrylat, Ethoxy-diethylenglycolacrylat, Methoxy-triethylenglycolacrylat, Methoxy-polyethyleneglycolacrylat, Methoxydipropylenglycolacrylat, Phenoxyethylacrylat, Phenoxy-polyethylenglycolacrylat, Tetrahydrofurfurylacrylat, Isobornylacrylate, 2-Hydroxyethylacrylat, 2-Hydroxypropylacrylat, 2-Hydroxy-3-phenoxypropylacrylat, 2-Acryloyloxyethylsuccinsäure, 2-Acryloyloxyethylphthalsäure, 2-Acryloyloxyethyl-2-hydroxyethylphthalsäure, Triethylenglycoldiacrylat, Neopentylglycoldiacrylat, 1,6-Hexandioldiacrylat, 1,9-Nonandioldiacrylat, Dimethyloltricyclodecanediacrylat, Trimethylolpropantriacrylat, Pentaerythritoltriacrylat, Pentaerythritoltetraacrylat, Dipentaerythritolhexaacrylat, 2-Hydroxy-3-acryloyloxypropylmethacrylat, Isooctylacrylat, Isomyristylacrylat, Isostearylacrylat, 2-Ethylhexyldiglycolacrylat, 2-Hydroxybutylacrylat, 2-Acryloyloxyethylhexahydrophthalsäure, Hydroxypivalinsäure Neopentylglycoldiacrylat, Polytetraethylenglycoldiacrylat, Ditrimethylolpropantetraacrylat, Methylmethacrylat, Ethylmethacrylat, n-Butylmethacrylat, Isobutylmethacrylat, 2-Ethylhexylmethacrylat, Isodecylmethacrylat, n-Laurylmethacrylat, Tridecylmethacrylat, n-Stearylmethacrylat, Methoxydiethylenglycolmethacrylat, Methoxypolyethylenglycolmethacrylat, Cyclohexylmethacrylat, Tetrahydrofurfuralmethacrylat, Benzylmethacrylat, Phenoxyethylmethacrylat, Isobornylmethacrylat, 2-Hydroxyethylmethacrylat, 2-Hydroxypropylmethacrylat, 2-Hydroxybutylmethacrylat, 2-Methacryloyloxyethylsuccinsäure, 2-Methacroyloxyethylhexahydrophthalsäure, 2-Methacryloyloxyethyl2-hydroxypropylphthalat, Ethyleneglycoldimethacrylat, Diethyleneglycoldimethacrylat, 1,4-Butandioldimethacrylat, 1,3-Butandioldimethacrylat, 1,6-Hexandioldimethacrylat, 1,9-Nonandioldimethacrylat, Trimethylolpropantrimethacrylat, Glycerindimethacrylat, 2-Hydroxy-3-acryloyloxypropylmethacrylat, t-Butylmethacrylat, Isostearylmethacrylat, Methoxytriethylenglycolmethacrylat, n-Butoxyethylmethacrylat, 3-Chloro-2-hydroxypropylmethacrylat, Triethylenglycoldimethacrylat, Neopentylglycoldimethacrylat oder Mischungen hiervon verwendet.

Besonders bevorzugt wird mindestens ein Monomer mit mindestens zwei, besonders bevorzugt drei, reaktiven Doppelbindungen (Vernetzer) verwendet. Besonders bevorzugt enthält oder besteht das Monomer daher aus 1,6-Hexandioldiacrylat, 1,9-Nonandioldiacrylat, Dimethyloltricyclodecandiacrylat, Neopentylglycoldimethacrylat, Trimethylolpropantriacrylat, Trimethylolpropantrimethacrylat oder deren Mischungen.

Weiterhin kann die (meth)acrylathaltige organische Polymerschicht zusätzlich Acrylsäure, Methacrylsäure oder Mischungen davon sowie weitere radikalisch polymerisierbare ungesättigte Verbindungen aufweisen.

Ferner kann die (meth)acrylathaltige organische Polymerschicht (meth)acrylathaltige Silanen enthalten. Hierbei entspricht der Mengenanteil der eingesetzten (meth)acrylathaltigen Silane maximal dem Mengenanteil der eingesetzten sonstigen (Meth)acrylatmonomere. Vorzugsweise liegt das Molverhältnis von (meth)acrylathaltigen Silanen zu weiteren (Meth)acrylatmonomere bei 1:2 bis 1:40, vorzugsweise bei 1:3 bis 1: 30.

Gemäß einer weiteren erfindungsgemäßen Variante wird die organische Polymerschicht ausgewählt aus der Gruppe bestehend aus Polyamid, Polycarbonat, Polyvinylchlorid, Polyethylenterephthalat und Mischungen davon. Erfindungsgemäße Pigmente mit mindestens einer derartigen organischen Polymerschicht zeichnen sich beispielsweise durch eine erhöhte Temperaturstabilität aus.

Gemäß einer bevorzugten Ausführungsform der Erfindung ist das für die wenigstens eine organische Polymerschicht eingesetzte Polymer bis zu einer Temperatur von wenigstens 180 °C, weiter bevorzugt bis zu einer Temperatur von wenigstens 260 °C, noch weiter bevorzugt bis zu einer Temperatur von wenigstens 350 °C, temperaturstabil. Unter temperaturstabil wird verstanden, dass die organische Polymerschicht der erfindungsgemäßen Pigmente bei der vorgenannten Temperatur nicht schmilzt und/oder sich zersetzt. Die Prüfung auf ein mögliches Schmelzen und/oder Zersetzen bei einer vorgegebenen Temperatur kann beispielsweise mittels dynamischer Differenzkalorimetrie erfolgen.

Gemäß einer weiteren bevorzugten Ausführungsform sind auf der organischen Polymerschicht physikalisch gebundene Oberflächenmodifizierungsmittel aufgebracht.

Auch hat sich gezeigt, dass es bei weiteren Ausführungsformen vorteilhaft ist, eine Kombination von wenigstens einer dünnen Metalloxidschicht und wenigstens einer dickeren organischen Polymerschicht auf die nasschemisch oxidierten Aluminiumeffektpigmente aufzubringen. Mittels der Kombination einer dünnen Metalloxidschicht und einer dickeren organischen Polymerschicht ist es möglich, ein Optimum an optischer Qualität zu erzielen und gleichzeitig eine hervorragende Chemikalienstabilität und Oxidationsbeständigkeit zu erreichen. Derartige Pigmente sind beispielsweise besonders geeignet für Einbrennprozesse unter besonders anspruchsvollen Bedingungen bei der Pulverlackbeschichtung oder der Coil-Coating-Lackierung.

Insbesondere ist es bei weiteren Ausführungsformen bevorzugt, dass die erfindungsgemäßen, beschichteten Pigmente eine (Zahl: 1) Metalloxidschicht und nur eine (Zahl: 1) organische Polymerschicht aufweisen.

Bei weiteren Ausführungsformen ist es bevorzugt, dass die wenigstens eine organische Polymerschicht mittels einer Initiator-induzierten radikalischen Polymerisation erzeugt wird. Es wurde gefunden, dass mittels dieses Verfahrens beispielsweise auf einfache Weise eine raue organische Polymerschicht erhalten werden kann. Erfindungsgemäße Pigmente mit einer derartigen Oberflächenstuktur zeichnen sich dadurch aus, dass sie sich leichter elektrostatisch aufladen lassen, wodurch eine vereinfachte Applikation der erfindungsgemäßen Pigmente mittels der Pulverlackbeschichtung ermöglicht wird.

Bei anderen Ausführungsformen ist es hingegen bevorzugt, dass die organische Polymerschicht mittels thermischer Polymerisation erzeugt wird. Mithin ist es beispielsweise besonders einfach glatte Oberflächen der organischen Polymerschicht zu erzeugen, welche sich wegen eines geringeren Bindemittelbedarfs besser in einen Lack wie beispielsweise einen Pulverlack oder einen Coil-Coating Lack einbetten lässt.

Der Schichtdicken der Metalloxidschichten und der organischen Polymerschichten auf den erfindungsgemäßen Pigmenten werden beispielsweise mittels REM-Aufnahmen an geeigneten Querschliffen ermittelt. Hierfür werden die Pigmente in einem Lack appliziert und dieser ausgehärtet. Hierbei ist auf eine möglichst gute planparallele Orientierung der Plättchen im Anwendungsmedium zu achten. Hierzu können die Pigmente zuvor durch geeignete Additive vorbehandelt werden. Anschließend wird der ausgehärtete Lack angeschliffen und nach üblicher Probenpräparation der Querschliff im REM betrachtet. Für die Bestimmung werden nur Teilchen ausgewählt, die eine gute Orientierung aufweisen. Schlecht orientierte Plättchen ergeben bei dieser Methode einen hohen Fehler aufgrund des unbekannten Betrachtungswinkels. Die Beschichtungen weisen einen sehr guten Kontrast zum Metallkern auf. Sollte man die Schichtdicken der Metalloxid- und der organischen Polymerschicht nicht gut unterscheiden können, so kann man örtlich aufgelöste EDX-Analysen verwenden, bevor die Schichtdicken vermessen werden. Der Begriff "mittlere Schichtdicke" im Sinne der Erfindung bezeichnet das arithmetische Mittel der Schichtdicken der Schichten von mindestens 20 Pigmenten. Sofern die Beschichtung ungleichmäßig ist, so wird das arithmetische Mittel der dünnsten und der dicksten Stelle der Beschichtung des jeweiligen Partikels gebildet. Einzelne gravierende Abweichungen, die beispielsweise auf dem Einschluss bereits beschichteter Feinteilpigmente in die Beschichtung beruhen, werden nicht bei der Berechnung der mittleren Schichtdicke berücksichtigt.

Der Gehalt an Metalloxid kann über eine Elementanalyse erfolgen. So kann beispielsweise im Fall einer SiO2-Schicht der Si-Gehalt bestimmt und auf $SiO_2$ hochgerechnet werden.

Besonders vorteilhafte Eigenschaftskombinationen der erfindungsgemäßen Pigmente, die ein breites Anwendungsspektrum bei hervorragenden optischen Qualitäten ermöglichen, können durch Abstimmung der Menge der wenigstens einen von Aluminiumoxid verschiedenen Metalloxidschicht zur wenigstens einen organischen Polymerschicht erzielt werden. Bei weiteren Ausführungsformen ist es beispielsweise bevorzugt, dass die Summe der wenigstens einen von Aluminiumoxid verschiedenen Metalloxidschicht und der wenigstens einen organischen Polymerschicht in dem Bereich von 10 bis 50 Gew.-%, vorzugsweise in dem Bereich von 13 bis 40 Gew.-%, mehr bevorzugt in dem Bereich von 14 bis 35 Gew.-%, noch mehr bevorzugt in dem Bereich von 15 bis 33 Gew.-% und am meisten bevorzugt in dem Bereich von 16 bis 29 Gew.-% liegt, jeweils bezogen auf das Gesamtgewicht der unbeschichteten, nasschemisch oxidierten Aluminiumeffektpigmente.

Ferner hat sich gezeigt, dass es für viele Anwendungen vorteilhaft ist, dass mehr organisches Polymermaterial als von Aluminiumoxid verschiedenes Metalloxid aufgebracht wird. Insbesondere ist es bei weiteren Ausführungsformen vorteilhaft, dass das Gewichtsverhältnis der wenigstens einen von Aluminiumoxid verschiedenen Metalloxidschicht zur wenigstens einen organischen Polymerschicht in einem Bereich von 1:2 bis 1:20, vorzugsweise in einem Bereich von 1:2,2 bis 1:17, weiter bevorzugt in einem Bereich von 1:2,5 bis 1:15 und noch weiter bevorzugt in einem Bereich von 1:2,7 bis 1:13 und am meisten bevorzugt in einem Bereich von 1:3 bis 1:10 auf, wobei die jeweils zugrundeliegenden Gewichtsprozent auf das Gesamtgewicht der unbeschichteten, nasschemisch oxidierten Aluminiumeffektpigmente bezogen sind.

Insbesondere ist es bei weiteren der vorgenannten Ausführungsformen vorteilhaft, dass die Summe der wenigstens einen von Aluminiumoxid verschiedenen Metalloxidschicht und der wenigstens einen organischen Polymerschicht in dem Bereich von 10 bis 50 Gew.-% und ein Gewichtsverhältnis der wenigstens einen von Aluminiumoxid verschiedenen Metalloxidschicht zur wenigstens einen organischen Polymerschicht in einem Bereich von 1:2 bis 1:20 aufweisen. Vorzugsweise liegt bei weiteren der vorgenannten Ausführungsformen die Summe der wenigstens einen von Aluminiumoxid verschiedenen Metalloxidschicht und der wenigstens einen organischen Polymerschicht in dem Bereich von 13 bis 40 Gew.-% und das Gewichtsverhältnis der wenigstens einen von Aluminiumoxid verschiedenen Metalloxidschicht zur wenigstens einen organischen Polymerschicht in einem Bereich von 1:2,2 bis 1:17. Bei weiteren mehr bevorzugten Ausführungsformen liegt die Summe der wenigstens einen von Aluminiumoxid verschiedenen Metalloxidschicht und der wenigstens einen organischen Polymerschicht in dem Bereich von 14 bis 35 Gew.-% und das Gewichtsverhältnis der wenigstens einen von Aluminiumoxid verschiedenen Metalloxidschicht zur wenigstens einen organischen Polymerschicht in einem Bereich von 1:2,5 bis 1:15. Bei weiteren noch mehr bevorzugten Ausführungsformen liegt die Summe der wenigstens einen von Aluminiumoxid verschiedenen Metalloxidschicht und der wenigstens einen organischen Polymerschicht in dem Bereich von 15 bis 33 Gew.-% und das Gewichtsverhältnis der wenigstens einen von Aluminiumoxid verschiedenen Metalloxidschicht zur wenigstens einen organischen Polymerschicht in einem Bereich von 1:2,7 bis 1:13.

Die vorgenannten Gewichtsverhältnisse sowie die Gew.-% beziehen sich auf das Gewicht der jeweiligen Beschichtungen bezogen auf das Gesamtgewicht der unbeschichteten, nasschemisch oxidierten Aluminiumeffektpigmente.

Zwischen der wenigstens einen von Aluminiumoxid verschiedenen Metalloxidschicht und der wenigstens einen organischen Polymerschicht oder können ein oder mehrere weitere Beschichtungen aufgebracht sein. Ferner können auch auf der organischen Polymerschicht ein oder mehrere weitere Beschichtungen, vorzugsweise umhüllend, aufgebracht sein. Selbstverständlich können auch zwischen der wenigstens einen von Aluminiumoxid verschiedenen Metalloxidschicht und der wenigstens einen organischen Polymerschicht oder können ein oder mehrere weitere Beschichtungen aufgebracht sein und auch auf der organischen Polymerschicht ein oder mehrere weitere Beschichtungen, vorzugsweise umhüllend, aufgebracht sein.

So kann die wenigstens eine organische Polymerschicht beispielsweise von einer weiteren Metalloxidschicht umhüllt sein. Insbesondere ist es bei weiteren Ausführungsformen jedoch bevorzugt, dass zusätzlich wenigstens eine Schicht eines Haftvermittlers und/oder einer anderen Schichtkomponente zwischen der wenigstens einen von Aluminiumoxid verschiedenen Metalloxidschicht und der wenigstens einen organischen Polymerschicht vorhanden ist. Die vorgenannten Zwischenschichten können beispielsweise aus organofunktionellen Silanen, Titanaten, Aluminaten, Phosphonsäuren (z.B. VPS: Vinylphosphonsäure), Phorsphorsäureester, Zirkonaten und Mischungen davon erzeugt worden sein. Mithin können derartige Beschichtungen beispielsweise aufgrund bekannter Hydrolyse- und Kondensationsreaktionen besonders gut auf der Metalloberfläche bzw. der Metalloxidoberfläche anbinden und gleichzeitig über eine polymerisierbare Gruppe, wie beispielsweise eine polymerisierbare Doppelbindung, vorzugsweise mindestens eine Acrylat- und/oder Methacrylatgruppe, an die organische Polymerschicht anbinden. Hierbei ist es wichtig, die polymerisierbare Gruppe an das organische Polymer anzupassen, um eine optimale Anbindung zu ermöglichen. So ist es beispielsweise vorteilhaft eine derartige Zwischenbeschichtung derart auszuwählen, dass sie radikalisch polymerisierbare Gruppen bereitstellt, wenn die organische Polymerschicht mittels radikalischer Polymerisation erzeugt wird.

Besonders bevorzugte Haftvermittler und/oder zusätzliche Schichtkomponenten sind organofunktionelle Silane. Beispielsweise können acrylat- und/oder methacrylathaltige Silane, insbesondere (Methacryloxymethyl)methyldimethoxysilan, Methacryloxymethyltrimethoxysilan, (Methacryloxymethyl)methyldiethoxysilan, Methacryloxymethyltriethoxysilan, 2-Acryloxyethylmethyldimethoxysilan, 2-Methacryloxyethyltrimethoxysilan, 3-Acryloxypropylmethyldimethoxysilan, 2-Acryloxyethyltrimethoxysilan, 2-Methacryloxyethyltriethoxysilan, 3-Acryloxypropyltrimethoxysilan, 3-Acryloxypropyltripropoxysilan, 3-Methacryloxypropyltriethoxysilan, 3-Methacryloxypropyltrimethoxysilan, 3-Methacryloxypropyltriacetoxysilan, 3-Methacryloxypropymethyldimethoxysilan, Vinyltrichlorsilan, Vinyltrimethoxysilan Vinyldimethoxymethylsilan, Vinyltriethoxysilan, Vinyltris(2-methoxyethoxy)silan, Vinyltriacetoxysilan oder Mischungen davon verwendet werden.

Bei besonderen Ausführungsformen der vorliegenden Erfindung ist die vorgenannte Zwischenschicht keine separate haftvermittelnde Beschichtung, sondern zumindest teilweise auch in die Kunststoffschicht einpolymerisiert, wie in der WO 2008/095697 A1 beschrieben, die hiermit unter Bezugnahme aufgenommen ist.

Zudem kann eine hochbrechende Metallchalkogenidschicht auf die wenigstens eine Metalloxidschicht und/oder die wenigstens eine organische Polymerschicht aufgebracht werden, um beispielsweise einen spezifischen Farbton mit einem weichen Farbflop hervorzurufen. Der Begriff "hochbrechend" im Sinne der vorliegenden Erfindung beschreibt einen Brechungsindex von mindestens 1,95, vorzugsweise von mindestens 2,1 und mehr bevorzugt von mindestens 2,2. Insbesondere ist es bei weiteren Ausführungsformen bevorzugt, dass die hochbrechende Metallchalkogenidschicht auf die erfindungsgemäße wenigstens eine Metalloxidschicht aufgebracht wird.

Hochbrechende Metallchalkogenide im Sinne der vorliegenden Erfindung werden bevorzugt ausgewählt aus der Gruppe bestehend aus Metalloxiden, Metallsulfiden, Metallseleniden, Metalltelluriden und Mischungen davon. Insbesondere ist es bei weiteren Ausführungsformen bevorzugt, dass das hochbrechende Metallchalkogenid ein hochbrechendes Metalloxid ist.

Bei weiteren Ausführungsformen ist es insbesondere bevorzugt, dass es sich bei den hochbrechenden Metallchalkogeniden um farbige, hochbrechende Metallchalkogenide handelt. Beispiele für farbige, hochbrechende Metallchalkogenide sind Eisenoxide, Eisenoxidhydrate, Vanadiumoxide, Wolframoxide, Chromoxide, Molybdänsulfid und Mischungen davon. Bei weiteren Ausführungsformen ist es insbesondere bevorzugt, dass das hochbrechende Metallchalkogenid Eisenoxid ist, insbesondere ausgewählt aus der Gruppe bestehend aus Hämatit, Goethit, Magnetit oder Mischungen davon. Beispielsweise zur Bereitstellung von Pigmenten zur Verwendung im Gold-Rot-Bereich sind insbesondere rote Eisenoxidschichten, welche im Wesentlichen aus der Hämatitmodifikation bestehen, bevorzugt. Zur Bereitstellung grüner Pigmente ist es bei weiteren Ausführungsformen hingegen bevorzugt Chrom(III)hydroxid als hochbrechendes Metallchalkogenid aufzubringen.

Bei anderen Ausführungsformen, bei denen beispielsweise die Erzeugung bestimmter Interferenzeffekte im Vordergrund steht, ist es hingegen bevorzugt, dass das hochbrechende Metallchalkogenid mit einem niedrigbrechenden Metallchalkogenid kombiniert wird. Hierbei weist das niedrigbrechende Metallchalkogenid bei weiteren Ausführungsformen vorzugsweise keine Farbe auf, da es lediglich zur Erzeugung eines Interferenzeffekts dient. Beispiele derartiger farbloser, hochbrechender Metallchalkogenide sind Titanoxid, Zirkonoxid, Zinkoxid, Zinnoxid, Ceroxid und Mischungen davon.

Bei weiteren der vorgenannten Ausführungsformen umfasst das erfindungsgemäße Pigment mindestens eine hochbrechende Metallchalkogenidschicht und mindestens eine niedrigbrechende Metallchalkogenidschicht. Der Begriff "niedrigbrechend" im Sinne der vorliegenden Erfindung beschreibt einen Brechungsindex von höchstens 1,8, vorzugsweise höchstens 1,7, mehr bevorzugt höchstens 1,65 und noch mehr bevorzugt höchstens 1,6. Es ist insbesondere bevorzugt, dass die Beschichtung nicht zu dick ist, damit ein ausreichendes Deckvermögen erzielt werden kann. Bei weiteren Ausführungsformen ist es daher bevorzugt, dass das erfindungsgemäße Pigment höchstens 4 Schichtpakete, mehr bevorzugt höchstens 3 Schichtpakete, noch mehr bevorzugt höchstens 2 Schichtpakete und am meisten bevorzugt höchstens ein Schichtpaket bestehend aus einer Schicht aus wenigstens einem hochbrechenden Metallchalkogenid und einer Schicht aus wenigstens einem niedrigbrechenden Metallchalkogenid umfasst. Insbesondere ist es bei weiteren Ausführungsformen bevorzugt, dass die vorgenannten Schichtpakete aus wenigstens einer Schicht eines farbigen, hochbrechenden Metallchalkogenids und wenigstens einer Schicht eines farblosen, niedrigbrechenden Metallchalkogenids bestehen. Die Begriffe "farblos" und "farbig" beziehen sich auf das makroskopische Erscheinungsbild der jeweiligen Stoffe, wobei eine weißliche, gräuliche oder schwärzliche Trübung oder ein weißes, graues oder schwarzes Erscheinungsbild bei größerer Schichtdicke nicht als Farbe im Sinne der Erfindung verstanden wird.

Für die nasschemische Oxidation werden die beispielsweise in einer Kugelmühle vermahlenen Aluminiumpartikel ohne Zwischenschaltung eines Entfettungsschrittes eingesetzt. Eine spezielle Ausführungsform eines derartigen Verfahrens ist beispielsweise in der US 5,964,936 A beschrieben. Die nasschemische Oxidation der Aluminiumeffektpigmente erfolgt in einem Gemisch mit oder aus wenigstens einem wassermischbaren Lösungsmittel, Wasser und optional einer Säure oder Base. Bei der Verwendung einer Säure wird der pH-Wert des Reaktionsgemisches bevorzugt zwischen 3 und 7 eingestellt. Bei der Verwendung einer Base wird der pH-Wert des Reaktionsgemisches bevorzugt zwischen 7 und 12 eingestellt. Die Bestimmung des pH-Wertes erfolgt hierbei beispielsweise mit einem pH-Wert Messgerät des Typs sympHony der Firma VWR. Der Wasseranteil, bezogen auf die Gesamtmenge des wenigstens einen wassermischbaren Lösungsmittels und des Wassers, beträgt hierbei bis zu 63 Gew.-%, vorzugsweise bis zu 59 Gew.-%, mehr bevorzugt bis zu 55 Gew.-%. Insbesondere ist es bei weiteren Ausführungsformen bevorzugt, dass der vorgenannte Wasseranteil in einem Bereich von 3 bis 42 Gew.-%, vorzugsweise in einem Bereich von 6 bis 33 Gew.-%, mehr bevorzugt in einem Bereich von 8 bis 31 Gew.-% liegt.

Die Wassergesamtmenge wird hierbei so gewählt, dass die Wassermenge in einem Bereich von 10 bis 150 Gew.-% liegt, bezogen auf das Gewicht der eingesetzten Aluminiumpartikel. Insbesondere ist es bei weiteren Ausführungsformen bevorzugt, dass die vorgenannte Gesamtwassermenge in einem Bereich von 13 bis 130 Gew.-%, vorzugsweise in einem Bereich von 16 bis 115 Gew.-%, mehr bevorzugt in einem Bereich von 18 bis 95 Gew.-% und am meisten bevorzugt in einem Bereich von 20 bis 80 Gew.-% liegt, jeweils bezogen auf das Gewicht der eingesetzten Aluminiumeffektpigmente.

Der Begriff "wassermischbare Lösungsmittel" im Sinne der vorliegenden Erfindung bezieht sich auf Lösungsmittel, die unter Standardbedingungen (25 °C, 1013,25 hPa) in den für die Reaktion geplanten Reaktionsbedingungen der sich verringernden Wassermenge keine Mehrphasensysteme ausbilden. Beispiele sind Alkohole wie Ethanol, n-Propanol, i-Propanol, n-Butanol, i-Butanol, Methoxypropanol, Glykole, Aceton oder Butylglykol. Es ist verständlich, dass gegebenenfalls in dem Lösungsmittel enthaltenes Wasser bei der vorgenannten in der nasschemischen Oxidation eingesetzten Wassermenge eingerechnet werden muss. Die Bestimmung der im Lösungsmittel enthaltenen Wassermenge kann beispielsweise mittels der Karl-Fischer-Titration erfolgen.

Die Base zur vorgenannten Verwendung in der nasschemischen Oxidation wird vorzugsweise ausgewählt aus der Gruppe bestehend aus aliphatischen oder aromatischen Aminen, Methanolaminen, Ethanolaminen und anorganischen Basen, z. B. Triethylamin, n-Butylamin, i-Butylamin, Dimethanolamin, Dietylamin, Pyridin, Ethylendiamin, Diethanolamin, Triethanolamin, Diisopropylethylamin, Ammoniak, Hydrazin, Natriumhydroxid, Kaliumhydroxid, Ammoniumhydroxid, Ammoniumcarbonat, Ammoniumhydrogencarbonat, Natriumcarbonat, Natriumhydrogencarbonat, Kaliumcarbonat, Kaliumhydrogencarbonat und/oder Natriumacetat.

Die Säure zur vorgenannten Verwendung in der nasschemischen Oxidation wird vorzugsweise ausgewählt aus der Gruppe bestehend aus Phosphorsäure, Phosphonsäure, Schwefelsäure, Salzsäure, Essigsäure und Salpetersäure. Der Begriff "Phosphorsäure" im Sinne der vorliegenden Erfindung umfasst daneben auch Phosphorsäurehalbester

Die Reaktionstemperatur der nasschemischen Oxidation liegt bevorzugt in einem Bereich zwischen 5 °C und 120 °C. Es muss verstanden werden, dass der Fachmann die jeweilige Obergrenze selbstverständlich anpasst, sofern das Reaktionsgemisch bereits bei tieferer Temperatur siedet. Insbesondere ist es bei weiteren Ausführungsformen bevorzugt, dass die Reaktionstemperatur zwischen 50 und 95 °C liegt.

Bei der nasschemischen Oxidation können eine große Zahl bereits bekannter Aluminiumeffektpigmente eingesetzt werden, deren Aluminiumgehalt mindestens 50 Gew.-%, vorzugsweise mindestens 60 Gew.-% und mehr bevorzugt mindestens 70 Gew.-% und noch mehr bevorzugt mindestens 80 Gew.-% beträgt. Bei weiteren Ausführungsformen ist es bevorzugt, dass der Aluminiumgehalt der Aluminiumeffektpigmente mindestens 99 Gew.-% und insbesondere bevorzugt mindestens 99,9 Gew.-% beträgt. Es muss verstanden werden, dass bei den vorgenannten Gew.-% eventuell vorhandene Spurenbestandteile von Verunreinigungen nicht mitberechnet werden. Der Begriff "Spurenbestandteile" im Sinne der vorliegenden Erfindung bezeichnet Bestandteile deren Menge unter 0,01 Gew.-% liegt. Bei weiteren Ausführungsformen beträgt der Gehalt an Aluminium in den Aluminiumeffektpigmenten mindestens 95 Gew.-%, da diese eine Farbskala von hellgold bis bronze aufweisen, weitgehend bis vollständig unabhängig von den zulegierten Metallen. Es kann jedoch auch bei weiteren Ausführungsformen bevorzugt sein, ein breiteres Farbspektrum bereitzustellen wie beispielsweise gelb-, grün-, rot- und rotbraunstichige Goldtöne bis hin zu dunkelbraun und schwarz. In diesem Zusammenhang haben sich beispielsweise Eisen, Mangan, Kupfer, Vanadium, Chrom, Nickel, Cobalt, Silizium, Magnesium, Zink oder Titan als nützliche Legierungsbestandteile erwiesen.

Das an der plättchenförmige Oberfläche von Metalleffektpigmenten einfallende Licht wird wie an einem Spiegel gerichtet reflektiert. Daher sind plättchenförmige Metallpigmente besonders geeignet, um einen hohen Glanz bei den mit den erfindungsgemäßen Aluminiumeffektpigmenten beschichteten Gegenständen zu erzielen. Die erfindungsgemäßen plättchenförmigen Pigmente sind somit Aluminiumeffektpigmente.

Die erfindungsgemäßen Aluminiumeffektpigmente weisen einen mittleren Pigmentdurchmesser ($D_{50}$) aus einem Bereich von etwa 1 $\mu$m bis etwa 180 $\mu$m, vorzugsweise von etwa 3 $\mu$m bis etwa 160 $\mu$m, noch weiter bevorzugt von etwa 4 $\mu$m bis etwa 120 $\mu$m, auf. Als sehr geeignet haben sich auch Pigmentdurchmesser aus einem Bereich von etwa 5 $\mu$m bis etwa 90 $\mu$m, vorzugsweise von etwa 6 $\mu$m bis etwa 80 $\mu$m, erwiesen. Der Begriff "$D_{50}$" im Sinne der vorliegenden Erfindung bezeichnet die Partikelgröße, bei der 50 % der vorgenannten mittels Lasergranulometrie volumengemittelten Partikelgrößenverteilung unterhalb des angegebenen Wertes liegen. Die Messungen können beispielsweise mit dem Partikelgrößenanalysator HELOS der Fa. Sympatec GmbH, Clausthal-Zellerfeld, Deutschland, durchgeführt werden. Die Dispergierung eines trockenen Pulvers kann hierbei mit einer Dispergiereinheit vom Typ Rodos T4.1 bei einem Primärdruck von beispielsweise 4 bar erfolgen. Alternativ kann Größenverteilungskurve der Partikel beispielsweise mit einem Gerät der Fa. Quantachrome (Gerät: Cilas 1064) gemäß Herstellerangaben vermessen werden. Hierzu werden 1,5 g des pulverförmigen Beschichtungsmaterials in ca. 100 ml Isopropanol dispergiert, 300 Sekunden im Ultraschallbad (Gerät: Sonorex IK 52, Fa. Bandelin) behandelt und anschließend mittels einer Pasteurpipette in die Probenvorbereitungszelle des Messgerätes gegeben und mehrmals vermessen. Aus den einzelnen Messergebnissen werden die resultierenden Mittelwerte gebildet. Die Auswertung der Streulichtsignale erfolgt dabei nach der Fraunhofer Methode.

Ferner ist es bevorzugt, dass die mittlere Dicke ($h_{50}$) der erfindungsgemäßen, beschichteten Aluminiumeffektpigmente vorzugsweise in einem Bereich von etwa 150 nm bis etwa 2,5 $\mu$m, vorzugsweise von etwa 170 nm bis etwa 2,1 $\mu$m, noch weiter bevorzugt von etwa 185 nm bis etwa 1,6 $\mu$m, noch weiter bevorzugt von etwa 200 nm bis etwa 1,4 $\mu$m liegt. Der Begriff "mittlere Dicke" oder "$h_{50}$" im Sinne der Erfindung bezieht sich auf das arithmetische Mittel der Dicken von mindestens 50 Aluminiumeffektpigmenten, die mittels der Rasterelektronenmikroskopie (REM) bestimmt wurden, wobei auf eine möglichst gute Orientierung der erfindungsgemäßen Aluminiumeffektpigmente im Anwendungsmedium zu achten ist. Hierzu können die Metalleffektpigmente zuvor durch geeignete Additive vorbehandelt werden. Anschließend wird das ausgehärtete Anwendungsmedium angeschliffen und nach üblicher Probenpräparation der Querschliff im REM betrachtet. Für die Zählung werden nur Teilchen ausgewählt, die eine gute Orientierung aufweisen. Die mittlere Dicke oder der $h_{50}$-Wert bezieht sich dabei auf das erfindungsgemäße Aluminiumeffektpigment.

Weiterhin weisen die erfindungsgemäßen Aluminiumeffektpigmente bei weiteren bevorzugten Ausführungsformen eine über Dickenauszählung mit Rasterelektronenmikroskopie (REM) ermittelte relative Breite der Dickenverteilung $\Delta h$, welche anhand der entsprechenden Summendurchgangskurve der relativen Häufigkeit nach der Formel

$$\Delta h = (h_{90} - h_{10}) / h_{50}$$

berechnet wird, von 0,3 bis 0,9, bevorzugt von 0,35 bis 0,85 und besonders bevorzugt von 0,4 bis 0,8, auf.

Für spezifische Anwendungen mit besonderen Anforderungen kann es jedoch auch bevorzugt sein PVD-Aluminiumeffektpigmente einzusetzen. Aufgrund der deutlich höheren Preise derartiger Pigmente ist es jedoch meist bevorzugt, dass die im erfindungsgemäßen Verfahren eingesetzten Pigmente durch Vermahlung erhalten wurden. Mithin enthalten die erfindungsgemäßen Aluminiumeffektpigmente bevorzugt ein durch Vermahlung erhaltenes Aluminiumeffektpigment.

Bevorzugt werden somit bei der nasschemischen Oxidation durch Vermahlung von Aluminiumgrieß erhaltene Aluminiumeffektpigmente eingesetzt.

Der Aluminiumgrieß kann beispielsweise eine Größenverteilung mit einem $D_{Grieß,50}$ von 1 bis 210 μm und einem $D_{Grieß,90}$ von 2 bis 450 μm aufweisen. Nach der Vermahlung kann eine Klassierung erforderlich sein, um die gewünschte plättchenförmige Aluminiumeffektpigmentfraktion zu erhalten. Für die Zusammensetzung des eingesetzten Aluminiumgrießes, d.h. die Reinheit und/oder Legierungsbestandteilen von Aluminiumlegierungen wird auf die vorstehenden Ausführungen im Zusammenhang mit den in der nasschemischen Oxidation eingesetzten Aluminiumeffektpigmenten verwiesen. Diese Offenbarung gilt selbstverständlich auch für den zur Herstellung der Aluminiumeffektpigmente zu verwendenden Grieß.

Die Vermahlung von Aluminiumgrieß erfolgt überwiegend als Nassvermahlung. Hierbei wird der Aluminiumgrieß in Kugelmühlen vorzugsweise in mehreren Mahlstufen bei unterschiedlichen Mahlbedingungen, wie beispielsweise Mühlengröße, -durchmesser, -drehgeschwindigkeit, Kugelgröße, Mahldauer unter Zugabe von Schmiermittel, wie beispielsweise Stearin- oder Ölsäure, zur Verhinderung einer Kaltverschweißung der Aluminiumeffektpigmente, einem Lösungsmittel und mit Mahlkörpern, wie z. B. Stahlkugeln, vermahlen. Als Lösungmittel können beispielsweise Testbenzin oder Solvent Naphtha eingesetzt werden. Auch die Verwendung von Alkoholen, wie z.B. Isopropanol, Ketone, Ester, Ether usw. ist möglich. Die unbeschichteten Aluminiumeffektpigmente werden nach dem Vermahlen und optionalen Klassieren in verschiedenen Behältnissen gesammelt und anschließend homogenisiert bzw. gemischt.

Weitere Informationen zu einem hierbei einsetzbaren Vermahlungsverfahren finden sich in der WO 2009/152941 A2, auf deren Offenbarung hiermit in ihrer Gesamtheit Bezug genommen wird.

Der Begriff "Formfaktor" im Sinne der vorliegenden Erfindung bezeichnet das Größen-Dicken-Verhältnis oder auch Aspektverhältnis. Es errechnet sich aus dem Verhältnis des $D_{50}$-Wertes zu dem $h_{50}$-Wert. Bei weiteren Ausführungsformen liegt der Formfaktor in einem Bereich von etwa 1500:1 bis etwa 2,5:1, bevorzugt von etwa 900:1 bis etwa 20:1, noch weiter bevorzugt in einem Bereich von etwa 700:1 bis etwa 30:1.

Ferner ist es möglich, die Farbeigenschaften der erfindungsgemäßen Pigmente durch gezielte Einfärbung der nasschemisch erzeugten Aluminiumoxidschicht zu variieren. Hierbei wird die Hydrolysereaktion, vorzugsweise der Sol-Gel-Prozess, zur Aufbringung der von Aluminiumoxid verschiedenen Metalloxidschicht, vorzugsweise Siliziumoxidschicht, in Gegenwart von Farbpigmenten durchgeführt. Durch die gezielte Herstellung einer Metalloxidschicht, in der entsprechende Farbpigmente eingeschlossen sind, können verschiedenste Farbtöne wie z.B. blau, rot und violett erzeugt werden.

Die Menge an Farbpigment beträgt hierbei vorzugsweise 5 bis 40 Gew.-% bezogen auf das Gewicht der nichtoxidierten Aluminiumeffektpigmente. Als Farbpigmente können organische Farbpigmente, anorganische Farbpigmente oder Mischungen davon eingesetzt werden. Bevorzugt sind Farbpigmente, die sich durch eine hohe Farbechtheit auszeichnen. Beispiele von Farbpigmenten, die für den erfindungsgemäßen Einsatz geeignet sind, sind C.I. Pigment Red 202C.I., C.I. Pigment Red 179, C.I. Pigment Red 101 und Pigment Blue 15 : 3.

Es hat sich bei bestimmten Ausführungsformen ferner als vorteilhaft erwiesen, ein Additiv zur Verbesserung der Pigmentdispergierbarkeit wie beispielsweise Antiterra U 80 der Fa. Byk-Chemie zuzugeben.

Bei weiteren Ausführungsformen, bei denen beispielsweise die Stabilität der von Aluminiumoxid verschiedenen Metalloxidschicht im Vordergrund steht, ist es bevorzugt, dass während der Aufbringung der von Aluminiumoxid verschiedenen Metalloxidschicht keine größeren Mengen an Farbpigmenten in der Reaktionslösung vorhanden sind. Insbesondere ist es bei weiteren Ausführungsformen bevorzugt, dass die entsprechenden Reaktionsgemische und nachfolgend die von Aluminiumoxid verschiedenen Metalloxidschichten der erfindungsgemäßen nasschemisch oxidierten Aluminiumeffektpigmente im Wesentlichen keine Farbpigmente enthalten, vorzugsweise weniger als 1 Gew.-% Farbpigmente, mehr bevorzugt weniger als 0,1 Gew.-% Farbpigmente und noch mehr bevorzugt weniger als 0,01 Gew.-% Farbpigmente, jeweils bezogen auf das Gewicht der eingesetzten nasschemisch oxidierten Aluminiumeffektpigmente. Insbesondere ist es bei weiteren der vorgenannten Ausführungsformen bevorzugt, dass während der Aufbringung der von Aluminiumoxid verschiedenen Metalloxidschicht keine Farbpigmente vorhanden sind bzw. die erfindungsgemäß aufzubringende von Aluminiumoxid verschiedene Metalloxidschicht keine Farbpigmente enthält. Ohne dass es als Einschränkung der Erfindung verstanden werden darf, ist es die Ansicht der Erfinder, dass derartige zusätzliche kleine Partikel das Eindringen der zur Herstellung der Metalloxidschicht verwendeten Edukte in die Poren der nasschemisch erzeugten Aluminiumoxidschicht behindern und die Ausbildung einer qualitativ verschlechterten Metalloxidschicht möglich sein kann.

Gemäß einer Variante der Erfindung ist es beispielsweise bevorzugt; dass die nasschemisch oxidierten Aluminiumeffektpigmente wenigstens eine von Aluminiumoxid verschiedene Metalloxidschicht und wenigstens eine umhüllende organische Polymerschicht aufweisen; dass das Gewichtsverhältnis des als Metalloxidschicht aufgebrachten von Aluminiumoxid verschiedenen Metalloxids zu der nasschemisch erzeugten Aluminiumoxidschicht in einem Bereich von 1:1 bis 1:40 liegt; dass die Summe der wenigstens einen von Aluminiumoxid verschiedenen Metalloxidschicht und der wenigstens einen organischen Polymerschicht in dem Bereich von 10 bis 50 Gew.-%, bezogen auf das Gesamtgewicht der unbeschichteten, nasschemisch oxidierten Aluminiumeffektpigmente, liegt; und dass das Gewichtsverhältnis der wenigstens einen von Aluminiumoxid verschiedenen Metalloxidschicht zur wenigstens einen organischen Polymerschicht in einem Bereich von 1:2 bis 1:20 liegt. Bei bestimmten Ausführungsformen der vorgenannten Variante ist es insbesondere bevorzugt, dass die Menge der wenigstens einen von Aluminiumoxid verschiedenen Metalloxidschicht in einem Bereich von 0,8 Gew.-% bis 20 Gew.-%, bezogen auf das Gesamtgewicht der unbeschichteten, nasschemisch oxidierten Aluminiumeffektpigmente, liegt; und dass die wenigstens eine von Aluminiumoxid verschiedene Metalloxidschicht der erfindungsgemäßen Aluminiumeffektpigmente im Wesentlichen aus Metalloxid bestehen, das ausgewählt wird aus der Gruppe bestehend aus Siliziumoxid, Boroxid, Zirkoniumoxid, Titanoxid, deren Oxidhydraten, deren Hydroxiden und Mischungen davon.

Gemäß einer weiteren Variante der Erfindung ist es beispielsweise bevorzugt; dass die nasschemisch oxidierten Aluminiumeffektpigmente wenigstens eine von Aluminiumoxid verschiedene Metalloxidschicht und wenigstens eine umhüllende organische Polymerschicht aufweisen; dass das Gewichtsverhältnis des als Metalloxidschicht aufgebrachten von Aluminiumoxid verschiedenen Metalloxids zu der nasschemisch erzeugten Aluminiumoxidschicht in einem Bereich von 1:2 bis 1:25 liegt; dass die Summe der wenigstens einen von Aluminiumoxid verschiedenen Metalloxidschicht und der wenigstens einen organischen Polymerschicht in dem Bereich von 10 bis 50 Gew.-%, bezogen auf das Gesamtgewicht der unbeschichteten, nasschemisch oxidierten Aluminiumeffektpigmente, liegt; und dass das Gewichtsverhältnis der wenigstens einen von Aluminiumoxid verschiedenen Metalloxidschicht zur wenigstens einen organischen Polymerschicht in einem Bereich von 1:2 bis 1:20 liegt. Bei bestimmten Ausführungsformen der vorgenannten Variante ist es insbesondere bevorzugt; dass der mittlere Pigmentdurchmesser ($D_{50}$) der erfindungsgemäßen Aluminiumeffektpigmente in einem Bereich von 1 $\mu$m bis 180 $\mu$m; dass die mittlere Dicke ($h_{50}$) der erfindungsgemäßen Aluminiumeffektpigmente in einem Bereich von 150 nm bis 2,5 $\mu$m; und dass der Formfaktor der erfindungsgemäßen Aluminiumeffektpigmente in einem Bereich von etwa 1500:1 bis etwa 2,5:1 liegt.

Gemäß beispielsweise einer Variante der Erfindung ist es bevorzugt; dass die nasschemisch oxidierten Aluminiumeffektpigmente wenigstens eine von Aluminiumoxid verschiedene Metalloxidschicht und wenigstens eine umhüllende organische Polymerschicht aufweisen; dass das Gewichtsverhältnis des als Metalloxidschicht aufgebrachten von Aluminiumoxid verschiedenen Metalloxids zu der nasschemisch erzeugten Aluminiumoxidschicht in einem Bereich von 1:1 bis 1:40 liegt; dass die Summe der wenigstens einen von Aluminiumoxid verschiedenen Metalloxidschicht und der wenigstens einen organischen Polymerschicht in dem Bereich von 10 bis 50 Gew.-% liegt; dass das Gewichtsverhältnis der wenigstens einen von Aluminiumoxid verschiedenen Metalloxidschicht zur wenigstens einen organischen Polymerschicht in einem Bereich von 1:2 bis 1:20 liegt; dass die Menge der wenigstens einen von Aluminiumoxid verschiedenen Metalloxidschicht in einem Bereich von 0,8 Gew.-% bis 20 Gew.-% liegt; und dass die Menge der wenigstens einen organischen Polymerschicht der erfindungsgemäßen Aluminiumeffektpigmente in einem Bereich von 8 bis 40 Gew.-% liegt; wobei die vorgenannten Gew-% auf das Gesamtgewicht der unbeschichteten, nasschemisch oxidierten Aluminiumeffektpigmente bezogen sind.

Gemäß beispielsweise einer Variante der Erfindung ist es bevorzugt; dass die nasschemisch oxidierten Aluminiumeffektpigmente wenigstens eine von Aluminiumoxid verschiedene Metalloxidschicht und wenigstens eine umhüllende organische Polymerschicht aufweisen; dass das Gewichtsverhältnis des als Metalloxidschicht aufgebrachten von Aluminiumoxid verschiedenen Metalloxids zu der nasschemisch erzeugten Aluminiumoxidschicht in einem Bereich von 1:1 bis 1:40 liegt; dass die Dicke der nasschemisch erzeugten Aluminiumoxidschicht in einem Bereich von 45 nm bis 210 nm liegt; und der Gehalt an elementarem Aluminium der erfindungsgemäßen Aluminiumeffektpigmente in einem Bereich von 31 bis 77 Gew.-%, bezogen auf das Gesamtgewicht der unbeschichteten, nasschemisch oxidierten Aluminiumeffektpigmente, liegt.

Ferner betrifft die vorliegende Erfindung die Verwendung der erfindungsgemäßen Aluminiumeffektpigmente, welche beispielsweise zum Einfärben verwendet werden können. So können die erfindungsgemäßen Aluminiumeffektpigmente beispielsweise in ein Beschichtungsmittel eingearbeitet werden. Beispiele von Beschichtungsmitteln sind Lacke, wie Coil-Coating-Lacke; Lackkonzentrate; Druckfarben; Druckfarbenkonzentrate; Farben; Farbkonzentrate; Pulverlacke oder Pulverlackkonzentrate. Ferner können die erfindungsgemäßen Aluminiumeffektpigmente in ein Material, wie beispielsweise einen Kunststoff, eingearbeitet werden. Hierbei können die erfindungsgemäßen Aluminiumeffektpigmente neben optischen auch funktionale Eigenschaften bereitstellen, beispielsweise als Lasermarkierungsadditiv in Kunststoffen dienen.

Zudem können die erfindungsgemäßen Pigmente beispielsweise in Kosmetika eingesetzt werden.

Die erfindungsgemäßen Aluminiumeffektpigmente können in Kombination mit für die jeweilige Anwendung geeigneten Roh-, Hilfs- und Wirkstoffen in kosmetischen Formulierungen Einsatz finden. Beispielsweise können die erfindungsgemäßen Aluminiumeffektpigmente in Körperpuder, Gesichtspuder, gepresstem und losem Puder, Gesichtsmakeup, Pudercreme, Crememakeup, Emulsionsmakeup, Wachsmakeup, Foundation, Moussemakeup, Wangenrouge, Augenmakeup wie Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haargel, Haarwachs, Haarmascara, permanente oder semi-permanente Haarfarben, temporäre Haarfarben, Hautpflegekompositionen wie Lotions, Gele, Emulsionen sowie Nagellackkompositionen eingesetzt werden. Hierbei liegt die Konzentration der erfindungsgemäßen Aluminiumeffektpigmente in der Formulierung vorzugsweise zwischen 0,001 Gew.-% für Rinse-off-Produkte und 40,0 Gew.-% für Leave-on-Produkte liegen.

Neben den Roh-, Hilfs- und Wirkstoffen können in den kosmetischen Formulierungen zudem auch weitere Farbmittel, herkömmliche Effektpigmente oder Mischungen hiervon, in variablen Mengenverhältnissen Verwendung finden. Beispielsweise können in Verbindung oder in Mischung mit den erfindungsgemäßen Aluminiumeffektpigmenten handelsübliche Perlglanzpigmente auf der Basis von mit hochbrechenden Metalloxiden beschichtetem natürlichen Glimmer (wie z.B. die Perlglanzpigmente der Produktgruppe Prestige der Fa. Eckart), BiOCI-Plättchen, Ti02-Plättchen, Perlglanzpigmente auf der Basis von mit hochbrechenden Metalloxiden beschichtetem synthetischen Glimmer oder basierend auf mit hochbrechenden Metalloxiden beschichteten Glasplättchen (wie z.B. die Perlglanzpigmente der Produktgruppe MIRAGE der Fa. Eckart), $Al_2O_3$-, Si02- oder $TiO_2$-Plättchen eingesetzt werden. Außerdem können auch Metalleffektpigmente, wie z.B. die Perlglanzpigmente der Produktgruppe Visionaire der Fa. Eckart, zugegeben werden. Ferner können beispielsweise die Farbmittel aus anorganischen oder organischen Pigmenten ausgewählt werden.

Bei weiteren Ausführungsformen ist es insbesondere bevorzugt, dass die erfindungsgemäßen Pigmente zur Verwendung in kosmetischen Formulierungen zusätzlich zur wenigstens einen Metalloxidschicht, insbesondere Silziumoxidschicht, eine hochbrechende Metallchalkogenidschicht, vorzugsweise eine hochbrechende Metalloxidschicht wie beispielsweise eine Eisenoxidschicht, aufweisen.

Die erfindungsgemäßen Pigmente können ferner in ein Beschichtungsmittel wie beispielsweise ein Lack, wie z.B. ein Coil-Coatinglack, ein Lackkonzentrat, eine Druckfarbe, ein Druckfarbenkonzentrat, eine Farbe, ein Farbkonzentrat, ein Pulverlack oder ein Pulverlackkonzentrat eingebracht werden.

Insbesondere für die Applikation als Pulverlack haben sich die erfindungsgemäßen Pigmente zusätzlich als sehr geeignet erwiesen, da erst mit der erfindungsgemäß aufzubringenden Beschichtung mit einem von Aluminiumoxid verschiedenen Metalloxid und einer umhüllenden Polymerschicht eine zufriedenstellende Applizierbarkeit der nasschemisch oxidierten Aluminiumeffektpigmente erzielt wurde, wie in dem erfindungsgemäßen Beispiel 1 veranschaulicht.

Ferner hat sich überraschenderweise gezeigt, dass ungeachtet der großen Wassermenge, die noch immer feinverteilt in beispielsweise einer mittels des Sol-Gel-Prozesses erzeugten Metalloxidschicht vorliegen sollte, die erfindungsgemäßen Pigmente mittels des Coil-Coating Verfahrens oder der Pulverlackbeschichtung aufgebracht und eingebrannt werden können, ohne dass durch eine Nachoxidation Blasen oder Störungen in der aufgebrachten Lackschicht entstehen.

Die erfindungsgemäßen Aluminiumefektpigmente sind besonders geeignet für beispielsweise Automobilkarosserien, Fassadenelemente, Drucksachen, wie beispielsweise bedruckte Folien, Papier, Kartonagen, Kunststoffformteile, etc.. Bevorzugt werden die erfindungsgemäßen Aluminiumeffektpigmente in Pulverlacken, Coil-Coating-Formulierungen, Wasserlacken und Polymeren oder Kunststoffen verwendet. Bei weiteren bevorzugten Ausführungsformen ist das Beschichtungsmittel ein Pulverlack oder ein Lack zur Verwendung im Coil-Coating-Verfahren.

Die Pulverlackbeschichtung ist ein vielseitiges und in der industriellen Fertigung oft eingesetztes, elektrostatisches Verfahren zur Beschichtung von Serienprodukten. Neben der Lösemittelfreiheit und der Möglichkeit der Wiederverwendung des Oversprays bietet das Verfahren zudem verschiedene Vorteile wie das Umgreifen des applizierten Pulvers. Der hierbei applizierte Pulverlack enthält Bindemittel, die erfindungsgemäßen Alumniumeffektpigmente, Füllstoffe und Vernetzer sowie optional Additive. Unter einem Bindemittel wird erfindungsgemäß die in der DIN 55 945 angegebene Definition verstanden. D.h., das Bindemittel umfasst sowohl den Filmbildner wie auch nicht flüchtige Hilfsstoffe wie Weichmacher und Trockenstoffe. Nach der Applikation des Pulverlacks erfolgt ein Härtungsschritt mittels Einbrennen oder Strahlungsenergie.

Die Herstellung entsprechender Pulverlacke kann auf verschiedenen Wegen erfolgen. Beim Mischverfahren wird das erfindungsgemäße Aluminiumeffektpigment bereits vor den Extrusions- und Vermahlungsprozessen zugegeben. Infolge der auftretenden Scherkräfte kann hierbei eine Beschädigung oder Zerstörung von insbesondere plättchenförmigen Pigmenten auftreten. Dies führt wiederum zu einer Beeinträchtigung des Glanzes und der Optik bei einer hiermit hergestellten Beschichtung.

Ferner können entsprechende Pulverlacke mittels des Dry-Blend-Verfahrens hergestellt werden. Hierbei werden die erfindungsgemäßen Aluminiumeffektpigmente erst nach der Extrusion und Vermahlung zugesetzt. Dies hat jedoch die Nachteile, dass eine Separation infolge unterschiedlicher elektrostatischer Aufladung während der Lackapplikation eine inhomogene Beschichtung hervorrufen kann. Ferner kann eine Ab- bzw. Anreicherung des Pigments sowohl beim Transport und der Lagerung als auch im Overspray auftreten, so dass ein erneutes Einsetzen dieses Materials verhindert wird. Daher ist es bevorzugt das Dry-Blend-Verfahren mit dem Bonding-Verfahren zu kombinieren. Hierbei wird das Pigment unter Erwärmen an die Partikel des Basislacks gebunden. Dieses Herstellverfahren liefert qualitativ höherwertige Pulverlacke mit beispielsweise guter Recyclebarkeit des Oversprays, ist jedoch verhältnismäßig kostenintensiv. Die kostengünstigsten Pulverlacke werden daher mittels des Mischverfahrens hergestellt.

Ein weiteres vielfach in der industriellen Fertigung eingesetztes Beschichtungsverfahren ist das Coil-Coating-Verfahren. Bei diesem umweltfreundlichen Verfahren kann durch eine optimierte Prozessführung ein Auftragswirkungsgrad von nahezu 100 % erzielt werden, während ansonsten bei den meisten Lackierverfahren beispielsweise größere Verluste durch das Overspray auftreten.

Weiterhin können die erfindungsgemäßen Aluminiumeffektpigmente in Kunststoffe eingearbeitet werden. Hierbei werden beispielsweise thermoplastische, duroplastische oder elastomere Kunststoffe eingesetzt. Insbesondere ist es bei weiteren Ausführungsformen bevorzugt, die erfindungsgemäßen Aluminiumeffektpigmente in thermoplastische Kunststoffe einzuarbeiten. Prinzipiell bestehen keine Einschränkungen hinsichtlich der dem Fachmann bekannten Thermoplaste. Informationen hierzu können verschiedensten Fachbüchern oder der Fachliteratur entnommen werden. Basierend auf dem jeweils geplanten Verwendungszweck können jedoch bestimmte Thermoplaste bevorzugt oder von geringerem Interesse sein.

Beispiele für geeignete Thermoplasten sind Polyoxyalkylene, Polycarbonate (PC), Polyester wie Polybutylenterephthalat (PBT) oder Polyethylenterephthalat (PET), Polyolefine wie Polyethylen oder Polypropylen (PP), Poly(meth)acrylate, Polyamide, vinylaromatische (Co)polymere wie Polystyrol, schlagzähmodifiziertes Polystyrol wie HIPS, oder ASA-, ABS- oder AES-Polymerisate, Polyarylenether wie Polyphenylenether (PPE), Polysulfone, Polyurethane, Polylactide, halogenhaltige Polymerisate, imidgruppenhaltige Polymere, Celluloseester, Silicon-Polymere oder thermoplastische Elastomere. Zudem ist es möglich, Mischungen unterschiedlicher Thermoplaste in Form sogenannter ein- oder mehrphasiger Polymerblends einzusetzen.

Polyoxyalkylenhomo- oder -copolymerisate, insbesondere (Co)polyoxymethylene (POM), sind im Handel erhältlich und werden beispielsweise unter der Markenbezeichnung Ultraform® (BASF SE, Deutschland) vertrieben. Ganz allgemein weisen diese Kunststoffe mindestens 50 Mol-% an wiederkehrenden Einheiten -$CH_2$O-in der Polymerhauptkette auf. Homopolymere, die als Kunststoffe bevorzugt sind, werden insbesondere durch Polymerisation von Formaldehyd oder Trioxan, vorzugsweise in der Gegenwart von entsprechenden Katalysatoren, erhalten. Bevorzugt sind Polyoxymethylencopolymere und Polyoxymethylenterpolymerisate. Die bevorzugten Polyoxymethylen(co)polymere haben Schmelzpunkte von mindestens 150°C und Molekulargewichte (Gewichtsmittelwert) M im Bereich von 5.000 bis 200.000, vorzugsweise von 7.000 bis 150.000 g/mol. Endgruppenstabilisierte Polyoxymethylenpolymerisate, die an den Kettenenden C-C-Bindungen aufweisen, werden besonders bevorzugt.

Polycarbonate können beispielsweise mittels Grenzflächenpolykondensation oder durch Umsetzung von Biphenylcarbonat mit Bisphenolen hergestellt werden. Hierbei kann als Bisphenol beispielsweise 2,2-Di(4-hydroxyphenyl)propan (Bisphenol A) eingesetzt werden. Sie sind kommerziell erhältlich und werden beispielsweise unter dem Handelsnamen Lexan® (GE Plastics B.V., Holland) vertrieben.

Polyester werden durch die Umsetzung von aromatischen Dicarbonsäuren, deren Estern oder anderen esterbildenden Derivaten mit aliphatischen Dihydroxyverbindungen hergestellt. Der in der Hauptkette enthaltene aromatische Ring kann substituiert sein, z.B. durch Halogen wie Chlor und Brom oder durch $C_1$-$C_4$-Alkylgruppen wie Methyl-, Ethyl-, i- bzw. n-Propyl- und n-, i- bzw. tert.-Butylgruppen. Beispiele von Dicarbonsäuren sind Naphthalindicarbonsäure, Terephthalsäure und Isophthalsäure oder deren Mischungen. Es können bis zu 10 mol-% der aromatischen Dicarbonsäuren durch aliphatische oder cycloaliphatische Dicarbonsäuren wie Adipinsäure, Azelainsäure, Sebacinsäure, Dodecandisäuren und Cyclohexandicarbonsäuren ersetzt werden. Als aliphatische Dihydroxyverbindungen werden beispielsweise Diole mit 2 bis 6 Kohlenstoffatomen wie 1,2-Ethandiol, 1,4-Butandiol, 1,6-Hexandiol, 1,4-Hexandiol, 1,4-Cyclohexaridiol und Neopentylglykol oder deren Mischungen eingesetzt. Beispiele einsetzbarer Polyester sind Polyethylenterephthalat (PET), Polyethylennaphthalat und Polybutylenterephthalat (PBT), welche beispielsweise unter den Markenbezeichnungen Rynite® (PET; Fa. DuPont, USA) bzw. Ultradur® (PBT; BASF SE) erhältlich sind.

Polyolefine wie Polyethylene, Polypropylene oder Copolymerisate auf der Basis von Ethylen oder Propylen sind unter den Handelsnamen Lupolen® bzw. Novolen® erhältlich.

Die Poly(meth)acrylate umfassen insbesondere Polymethylmethacrylat (PMMA) und Copolymere auf der Basis von Methylmethacrylat mit bis zu 40 Gew.-% weiterer copolymerisierbarer Monomere, wie n-Butylacrylat, t-Butylacrylat oder 2-Ethylhexylacrylat. Derartige Polymere werden beispielsweise unter den Markenbezeichnungen Lucryl® (BASF SE) und Plexiglas® (Röhm GmbH, Deutschland) vertrieben. Im Sinne der Erfindung sind hierunter auch schlagzähmodifizierte Poly(meth)acrylate sowie Mischungen aus Poly(meth)acrylaten und SAN-Polymerisaten, die mit Polyacrylatkautschuken schlagzäh modifiziert sind (z.B. das Handelsprodukt Terlux® der BASF SE), zu verstehen.

Beispiele der Polyamide sind Polycaprolactam, Polycapryllactam, Polylaurinlactam, und Polyamide, die durch Umsetzung von Dicarbonsäuren mit Diaminen erhalten werden, und umfassen auch beispielsweise Polyetheramide wie Polyetherblockamide. Insbesondere können beispielsweise Polyhexamethylenadipinsäureamid (PA 66, Ultramid® A von BASF SE), und Polyhexamethylensebacinsäureamid (PA 610, Nylon® 610 von Fa. DuPont), Polycaprolactam (PA 6, Ultramid® B von BASF SE) sowie Copolyamide 6/66, insbesondere mit einem Anteil von 5 bis 95 Gew.-% an Caprolactam-Einheiten, (Ultramid® C von BASF SE).

Beispiele vinylaromatischer (Co)polymere sind Polystyrol, Styrol-Acrylnitril-Copolymere (SAN) und schlagzähmodifiziertes Polystyrol (HIPS = High Impact Polystyrene). Hierbei können auch vorzugsweise nicht mehr als 20 Gew.-%, insbesondere nicht mehr als 8 Gew.-%, der Monomere durch Comonomere wie (Meth)acrylnitril oder (Meth)acrylsäureester ersetzt sein. Weitere Beispiele sind ASA-, ABS- und AES-Polymerisate (ASA = Acrylnitril-Styrol-Acrylester, ABS = Acrylnitril-Butadien-Styrol, AES = Acrylnitril-EPDM-Kautschuk-Styrol) besonderes bevorzugt. Diese schlagzähen vinylaromatischen Polymere enthalten mindestens ein kautschukelastisches Pfropfpolymerisat und ein thermoplastisches Polymerisat (Matrixpolymerisat).

Die Polyarylenether umfassen unter anderem auch Polyarylenethersulfide, Polyarylenethersulfone oder Polyarylenetherketone, wobei die Arylengruppen gleich oder verschieden sein können und unabhängig voneinander einen aromatischen Rest mit 6 bis 18 C-Atomen darstellen. Entsprechende Polymere sind beispielsweise unter dem Handelsnamen Noryl® (GE Plastics B.V., Holland) kommerziell erhältlich.

Polyurethane, Polyisocyanurate und Polyharnstoffe sowie deren Herstellung, beispielsweise durch Umsetzung von Isocyanaten mit gegenüber Isocyanaten reaktiven Verbindungen, sind dem Fachmann bekannt. Entsprechende Polymere sind beispielsweise unter der Bezeichnung Elastolan® (Elastogran GmbH, Deutschland) erhältlich.

Polylactide sind Polymere der Milchsäure, umfassen im Sinne der vorliegenden Erfindung jedoch prinzipiell auch Co- oder Blockcopolymere auf der Basis von Milchsäure und weiteren Monomeren. Bei weiteren Ausführungsformen ist es jedoch insbesondere bevorzugt, dass die Monomere der Polylactide im Wesentlichen Milchsäure sind.

Beispiele halogenhaltiger Polymerisate sind Polyvinylchlorid (PVC) wie Hart-PVC und Weich-PVC, und Copolymerisate des Vinylchlorids wie PVC-U-Formmassen.

Beispiele fluorhaltiger Polymere sind Polytetrafluorethylen (PTFE), Tetrafluorethylen-PerfluorpropylenCopolymere (FEP), Copolymere des Tetrafluorethylens mit Perfluoralkylvinylether, Ethylen-Tetrafluorethylen-Copolymere (ETFE); Polyvinylidenfluorid (PVDF), Polyvinylfluorid (PVF), Polychlortrifluorethylen (PCTFE) und Ethylen-Chlortrifluorethylen-Copolymere (ECTFE).

Imidgruppenhaltige Polymere sind insbesondere Polyimide, Polyetherimide, und Polyamidimide.

Beispiele von Celluloseestern sind Celluloseacetat, Celluloseacetobutyrat, und Cellulosepropionat.

Beispiele der Silicon-Polymere sind insbesondere Siliconkautschuke. Dabei handelt es sich üblicherweise um Polyorganosiloxane, die zu Vernetzungsreaktionen fähige Gruppen aufweisen.

Die thermoplastischen Elastomere können wie Thermoplaste verarbeitet werden, weisen jedoch auch elastoplastische Eigenschaften auf. Beispiele sind thermoplastische Polyurethanelastomere (TPE-U oder TPU), Styrol-Oligoblock-Copolymere (TPE-S) wie SBS (Styrol-Butadien-Styrol-oxyBlockcopolymer) und SEES (Styrol-Ethylen-Butylen-Styrol-Blockcopolymer, erhältlich durch Hydrieren von SBS), thermoplastische Polyolefin-Elastomere (TPE-O), thermoplastische Polyester-Elastomere (TPE-E), thermoplastische Polyamid-Elastomere (TPE-A) und insbesondere thermoplastische Vulkanisate (TPE-V).

Zusätzlich zu den erfindungsgemäßen Pigmenten können die vorgenannten Kunststoffe übliche Zusatzstoffe enthalten. Diese Zusatzstoffe können beispielsweise ausgewählt werden aus der Gruppe bestehend aus Füllstoffen, Additiven, Weichmachern, Gleit- oder Entformungsmitteln, Schlagzähigkeitsverbesserern, Farbpigmenten, Farbstoffen, Flammschutzmitteln, Antistatika, optischen Aufhellern, Antioxidantien, antimikrobiell wirksame Biostabilisatoren, chemischen Treibmitteln, organischen Vernetzungsmitteln sowie andere Zusatzstoffen und Mischungen davon.

Der farbgebende Effekt ist unterhalb von 0,1 Gew.-% der erfindungsgemäßen Aluminiumeffektpigmente, bezogen auf das Gesamtgewicht des die erfindungsgemäßen Aluminiumeffektigmente enthaltenden Kunststoffs, nur sehr schwach. Ferner wurde gefunden, dass die mechanische Festigkeit eines entsprechenden Kunststoffs bei einer zu großen Menge an Pigment abnimmt. Gemäß weiteren bevorzugten Ausführungsformen liegt der Anteil der erfindungsgemäßen Aluminiumeffektpigmente im Kunststoff in einem Bereich von 0,01 bis 12 Gew.-%, vorzugsweise von 0,1 bis 9 Gew.-%, mehr bevorzugt von 0,25 bis 5 Gew.-%, noch mehr bevorzugt von 0,5 bis 2,5 Gew.-% liegt, jeweils bezogen auf das Gesamtgewicht des die erfindungsgemäßen Aluminiumeffektigmente enthaltenden Kunststoffs.

Sofern die erfindungsgemäßen jedoch beispielsweise nicht zur Einfärbung eines Kunststoffs, sondern zur Lasermarkierung eingesetzt werden, können andere Konzentrationen des erfindungsgemäßen Aluminiumeffektpigments bevorzugt sein. Bei der Lasermarkierung erhitzt ein Laserstrahl im Kunststoff enthaltene erfindungsgemäße Aluminiumeffektpigmente, infolge dessen durch ein Aufschäumen oder Verkohlen eine sichtbare Veränderung des Kunststoffes hervorgerufen wird. Da für diesen Zweck meist keine optische Beeinträchtigung des Kunststoffes gewünscht ist, wird bei vielen Ausführungsformen beispielsweise eine Konzentration von höchstens 0,8 Gew.-% bevorzugt, bezogen auf das Gesamtgewicht des Kunststoffs umfassend das erfindungsgemäße Aluminiumeffektpigment. Unterhalb einer Menge von 0,0005 Gew.-%, bezogen auf das Gesamtgewicht des Kunststoffs umfassend das erfindungsgemäße Aluminiumeffektpigment, sind die mittels Lasermarkierung hervorgerufenen Effekte jedoch nur sehr schwach. Bei weiteren Ausführungsformen ist es daher bevorzugt, dass die Konzentration des erfindungsgemäßen Aluminiumeffektpigments bei 0,0005 bis 0,8 Gew.-%, vorzugsweise bei 0,001 bis 0,6 Gew.-% und noch weiter bevorzugt bei 0,005 bis 0,55 Gew.-% liegt, jeweils bezogen auf das Gesamtgewicht des Kunststoffs umfassend das erfindungsgemäße Aluminiumeffektpigment.

Es hat sich jedoch gezeigt, dass einige besonders vorteilhaft eingesetzten Reaktanden kosmetisch nicht verträglich sind. Insbesondere ist es bei weiteren Ausführungsformen daher bevorzugt, dass die Verwendungen nicht die kosmetische Verwendung umfassen. Ferner umfassen die erfindungsgemäßen Pigmente bei weiteren Ausführungsformen Stoffe, die für die kosmetische Verwendung ungeeignet sind.

Das erfindungsgemäße Verfahren umfasst die folgenden Schritte:

(1) Nasschemisches Oxidieren von Aluminiumeffektpigmenten,

(2) Beschichten der in Schritt (1) erhaltenen nasschemisch oxidierten Aluminiumeffektpigmente mit von Aluminiumoxid verschiedenem Metalloxid, wobei das Gewichtsverhältnis des als wenigstens eine Metalloxidschicht aufgebrachten Metalloxids zu der in Schritt (1) nasschemisch erzeugten Aluminiumoxidschicht in einem Bereich von 1:1 bis 1:40 liegt,

(3) Beschichten der in Schritt (2) erhaltenen metalloxidbeschichteten nasschemisch oxidierten Aluminiumeffektpigmente mit wenigstens einer umhüllenden organischen Polymerschicht.

Der Verfahrensschritt zum Aufbringen der Metalloxidschicht umfasst wenigstens eine Hydrolyse- und Kondensationsreaktion. Insbesondere ist hierbei das Sol-Gel-Verfahren bevorzugt. Als Metalloxidquelle zur Erzeugung der wenigstens einen Metalloxidschicht können beispielsweise Metallhalogenide wie Metallchloride oder Metallalkoholate eingesetzt werden.

Insbesondere ist es bei weiteren Ausführungsformen bevorzugt, dass die wenigstens eine Metalloxidschicht mittels des Sol-Gel-Prozesses erzeugt wird. Hierbei werden entsprechende Metallalkoholate durch Wasser hydrolysiert, wobei vorzugsweise Säuren oder Basen als Katalysatoren eingesetzt werden. Nachfolgend setzt eine Kondensationsreaktion ein, bei der eine Metalloxidbeschichtung ausgebildet wird. Vorzugsweise finden in dem erfindungsgemäßen Sol-Gel-Prozess Metallalkoholate Einsatz, jedoch können auch Stoffe wie beispielsweise Wasserglas Einsatz finden.

Die erfindungsgemäß in dem Sol-Gel-Prozess zu verwendenden Metallalkoholate tragen vorzugsweise Alkoxygruppen, die ausgewählt werden aus der Gruppe bestehend aus Methoxygruppen, Ethoxygruppen, Propoxygruppen, Butoxygruppen, Pentoxygruppen und Mischungen davon. Äußerst bevorzugt handelt es sich bei den Alkoxygruppen um Methoxygruppen, Ethoxygruppen oder Mischungen davon.

Zur Vereinfachung des Reaktionsablaufs ist es bevorzugt, dass die Hydrolysereaktion zur Erzeugung der Metalloxidschicht im Reaktionsmedium der nasschemischen Oxidationsreaktion abläuft. Es ist jedoch auch möglich, die nasschemisch oxidierten Aluminiumeffektpigmente zu isolieren, beispielsweise mittels Filtration oder Zentrifugieren, und in einem anderen Lösungsmittel zu dispergieren.

Die Hydrolysereaktion, vorzugsweise der Sol-Gel-Prozess, erfolgt üblicherweise in einem organischen Lösungsmittel in Gegenwart von geringen Mengen an Wasser, wie beispielsweise 1 bis 9 Vol.-%, vorzugsweise 2 bis 4 Vol.-%, Wasser, bezogen auf das Gesamtvolumen des wasserhaltigen organischen Lösungsmittels. Das Wasser kann hierbei bereits im organischen Lösungsmittel enthalten sein oder zu einem späteren Zeitpunkt zugegeben werden. Als Katalysatoren werden üblicherweise Säuren oder Basen zugesetzt.

Als organische Lösemittel werden vorzugsweise Alkohole, Glykole, Ester, Ketone sowie Mischungen dieser Lösemittel, mehr bevorzugt Alkohole, Glykole und bei Raumtemperatur flüssige Ketone verwendet. Als besonders geeignet ist die Verwendung von Alkoholen, Glykolen oder deren Mischungen. Besonders bevorzugt werden Alkohole verwendet.

Der Alkohol wird vorzugsweise aus der Gruppe, die aus Methanol, Ethanol, Isopropanol, n-Propanol, t-Butanol, n-Butanol, Isobutylalkohol, Pentanol, Hexanol und deren Mischungen besteht, ausgewählt. Als sehr geeignet haben sich Ethanol und Isopropanol erwiesen. Bei weiteren Ausführungsformen ist es bevorzugt, dass Ethanol oder Isopropanol als Lösungsmittel eingesetzt werden.

Bevorzugte Glykole sind Butylglykol, Propylglykol, Ethylenglykol oder deren Mischungen.

Als Katalysator können beispielsweise organische Säuren, anorganische Säuren oder Mischungen davon eingesetzt werden. Organische Säuren werden vorzugsweise ausgewählt aus der Gruppe bestehend aus Ameisensäure, Essigsäure, Propionsäure, Oxalsäure, Malonsäure, Maleinsäure, Bernsteinsäure, Anhydriden der genannten Säuren und Mischungen davon. Besonders bevorzugt sind Ameisensäure, Essigsäure, Oxalsäure oder deren Mischungen. Anorganische Säuren werden vorzugsweise ausgewählt aus der Gruppe bestehend aus Salpetersäure, Schwefelsäure, Phosphorsäure, Salzsäure, Borsäure, Flusssäure und deren Mischungen. Besonders bevorzugt sind Salpetersäure, Flusssäure oder Mischungen davon.

Als basische Katalysatoren können verschiedenste dem Fachmann bekannte Basen eingesetzt werden, vorzugsweise stickstoffhaltige Basen, insbesondere Amine. Hierbei kann es sich um primäre, sekundäre oder tertiäre Amine handeln.

Gemäß einer bevorzugten Ausführungsform wird das Amin ausgewählt aus der Gruppe bestehend aus Dimethylethanolamin (DMEA), Monoethanolamin (MEA), Diethanolamin (DEA), Triethanolamin (TEA), Ethylendiamin (EDA), t-Butylamin, Monomethylamin, Dimethylamin, Trimethylamin, Monoethylamin, Diethylamin, Triethylamin, Diethylentriamin, Diisopropylethylamin, Pyrridin, Pyrridinderivate, Anilin, Anilinderivate, Cholin, Cholinderivate, Harnstoff, Harnstoffderivat, Hydrazinderivate und Mischungen davon.

Als sehr geeignet haben sich als basischer aminischer Katalysator Ethylendiamin, Monoethylamin, Diethylamin, Monomethylamin, Dimethylamin, Triethylamin oder Mischungen davon erwiesen.

Selbstverständlich können auch anorganische Basen, wie Ammoniak, Hydrazin, Natriumhydroxid, Kaliumhydroxid, Ammoniumhydroxid, Ammoniumcarbonat, Ammoniumhydrogencarbonat, Natriumcarbonat, Natriumhydrogencarbonat, Kaliumcarbonat, Natriumhydrogencarbonat oder Mischungen davon verwendet werden. Als sehr geeignet haben sich Ammoniak, Hydrazin oder Mischungen davon erwiesen.

Gemäß einer äußerst bevorzugten Ausführungsform wird als Metallalkoxid Tetraalkoxysilan verwendet. Als Tetraalkoxysilan werden vorzugsweise Tetramethoxysilan, Tetraethoxysilan, Tetraisopropoxysilan oder Kondensate davon oder deren Mischungen davon verwendet. Als sehr geeignet haben sich Tetraethoxysilan, Oligomere des Tetraethoxysilans oder Mischungen davon erwiesen.

Die Aufbringung der wenigstens einen organischen Polymerschicht erfolgt hierbei bevorzugt durch Polymerisation geeigneter Monomere oder Oligomere. Die Monomere oder Oligomere können dabei Funktionalitäten aufweisen, die aus der Gruppe, die aus Amino-, Hydroxy-, Thiol-, Epoxy-, Acrylat-, Methacrylat-, Vinyl-, Allyl-, Alkenyl-, Alkinyl-, Carboxy-, Carboxylanhydrid-, Isocyanat-, Cyanat-, Ureido-, Carbamat-, Estergruppen und Mischungen davon besteht, ausgewählt werden.

Als Edukte der organischen Polymerschicht sind als Monomere oder reaktive Oligomere oder Polymere insbesondere vernetzende, d.h. mehrfunktionelle (Meth)acrylate geeignet. Beispiele für solche Verbindungen sind: Allylmethacrylat, Bisphenol-A-dimethacrylat, 1,3-Butandioldimethacrylat, 1,4-Butandioldimethacrylat, Ethylenglykoldimethacrylat, 1,6-Hexandioldiacrylat, 1,6-Hexandioldimethacrylat, Diethylenglykoldimethacrylat, Diurethandimethacrylat, Dipropylenglykoldiacrylat, 1,12-Dodecandioldimethacrylat, Ethylenglykoldimethacrylat, Methacrylsäureanhydrid, N,N-Methylen-bis-methacrylamid, Neopentylglykoldimethacrylat, Polyethylenglykoldimethacrylat, Polyethylenglykol-200-diacrylat, Polyethylenglykol-400-diacrylat, Polyethylenglykol-400-dimethacrylat, Tetraethylenglykoldiacrylat, Tetraethylenglykoldimethacrylat, Tricyclodecandimethanoldiacrylat, Tripropylenglykoldiacrylat, Triethylenglykoldimethacrylat, Pentaerythritoltriacrylat, Trimethylol¬propantriacrylat, Trimethylolpropan¬trimethacrylat, Tris-(2-hydroxyethyl)isocyanurattriacrylat, Penta-'erythritoltetraacrylat, Dipentaerythritolpentaacrylat oder Mischungen davon.

Bei weiteren Ausführungsformen ist die Verwendung von tri- und höherfunktionellen (Meth)acrylaten, insbesondere trifunktionellen (Meth)acrylaten bevorzugt. Der Begriff "(Meth)acrylat" im Sinne der vorliegenden Erfindung umfasst Methacrylate und Acrylate.

Das Aushärten oder Auspolymerisieren von Vinyl- und/oder (Meth)acrylatfunktionellen Monomeren beim Erzeugen der wenigstens einen organischen Polymerschicht erfolgt bei weiteren Ausführungsformen thermisch.

Bei weiteren bevorzugten Ausführungsformen erfolgt das Aushärten oder Auspolymerisieren durch radikalische Polymerisation unter Verwendung von Polymerisationsstartern, vorzugsweise von Radikalinitiatoren. Dabei handelt es sich um handelsübliche in der Regel organische oder anorganische Peroxide oder Diazoniumverbindungen. Beispiele solcher Verbindungen sind: Acetyl-cyclohexan-sulfonylperoxid, Bis(2,4-dichlorbenzoyl)peroxid, Diisononanylperoxid, Dioctanoylperoxid, Diacetyl- und Dibenzoylperoxid; Peroxidicarbonate (z.B. Diisopropyl-peroxydicarbonat, Di-n-butylperoxydicarbonat, Di-2-ethylhexylperoxydi-carbonat, Dicyclohexyl-peroxydicarbonat), Alkylperester (z.B. Cumylperneodecanoat, t-Butyl-perneodecanoat, t-Amyl-perpivalat, t-Butyl-per-2-ethylhexanoat, t-Butylperiso-butyrat, t-Butylperbenzoat), Dialkylperoxide (z.B. Dicumylperoxid, t-Butylcumyl-peroxid, 2,5-Dimethylhexan-2,5-di-t-butylperoxid, Di(t-butylperoxyisopropyl)benzol, Di-t-butyl-peroxid, oder 2,5-Dimethylhexin-3-2,5-di-t-butylperoxid), Perketale (z.B. 1,1'-Bis-(t-butylperoxy)-3,3,5-trimethylcyclohexanonperoxid, Methylisobutylketon-peroxid, Methylethylketonperoxid, Acetylacetonperoxid), Alkylhydroperoxide (z.B. Pinanhydroperoxid, Cumolhydroperoxid, 2,5-Dimethylhexan-2,5-dihydroperoxid oder t-Butylhydroperoxid), Azoverbindungen (z.B. 4-4'-Azo-bis(4-cyanvaleriansäure), 1,1'-Azo-bis(cyclohexan-carbonsäurenitril), 1,1'-Azo-bis(isobutyrosäureamidin)dihydro-chlorid, 2,2'-Azo-bis(iso-butyronitril), Dimethyl 2,2'-azobis(2-methylpropionat) oder Persulfate wie Natriumperoxodisulfat und Kaliumperoxodisulfat. Bevorzugt ist 2,2'-Azo-bis(isobutyronitril) und Dimethyl 2,2'-azobis(2-methylpropionate). Diese Verbindungen sind kommerziell erhältlich bei Aldrich Chemie, D-89552, Steinheim oder Wako Chemicals GmbH, Fuggerstraße 12, 41468 Neuss.

Bei den Edukten der wenigstens einen organischen Polymerschicht kann es sich insbesondere auch um reaktive Oligomere, Polymere oder Mischungen davon handeln, die ausgewählt werden aus der Gruppe bestehend aus Polyacrylaten, Poly(meth)acrylaten, Polyethern, Polyestern, Polyaminen, Polyamiden, Polyolen, Polyurethanen, Polyolefinen und Mischungen davon.

Die mit Metalloxid beschichteten erfindungsgemäßen Pigmente werden gemäß einer Variante der Erfindung in einem, vorzugsweise organischen, Lösemittel dispergiert und die Suspension auf Reaktionstemperatur gebracht. Dann werden die Edukte der organischen Polymerschicht, beispielsweise in Form von organischen Monomeren, reaktiven Oligomeren, reaktiven Polymeren oder Mischungen davon, sowie gegebenenfalls Polymerisationsinitiatoren zugegeben, beispielsweise durch Zutropfen, wodurch die organische Polymerschicht auf den erfindungsgemäßen Pigmenten gebildet wird. Vorzugsweise wird die Dispersion während des Aufbringens der organischen Polymerschicht gerührt oder bewegt.

Selbstverständlich kann die organische Polymerschicht auch durch Aufsprühen der Edukte, beispielsweise der organischen Monomere, reaktiven organischen Oligomere, reaktiven organischen Polymeren oder Mischungen davon, sowie optional von Polymerisationsinitiatoren in einer Wirbelschicht, in der die erfindungsgemäßen Pigmente verwirbelt werden, aufgebracht werden.

Gemäß einer bevorzugten Variante der Erfindung erfolgt die Beschichtung in einer Flüssigphase.

Gemäß einer weiteren Variante der Erfindung erfolgt die Aufbringung der umhüllenden organischen Polymerschicht in dem gleichen Lösungsmittel, in dem die von Aluminiumoxid verschiedene Metalloxidschicht aufgebracht wurde. Diese Verfahrensvariante ist im Unterschied zu der oben beschriebenen zweistufigen Verfahrensvariante einstufig.

Nach Aufbringung der organischen Polymerschicht werden diese aus der Suspension vorzugsweise abfiltriert.

Gemäß einer weiteren Variante der Erfindung erfolgt die Aufbringung der umhüllenden organischen Polymerschicht auf der von Aluminiumoxid verschiedenen Metalloxidschicht in Form einer thermischen Polymerisation ohne Zugabe eines Initiators. Es wurde beobachtet, dass die thermische Polymerisation insbesondere zur Ausbildung glatter Oberflächen geeignet ist.

Die erfindungsgemäßen Aluminiumeffektpigmente werden in weiteren erfindungsgemäßen Ausführungsformen pelletiert, granuliert, stranggranuliert, stranggepresst, brikettiert, tablettiert und liegen mithin in einer im Wesentlichen staubarmen, vorzugsweise staubfreien, kompaktierten Form vor. In diesen Darreichungsformen können die erfindungsgemäßen Aluminiumpigmente leicht gehandhabt und in Beschichtungsmittel, wie beispielsweise Lacke, Farben, Druckfarben, Pulverlacken, Kunststoffe, Kosmetika, etc. einfach eingearbeitet werden.

Gemäß einer bevorzugten Variante werden die erfindungsgemäßen Pigmente in Pulverlack eingearbeitet. Gemäß einer bevorzugten Variante der Erfindung ist die umhüllende organische Polymerschicht der erfindungsgemäßen Pigmente kompatibel mit dem Bindemittel oder Bindemittelsystem des Pulverlacks.

Die Erfindung wird nachfolgend anhand von Beispielen näher erläutert, ohne darauf beschränkt zu sein.

**Beispiele**

**Beispiel 1**

*Nasschemische Oxidation und Metalloxidbeschichtung - Vergleichsbeispiel 1a*

Es wurden 144 g einer Aluminiumeffektpigment-Testbenzinpaste ($D_{50}$: 20$\mu$m; 69,3 Gew.-% Feststoffanteil, 30,7 Gew.-% Testbenzin, entspricht 100 g Aluminiumeffektpigment) in 200 g Isopropanol dispergiert und 30 min gerührt. Anschließend wurden 30 g Wasser und 3 g Ethylendiamin zugegeben und für 7 h auf 60 °C erhitzt. Nachfolgend wurde die Metalloxidschicht (Siliziumoxidschicht) auf das Reaktionsprodukt aufgetragen unter Einsatz von 18,8 g Tetraethoxysilan (TEOS) und 15 g Wasser, wobei das Reaktionsgemisch über 4 Stunden auf 75 °C erhitzt wurde.

*Organische Polymerbeschichtung - erfindungsgemäßes Beispiel 1b*

Es wurden 139 g der vorgenannten mit Siliziumoxid beschichteten nasschemisch oxidierten Aluminiumeffektpigmente (mit einem Feststoffanteil von 57,73 Gew.-%, entspricht 80 g Aluminium) in 300 g Isopropanol dispergiert. Danach wurden 1,0 g Methacryloxypropyltrimethoxysilan zugegeben und es wurde auf 90 °C erhitzt und 1 h bei dieser Temperatur gerührt. Danach wurde eine Mischung bestehend aus: 0,9 g Dimethyl-2,2'-azobis(2-methylpropionat) (Handelsname V 601; erhältlich bei WAKO Chemicals GmbH, Fuggerstraße 12, 41468 Neuss), 17,5 g Trimethylolpropantrimethacrylat, 5,8 g Methacryloxypropyltrimethoxysilan aufgefüllt mit Isopropanol auf ein Volumen von 144 ml über einen Zeitraum von 3 h zudosiert. Es wurde weitere 3 h bei 90 °C gerührt, das Produkt isoliert, getrocknet und mit einem Sieb mit 71 $\mu$m Maschenweite gesiebt.

| Versuchs-Nr | SiO$_2$-Gehalt | KunststoffGehalt | Verhältnis SiO$_2$:Al$_2$O$_3$ | Helos D$_{10}$/D$_{50}$/D$_{90}$ |
|---|---|---|---|---|
| **Beispiel 1b** | 5,2 Gew.-% | 22,7 Gew.-% | 1 : 6,5 | 8,4/18,3/32,2 |

**Beispiel 2**

*Nasschemische Oxidation und Metalloxidbeschichtung - Vergleichsbeispiel 2a*

Es wurden 204 g einer Aluminiumeffektpigment-Testbenzinpaste ($D_{50}$: 15$\mu$m; 68,8 Gew.-% Feststoffanteil, 31,2 Gew.-% Testbenzin, entspricht 140 g AI) in 400 g Isopropanol dispergiert und 30 min gerührt. Anschließend wurden 70 g Wasser und 5 g Ethylendiamin zugegeben und für 7 h auf 60 °C erhitzt. Nachfolgend wurde die Metalloxidschicht (Siliziumoxidschicht) auf das Reaktionsprodukt aufgetragen unter Einsatz von 68,5 g Tetraethoxysilan (TEOS) und 90 g Wasser, wobei das Reaktionsgemisch über 4 Stunden auf 75 °C erhitzt wurde.

*Organische Polymerbeschichtung - erfindungsgemäßes Beispiel 2b*

Es wurden 177 g der vorgenannten mit Siliziumoxid beschichteten nasschemisch oxidierten Aluminiumeffektpigmente (mit einem Feststoffanteil von 57 Gew.-%, entspricht 100 g Aluminiumeffektpigment) in 323 g Isopropanol dispergiert. Danach wurden 1,25 g Methacryloxypropyltrimethoxysilan zugegeben und es wurde auf 90 °C erhitzt und 1 h bei dieser Temperatur gerührt. Danach wurde eine Mischung bestehend aus: 1,12 g Dimethyl- 2,2'-azobis(2-methylpropionat) (Handelsname V 601; erhältlich bei WAKO Chemicals GmbH, Fuggerstraße 12, 41468 Neuss), 21,9 g Trimethylolpropantrimethacrylat, 7,25 g Methacryloxypropyltrimethoxysilan aufgefüllt mit Isopropanol auf ein Volumen von 180 ml über einen Zeitraum von 3 h zudosiert. Es wurde weitere 3 h bei 90 °C gerührt und anschließend wurde das Produkt isoliert, getrocknet und mit einem Sieb mit 71 $\mu$m Maschenweite gesiebt.

| Versuchs-Nr. | SiO$_2$-Gehalt | Kunststoff-Gehalt | Verhältnis SiO$_2$:Al$_2$O$_3$ | Helos D$_{10}$ / D$_{50}$ / D$_{90}$ |
|---|---|---|---|---|
| Beispiel 2b | 11,2 Gew.-% | 28 Gew.-% | 1 : 3,4 | 7,7/16,3/27,8 |

## Beispiel 3

*Nasschemische Oxidation und Metalloxidbeschichtung - Vergleichsbeispiel 3a*

Es wurden 144 g einer Aluminiumeffektpigment-Testbenzinpaste (D$_{50}$: 20$\mu$m; 69,3 Gew.-% Feststoffanteil, 30,7 Gew.-% Testbenzin, entspricht 100 g Aluminiumeffektpigment) in 200 g Isopropanol dispergiert und 30 min gerührt. Anschließend wurden 35 g Wasser und 3,5 g Ethylendiamin zugegeben und auf 60 °C für 7 h erhitzt. Nachfolgend wurde die Metalloxidschicht (Siliziumoxidschicht) auf das Reaktionsprodukt aufgetragen unter Einsatz von 28,8 g Tetraethoxysilan (TEOS) und 22,5 g Wasser, wobei das Reaktionsgemisch über 4 h auf 75 °C erhitzt wurde.

*Organische Polymerbeschichtung - erfindungsgemäßes Beispiel 3b*

Es wurden 100 g der vorgenannten mit Siliziumoxid beschichteten nasschemisch oxidierten Aluminiumeffektpigmente in 400 g Isopropanol dispergiert. Danach wurden 1,25 g Methacryloxypropyltrimethoxysilan zugegeben und es wurde auf 90 °C erhitzt und 1 h bei dieser Temperatur gerührt. Danach wurde eine Mischung bestehend aus: 1,12 g Dimethyl-2,2'-azobis(2-methylpropionat) (Handelsname V 601; erhältlich bei WAKO Chemicals GmbH, Fuggerstraße 12, 41468 Neuss), 21,9 g Trimethylolpropantrimethacrylat, 7,25 g Methacryloxypropyltrimethoxysilan aufgefüllt mit Isopropanol auf ein Volumen von 180 ml über einen Zeitraum von 3 h zudosiert. Es wurde weitere 3 h bei 90 °C gerührt und anschließend wurde das Produkt isoliert, getrocknet und mit einem Sieb mit 71 $\mu$m Maschenweite gesiebt.

| Versuchsnr. | SiO$_2$-Gehalt | Kunststoff-Gehalt | Verhältnis SiO$_2$:Al$_2$O$_3$ | Helos D$_{10}$/D$_{50}$/D$_{90}$ |
|---|---|---|---|---|
| Beispiel 3b | 7,5 Gew.-% | 26,1 Gew.-% | 1 : 4,7 | 8,8/19,3/33,4 |

## Beispiel 4:

*Nasschemische Oxidation und Metalloxidbeschichtung - Vergleichsbeispiel 4a*

Es wurden 250 g einer Aluminiumeffektpigment-Testbenzinpaste (D$_{50}$: 20$\mu$m; 70 Gew.-% Feststoffanteil, 30 Gew.-% Testbenzin, entspricht 175 g Aluminiumeffektpigment) in 800 g Isopropanol dispergiert und 30 min gerührt. Anschließend wurden 100 g Wasser und 5,6 g Ethylendiamin zugegeben und auf 60 °C für 7 h erhitzt. Nachfolgend wurde die Metalloxidschicht (Siliziumoxidschicht) auf das Reaktionsprodukt aufgetragen unter Einsatz von 55,2 g TEOS und 22,5 g Wasser, wobei das Reaktionsgemisch über 4 h auf 75 °C erhitzt wurde.

*Organische Polymerbeschichtung - erfindungsgemäßes Beispiel 4b*

Es wurden 100 g der vorgenannten mit Siliziumoxid beschichteten nasschemisch oxidierten Aluminiumeffektpigmente in 400 g Isopropanol dispergiert. Danach wurden 1,25 g Methacryloxypropyltrimethoxysilan zugegeben und es wurde auf 90 °C erhitzt und 1 h bei dieser Temperatur gerührt. Danach wurde eine Mischung bestehend aus: 1,12 g Dimethyl-2,2'-azobis(2-methylpropionat) (Handelsname V 601; erhältlich bei WAKO Chemicals GmbH, Fuggerstraße 12, 41468 Neuss), 21,9 g Trimethylolpropantrimethacrylat, 7,25 g Methacryloxypropyltrimethoxysilan aufgefüllt mit Isopropanol auf ein Volumen von 180 ml über einen Zeitraum von 3 h zudosiert. Es wurde weitere 3 h bei 90 °C gerührt und anschließend wurde das Produkt isoliert, getrocknet und mit einem Sieb mit 71 $\mu$m Maschenweite gesiebt.

| Versuchsnr. | SiO$_2$-Gehalt | Kunststoff-Gehalt | Verhältnis SiO$_2$:Al$_2$O$_3$ |
|---|---|---|---|
| **Beispiel 4b** | 7,3 Gew.-% | 28,7 Gew.-% | 1 : 4,7 |

**Beispiel 5:**

*Nasschemische Oxidation und Metalloxidbeschichtung - Vergleichsbeispiel 5a*

Es wurden 250 g einer Aluminiumeffektpigment-Testbenzinpaste (D$_{50}$: 20$\mu$m; 70 Gew.-% Feststoffanteil, 30 Gew.-% Testbenzin, entspricht 175 g Aluminiumeffektpigment) in 800 g Isopropanol dispergiert und 30 min gerührt. Anschließend wurden 100 g Wasser und 10 g Triethylamin zugegeben und auf 60 °C für 7 h erhitzt. Nachfolgend wurde die Metalloxidschicht (Siliziumoxidschicht) auf das Reaktionsprodukt aufgetragen unter Einsatz von 55,2 g TEOS und 22,5 g Wasser, wobei das Reaktionsgemisch über 4 h auf 75 °C erhitzt wurde.

*Organische Polymerbeschichtung - erfindungsgemäßes Beispiel 5b*

Es wurden 100 g der vorgenannten metalloxidbeschichteten nasschemisch oxidierten Aluminiumeffektpigmente in 400 g Isopropanol dispergiert. Danach wurden 1,25 g Methacryloxypropyltrimethoxysilan zugegeben und es wurde auf 90 °C erhitzt und 1 h bei dieser Temperatur gerührt. Danach wurde eine Mischung bestehend aus: 1,12 g Dimethyl-2,2'-azobis(2-methylpropionat) (Handelsname V 601; erhältlich bei WAKO Chemicals GmbH, Fuggerstraße 12, 41468 Neuss), 21,9 g Trimethylolpropantrimethacrylat, 7,25 g Methacryloxypropyltrimethoxysilan aufgefüllt mit Isopropanol auf ein Volumen von 180 ml über einen Zeitraum von 3 h zudosiert. Es wurde weitere 3 h bei 90 °C gerührt und anschließend wurde das Produkt isoliert, getrocknet und mit einem Sieb mit 71 $\mu$m Maschenweite gesiebt.

| Versuchsnr. | SiO$_2$-Gehalt | Kunststoff-Gehalt | Verhältnis SiO$_2$:Al$_2$O$_3$ |
|---|---|---|---|
| Beispiel 5b | 7,3 Gew.-% | 28,7 Gew.-% | 1 : 5,2 |

**Beispiel 6**

*Nasschemische Oxidation und Metalloxidbeschichtung - Vergleichsbeispiel 6a*

Es wurden 286 g einer Aluminiumeffektpigment-Testbenzinpaste (D$_{50}$: 15$\mu$m; 70,0 Gew.-% Feststoffanteil, 30 Gew.-% Testbenzin, entspricht 200 g Aluminiumeffektpigment) in 850 g Isopropanol dispergiert und 30 min gerührt. Anschließend wurden 110 g Wasser und 12 g Triethylamin zugegeben und für 7 h auf 60 °C erhitzt. Nachfolgend wurde die Metalloxidschicht (Siliziumoxidschicht) auf das Reaktionsprodukt aufgetragen unter Einsatz von 68,5 g Tetraethoxysilan (TEOS) und 28 g Wasser, wobei das Reaktionsgemisch über 4 Stunden auf 75 °C erhitzt wurde.

*Organische Polymerbeschichtung - erfindungsgemäßes Beispiel 6b*

Es wurden 178 g der vorgenannten mit Siliziumoxid beschichteten nasschemisch oxidierten Aluminiumeffektpigmente (mit einem Feststoffanteil von 56 Gew.-%, entspricht 100 g Aluminium) in 650 g Isopropanol dispergiert. Danach wurden 1,25 g Methacryloxypropyltrimethoxysilan zugegeben und es wurde auf 90 °C erhitzt und 1 h bei dieser Temperatur gerührt. Danach wurde eine Mischung bestehend aus: 2,3 g Dimethyl-2,2'-azobis(2-methylpropionat) (Handelsname V 601; erhältlich bei WAKO Chemicals GmbH, Fuggerstraße 12, 41468 Neuss), 21,9 g Trimethylolpropantrimethacrylat, 7,25 g Methacryloxypropyltrimethoxysilan aufgefüllt mit Isopropanol auf ein Volumen von 180 ml über einen Zeitraum von 3 h zudosiert. Es wurde weitere 3 h bei 90 °C gerührt, das Produkt isoliert, getrocknet und mit einem Sieb mit 71 $\mu$m Maschenweite gesiebt.

| Versuchs-Nr | SiO$_2$-Gehalt | Kunststoff-Gehalt | Verhältnis SiO$_2$:Al$_2$O$_3$ |
|---|---|---|---|
| **Beispiel 6b** | 12 Gew.-% | 26,1 Gew.-% | 1 :4,62 |

**Beispiel 7**

*Nasschemische Oxidation und Metalloxidbeschichtung - Vergleichsbeispiel 7a*

Es wurden 286 g einer Aluminiumeffektpigment-Testbenzinpaste ($D_{50}$: 15 $\mu$m; 70,0 Gew.-% Feststoffanteil, 30 Gew.-% Testbenzin, entspricht 200 g Aluminiumeffektpigment) in 850 g Isopropanol dispergiert und 30 min gerührt. Anschließend wurden 110 g Wasser und 10 g Triethylamin zugegeben und für 7 h auf 60 °C erhitzt. Nachfolgend wurde die Metalloxidschicht (Siliziumoxidschicht) auf das Reaktionsprodukt aufgetragen unter Einsatz von 94,5 g Tetraethoxysilan (TEOS) und 39 g Wasser, wobei das Reaktionsgemisch über 4 Stunden auf 75 °C erhitzt wurde.

*Organische Polymerbeschichtung - erfindungsgemäßes Beispiel 7b*

Es wurden 178 g der vorgenannten mit Siliziumoxid beschichteten nasschemisch oxidierten Aluminiumeffektpigmente (mit einem Feststoffanteil von 56 Gew.-%, entspricht 100 g Aluminium) in 650 g Isopropanol dispergiert. Danach wurden 1,25 g Methacryloxypropyltrimethoxysilan zugegeben und es wurde auf 90 °C erhitzt und 1 h bei dieser Temperatur gerührt. Danach wurde eine Mischung bestehend aus: 2,3 g Dimethyl-2,2'-azobis(2-methylpropionat) (Handelsname V 601; erhältlich bei WAKO Chemicals GmbH, Fuggerstraße 12, 41468 Neuss), 21,89 g Trimethylolpropantrimethacrylat, 7,25 g Methacryloxypropyltrimethoxysilan aufgefüllt mit Isopropanol auf ein Volumen von 180 ml über einen Zeitraum von 3 h zudosiert. Es wurde weitere 3 h bei 90 °C gerührt, das Produkt isoliert, getrocknet und mit einem Sieb mit 71 $\mu$m Maschenweite gesiebt.

| Versuchs-Nr | $SiO_2$-Gehalt | Kunststoff-Gehalt | Verhältnis $SiO_2$:$Al_2O_3$ |
|---|---|---|---|
| Beispiel 7b | 15,7 Gew.-% | 26,1 Gew.-% | 1 :3,31 |

*Anwendungsbeispiel 1: Nachoxidation*

Gemäß Vergleichsbeispiel 1a nasschemisch oxidierte Aluminiumeffektpigmente ohne Siliziumoxidbeschichtung und die erfindungsgemäßen Pigmente gemäß Beispiel 1b wurden über 6 Monate bei Raumtemperatur gelagert. Die anfangs farblich sehr ähnlichen Pigmente zeigten hiernach deutliche Unterschiede in ihrer Färbung. Der Vergleich mit analog hergestellten, neuen Pigmentchargen zeigte, dass die erfindungsgemäßen Pigmente keine merkliche Verfärbung aufwiesen, während sich die Eigenfarbe der unbeschichteten, nasschemisch oxidierten Aluminiumeffektpigmente deutlich verändert hatte.

*Anwendungsbeispiel 2: Pulverlack*

*Anwendungsbeispiel 2a: Applizierbarkeit*

Das jeweilige Effektpigment wurde zusammen mit einem Pulverlack AL 96, (Fa. Dupont) sowie mit 0,2 % Aeroxide Alu C (Fa. Evonik) oder Acematt OK 412 (Fa. Evonik) mittels ThermoMix (Fa. Vorwerk) für 4 Minuten auf Stufe 4 eingearbeitet. Die Pigmentierungshöhe betrug 3,0 Gew.-%.
Die Gesamtmenge an Pulverlack im Mischer betrug 300 g plus 0,6 g Aeroxid Alu C oder Acematt OK 412. Aeroxid Alu C ist ein Rieselmittel bestehend aus $Al_2O_3$-Partikeln. Acematt OK 412 ist ein Rieselmittel basierend auf Kieselsäurebasis. Die Pulverlacke wurden mit der OptiSelect (Fa. ITWGema) in einer handelsüblichen Pulverkabine appliziert. Zur Beurteilung der Applikationseigenschaften wurde für 10 Sekunden bei 100 kV und 100 $\mu$A appliziert, anschließend die Beschichtung des Substrats durchgeführt und dann die Anhaftungen an den Elektroden und am Prallteller vergleichend beurteilt. Dieses Verfahren lässt einen Schluss auf das Langzeitverhalten der Pigmente während der praxisgerechten Lackierung zu. Weiterhin wurde anhand der eingebrannten Pulverlackierung das Spritzbild bewertet. Das Augenmerk wurde vor allem auf den Verlauf, also die Glätte der Oberflächenstruktur, sowie auf schwarze, mikroskopisch kleine Fehlstellen, sogenannte Black Spots geworfen. Als Black Spots werden Bereiche auf der Pulverlackoberfläche bezeichnet, die durch eine inhomogene Verteilung von Effektpigmenten hervorgerufen werden. Da diese Erscheinungen im makroskopischen Bereich liegen, erfolgt die Beurteilung dieser Erscheinung durch Begutachtung mit dem Auge. Bevorzugt sind insbesondere sehr glatte Strukturen mit sehr glattem Verlauf ohne Erscheinungen von Black Spots. Das Applikationsverhalten, das Vorhandensein von Black Spots und die Struktur bzw. der Verlauf der Pulverlacke wurden visuell beurteilt.

Als Pigmente wurden hierbei Aluminiumeffektpigmente vor (Anwendungsbvergleichsbeispiel (AVB 2.1)) und nach (AVB 2.2 und AVB 2.3) der nasschemischen Oxidation gemäß Vergleichsbeispiel (VB) 3a (ohne $SiO_2$-Beschichtung)

appliziert. Ferner wurden die erfindungsgemäßen Pigmente gemäß Beispiel 3b (Anwendungsbeispiel (AB) 2.4) und Beispiel 4b (AB 2.5 und AB 2.6) appliziert.

Tabelle A2-1: Anwendungsbeispiele Pulverlack - Applizierbarkeit

| Beispiel | Pigment | Rieselmittel | Ergebnis |
|---|---|---|---|
| AVB 2.1 | Aluminiumeffektpigmente | Acematt OK 412 | gut applizierbar |
| AVB 2.2 | nasschemisch oxidierte Aluminiumeffektpigmente gemäß VB 3a | Acematt OK 412 | sehr starke Anhaftungen |
| AVB 2.3 | nasschemisch oxidierte Aluminiumeffektpigmente gemäß VB 3a | Aeroxide Alu C | leichte Anhaftungen |
| AB 2.4 | Beispiel 3b | Acematt OK 412 | gut applizierbar |
| AB 2.5 | Beispiel 4b | Acematt OK 412 | gut applizierbar |
| AB 2.6 | Beispiel 4b | Aeroxide Alu C | gut applizierbar |
| AB: Anwendungsbeispiel<br>AVB: Anwendungsvergleichsbeispiel | | | |

*Anwendungsbeispiel 2b: Chemikalientest*

Das beschichtete Prüfblech wurde in eine waagrechte Lage gebracht. Es wurden 5 Tropfen 10 %-iger HCl mit den Einwirkzeiten 180, 150, 120, 90, und 60 min aufgebracht. Weiterhin wurden 5 Tropfen 1 M NaOH mit den Einwirkzeiten 180, 120, 60, 30 und 15 min aufgebracht.
Danach wurden die Tropfen mit Wasser entfernt und die ehemals bedeckten Flächen mit den unbedeckten Flächen optisch verglichen. Hierbei wurde eine Bewertungsskala von 0-3 (für jeden einzelnen Punkt) herangezogen (0 = kein Angriff, 3 maximaler Abbau von Pigmenten). Die ermittelten Punke wurden anschließend summiert.

Tabelle A2-2: Anwendungsbeispiele Pulverlack - Chemikalientest

| Versuchs-Nr. | Chemikalientest |
|---|---|
| Beispiel 1b | 0 |
| Vergleichsbeispiel 2a | 9 |
| Beispiel 2b | 0 |
| Beispiel 3b | 0 |
| Beispiel 4b | 0 |
| Beispiel 6b | 0 |
| Beispiel 7b | 0 |

**Anwendungsbeispiel 3: Nasslack**

Anwendungsbeispiel 3a: Chemikalientest

Gemäß Vergleichsbeispiel 5a nasschemisch oxidierte Aluminiumeffektpigmente ohne Siliziumoxidbeschichtung, Aluminiumeffektpigmente gemäß Vergleichsbeispiel 5a mit Siliziumoxidbeschichtung und die erfindungsgemäßen Aluminiumeffektpigmente gemäß Beispiel 5b wurden in einem Wasserlack (ZW-42-11000-01, BASF SE) auf einer Glasträger appliziert. Nach dem Aushärten des Lacks wurde das beschichtete Prüfblech in eine waagrechte Lage gebracht. Es wurden 5 Tropfen 10 %-iger HCl mit den Einwirkzeiten 180, 150, 120, 90, und 60 min aufgebracht. Weiterhin wurden 5 Tropfen 1 M NaOH mit den Einwirkzeiten 180, 120, 60, 30 und 15 min aufgebracht.
Danach wurden die Tropfen mit Wasser entfernt und die ehemals bedeckten Flächen mit den unbedeckten Flächen optisch verglichen. Hierbei wurde eine Bewertungsskala von 0-3 (für jeden einzelnen Punkt) herangezogen (0 = kein Angriff, 3 maximaler Abbau von Pigmenten). Die ermittelten Punke wurden anschließend summiert.

Tabelle A3-1: Anwendungsbeispiele Nasslack - Chemikalientest

| Versuchs-Nr. | Chemikalientest |
|---|---|
| gemäß Vergleichsbeispiel 5a nasschemisch oxidierte Aluminiumeffektpigmente (ohne SiO$_2$-Beschichtung) | 19 |
| Vergleichsbeispiel 5a | 6 |
| Beispiel 5b | 1 |

Anwendungsbeispiel 3b: Spektrometrie

Gemäß Vergleichsbeispiel 4a nasschemisch oxidierte Aluminiumeffektpigmente ohne Siliziumoxidbeschichtung und die erfindungsgemäßen Aluminiumeffektpigmente gemäß Beispiel 4b wurden in einem Wasserlack (ZW-42-11000-01, BASF SE) auf einer Glasträger appliziert. Nach dem Aushärten des Lacks wird 10 %-ige HCl-Lösung sowie an anderer Stelle 1 molare NaOH-Lösung appliziert. Nach 3 h wird die Farbmetrik der unbelasteten Oberfläche, des mittels HCl-Lösung behandelten Oberfläche und der mittels NaOH-Lösung behandelten Oberfläche mit Hilfe eines Minolta Spectrophotometer CM-700d bestimmt.

Tabelle A3-2: Anwendungsbeispiele Nasslack - Spektrometrie

| | Gemäß VB 4a nasschemisch oxidierte Aluminiumeffektpigmente | | | Beispiel 4b | | |
|---|---|---|---|---|---|---|
| | unbelastet | HCl | NaOH | unbelastet | HCl | NaOH |
| L | 79,99 | 64,73 | 77,13 | 78,76 | 78,22 | 78,86 |
| a | -0,17 | 0,69 | -0,02 | -0,20 | -0,18 | -0,20 |
| b | 7,20 | 6,69 | 7,18 | 7,08 | 7,08 | 7,10 |
| ΔE | | 15,29 | 2,86 | | 0,54 | 0,10 |
| VB: vergleichsbeispiel | | | | | | |

Während die Farbe der erfindungsgemäßen Pigmente infolge der Einwirkung der Salzsäure und Natronlauge nahezu unverändert ist, zeigt die Farbe bei den nasschemisch oxidierten Aluminiumeffektpigmenten eine gravierende Abweichung der Farbwerte. Die erfindungsgemäßen Pigmente zeigen somit eine bedeutend bessere Farbstabilität auch unter sehr aggressiven Bedingungen.

**Anwendungsbeispiel 4: kosmetische Formulierungen**

**a. Körper Lotion Wasser-in-Silikon**

Tabelle A4-1: Anwendungsbeispiel - Körper Lotion Wasser-in-Silikon

| INCI Name | Produktname | Gew.-% | Hersteller/ Lieferant |
|---|---|---|---|
| *Phase A* | | | |
| Cyclopentasiloxane (and) Dimethiconol | Dow Corning 1501 | 11,20 | Dow Corning |
| Cyclopentasiloxane | Xiameter PMX-0245 Cyclosiloxane | 5,75 | Dow Corning |
| Cyclopentasiloxane (and) PEG/PPG-18/18 Dimethicone | Dow Corning 5225 C | 13,80 | Dow Corning |
| C 30-45 Alkyl Methicone | Dow Corning Cosmetic Wax AMS-C30 | 3,45 | Dow Corning |

(fortgesetzt)

| INCI Name | Produktname | Gew.-% | Hersteller/ Lieferant |
|---|---|---|---|
| *Phase A* | | | |
| | erfindungsgemäße Aluminiumeffektpigmente gemäß Beispiel 1b | 1,00 | |
| *Phase B* | | | |
| Polysorbate 20 | Tween 20 | 0,60 | Croda |
| Phenoxyethanol (and) Methylparaben (and) Ethylparaben (and) Butylparaben (and) Probylparaben (and) Isobutylparaben | Uniphen P-23 | 0,35 | Induchem |
| Sodium Chloride | Natriumchlorid | 0,75 | VWR |
| Aqua | Wasser | 63,10 | |

Das erfindungsgemäßen Aluminiumeffektpigment kann in einem Bereich von 0,2 bis 2,5 Gew.-%, bezogen auf das Gesamtgewicht der Formulierung, eingesetzt werden. Der Ausgleich kann mit Wasser erfolgen.

Phase A wurde gemischt und auf 75°C erhitzt, Phase B nach dem Mischen auf 70°C erhitzt, anschließend wurde Phase B langsam unter Homogenisierung zu Phase A hinzugefügt. Unter Rühren wurde die Emulsion abgekühlt und in ein angemessenes Behältnis abgefüllt.

**b. Creme Lidschatten**

Tabelle A4-2: Anwendungsbeispiel - Creme Lidschatten

| INCI Name | Produktname | Gew.-% | Hersteller/ Lieferant |
|---|---|---|---|
| *Phase A* | | | |
| Castor Oil | Rizinusöl | 43,70 | Honeywell Riedel-de Haen |
| Ethylhexyl Palmitate | Cegesoft C24 | 6,00 | Cognis |
| Cocos Nucifera (Coconut) Oil | Lipovol C-76 | 7,00 | Lipo Chemicals |
| Cera Alba | Ewacera 12 | 6,00 | H. Erhard Wagner |
| Isopropyl Lanolate | Ewalan IP | 5,00 | H. Erhard Wagner |
| Persea Gratissima (Avocado) Oil and Hydrogenated Vegetable Oil | Avocado Butter | 7,00 | Impag |
| Magnesium Stearate | Magnesiumstearat | 3,00 | Sigma-Aldrich |
| Bis-Hydroxyethoxypropyl Dimethicone | Dow Corning 5562 Carbinol Fluid | 7,00 | Dow Corning |
| Dimethicone/ Vinyl Dimethicone Crosspolymer and Silica | Dow Corning 9701 Cosmetic Powder | 5,00 | Dow Corning |
| Phenoxyethanol (and) Methylparaben (and) Ethylparaben (and) Butylparaben (and) Probylparaben (and) Isobutylparaben | Uniphen P-23 | 0,30 | Induchem |

(fortgesetzt)

| Phase B | | | |
|---|---|---|---|
| | erfindungsgemäße Aluminiumeffektpigmente gemäß Beispiel 1b | 10,00 | |

Das erfindungsgemäße Aluminiumeffektpigment kann in einem Bereich von 5 bis 22,0 Gew.-%, bezogen auf das Gesamtgewicht der Formulierung, eingesetzt werden. Der Ausgleich kann mit Castor Oil erfolgen.

Phase A wurde gemischt und auf 85 °C erhitzt, Phase B wurde anschließend unter Rühren zu Phase A hinzugegeben. Nach Abfüllen in ein entsprechendes Behältnis wird die Mischung auf Raumtemperatur abgekühlt.

**c. Duschgel**

Tabelle A4-3: Anwendungsbeispiel - Duschgel

| INCI Name | Produktname | Gew.-% | Hersteller/ Lieferant |
|---|---|---|---|
| Phase A | | | |
| | erfindungsgemäße Aluminiumeffektpigmente gemäß Beispiel 1b | 0,50 | |
| Aqua | Wasser | 58,10 | |
| Acrylates Copolymer | Carbopol Aqua SF-1 | 5,50 | Lubrizol |
| Phase B | | | |
| Sodium Hydroxide | NaOH (10 Gew.-%) | 1,50 | |
| Phase C | | | |
| Sodium Laureth Sulfate | Texapon NSO | 22,00 | Cognis |
| Cocamidopropyl Betaine | Tego Betain F 50 | 6,00 | Evonik |
| PEG-7 Glyceryl Cocoate | Emanon HE | 2,00 | Kao Corp. |
| Disodium Laureth Sulfosuccinate | Sectacin 103 Spezial | 2,00 | Zschimmmer & Schwarz |
| Phase D | | | |
| Phenoxyethanol (and) Piroctone Olamine | Nipaguard PO 5 | 0,60 | Clariant |
| Fragrance | Water Lily OA | 0,20 | Bell Flavors and Fragrances |
| Sodium Chloride | Natriumchlorid | 1,60 | VWR |

Das erfindungsgemäße Aluminiumeffektpigment kann in einem Bereich von 0,01 bis 1,0 Gew.-%, bezogen auf das Gesamtgewicht der Formulierung, eingesetzt werden. Der Ausgleich kann mit Wasser erfolgen.

Phase A wurde gemischt und gerührt. Danach wurde Phase B zugegeben und gerührt bis ein homogenes Erscheinungsbild erreicht wurde. Phase C wurde separat eingewogen, vermischt und zu Phase AB hinzugefügt. Anschließend erneut rühren lassen und Phase D einzeln zugeben.

**d. Gepresster Lidschatten**

Tabelle A4-4: Anwendungsbeispiel - Gepresster Lidschatten

| INCI Name | Produktname | Gew.-% | Hersteller/ Lieferant |
|---|---|---|---|
| *Phase A* | | | |
| Mica | Silk Mica | 17,00 | VWR |
| Boron Nitride | Softouch CCS 102 | 2,50 | Momentive |
| Zinc Stearate | Zinkstearat | 7,00 | VWR |
| Talc | Talkumpuder | 43,50 | Sigma-Aldrich |
| | erfindungsgemäße Aluminiumeffektpigmente gemäß Beispiel 1b | 20,00 | |
| *Phase B* | | | |
| Dimethicone | Xiameter PMX-200 Silicone Fluid 5cs | 5,00 | Dow Corning |
| Cyclopentasiloxane (and) Dimethicone Crosspolymer | Dow Corning 9040 Elastomer | 5,00 | Dow Corning |

Das Pigment kann in einem Bereich von 5,0 bis 40,0 Gew.-%, bezogen auf das Gesamtgewicht der Formulierung, eingesetzt werden. Der Ausgleich kann mit Talc erfolgen.

Phase A wurde für 30s bei 2500rpm in einem Hochgeschwindigkeitsmixer gemischt. Anschließend wurde Phase B zugegeben und die Mischung für 60s bei 3000rpm im gleichen Mixer gemischt. Zuletzt wird die Pulvermischung mittels einer Lidschattenpresse bei 150 bar für 30s in Form gepresst.

**e. Haar Mascara**

Tabelle A4-5: Anwendungsbeispiel - Haar Mascara

| INCI Name | Produktname | Gew.-% | Hersteller/ Lieferant |
|---|---|---|---|
| *Phase A* | | | |
| Polyquaternium-16 | Luviquat FC 905 (Luviquat Exellence) | 2,70 | BASF |
| Propylene Glycol | 1,2-Propandiol | 1,80 | VWR |
| Methylparaben | Methyl-4-hydroxybenzoat | 0,20 | Sigma-Aldrich |
| Aqua | Wasser | 64,45 | |
| *Phase B* | | | |
| Cetearyl Alcohol | Lanette O | 5,00 | Cognis |
| Dimethicone | Xiameter PMX-200 Silicone Fluid 350cs | 1,00 | Dow Corning |
| Ceteareth-25 | Cremophor A 25 | 2,00 | BASF |
| Propylparaben | Propyl-4-hydroxybenzoat | 0,10 | Sigma-Aldrich |
| *Phase C* | | | |
| Hydroxypropylcellulose | Klucel G | 0,50 | Ashland |
| Magnesium Aluminium Silicate | Veegum HV | 0,50 | R. T. Vanderbilt |

(fortgesetzt)

| | | | |
|---|---|---|---|
| *Phase C* | | | |
| Aqua | Wasser | 19,00 | |
| *Phase D* | | | |
| | erfindungsgemäße Aluminiumeffektpigmente gemäß Beispiel 1b | 2,50 | |
| Phenoxyethanol (and) Methylparaben (and) Butylparaben (and) Ethylparaben (and) Propylparaben (and) Isobutylparaben | Phenonip | 0,20 | Clariant |
| Fragrance | Blue Shadow ÖKO | 0,05 | Bell Flavors and Fragrances |

Das Pigment kann in einem Bereich von 1,0 bis 10,0 Gew.-%, bezogen auf das Gesamtgewicht der Formulierung, eingesetzt werden. Der Ausgleich kann mit dem Wasser aus Phase A erfolgen.

Phase A und Phase B wurden getrennt auf 80°C erhitzt, danach wurde Phase B langsam zu Phase A hinzugefügt. In einem separaten Gefäß wurden Klucel und Veegum dem Wasser von Phase C hinzugegeben. Anschließend wurde Phase AB auf 40°C abgekühlt und während des Abkühlens Phasen C und D unter Rühren hinzugefügt.

**f. Haargel**

Tabelle A4-6: Anwendungsbeispiel - Haargel

| INCI Name | Produktname | Gew.-% | Hersteller/ Lieferant |
|---|---|---|---|
| *Phase A* | | | |
| | erfindungsgemäße Aluminiumeffektpigmente gemäß Beispiel 1b | 0,10 | |
| Ammonium Acryloyldimehtyltaurate/ VP Copolymer | Aristoflex AVC | 1,40 | Clariant |
| Citric Acid | Zitronensäure | 0,10 | VWR |
| Aqua | Wasser | 55,10 | |
| *Phase B* | | | |
| PVP | Luviskol K 30 Powder | 1,50 | BASF |
| Propylene Glycol, Diazolidinyl, Urea, Methylparaben, Propylparaben | Germaben II | 0,20 | International Speciality Products |
| Triethanolamine | Triethanolamin | 1,20 | VWR |
| Wasser | Wasser | 40,40 | |

Das Pigment kann in einem Bereich von 0,01 bis 2,0 Gew.-%, bezogen auf das Gesamtgewicht der Formulierung, eingesetzt werden. Der Ausgleich kann mit Wasser erfolgen.

Das Pigment wurde mit Wasser aus Phase A verrührt, Aristoflex AVC und Citric Acid wurden unter Rühren hinzugegeben und bei einer Geschwindigkeit von 800 rpm für 15 Minuten gemischt. Phase B wurde gelöst bis eine homogene Lösung entstand, anschließend wurde Phase B zu Phase A gegeben und gemischt.

**g. Körperpuder**

Tabelle A4-7: Anwendungsbeispiel - Körperpuder

| INCI Name | Produktname | Gew.-% | Hersteller/ Lieferant |
|---|---|---|---|
| *Phase A* | | | |
| Mica | Silk Mica | 58,70 | VWR |
| Talc | Talkumpuder | 18,00 | Sigma-Aldrich |
| Boron Nitride | Softouch CCS 102 | 5,00 | Advanced Ceramics |
| Nylon-12 | Orgasol 2002 D/Nat | 8,00 | Arkema |
| Magnesium Stearate | Magnesiumstearat | 6,00 | Sigma-Aldrich |
| Methylparaben, Propylparaben | Rokonsal SSH-1 | 0,30 | ISP Biochema |
| | erfindungsgemäße Aluminiumeffektpigmente gemäß Beispiel 1b | 2,00 | |
| *Phase B* | | | |
| Tridecyl Stearate (and) Tridecyl Trimellitate (and) Dipentaerythrityl Hexacaprylate/ Hexacaprate | Lipovol MOS-130 | 2,00 | Lipo Chemicals |

Das Pigment kann in einem Bereich von 0,2 bis 5,0 Gew.-%, bezogen auf das Gesamtgewicht der Formulierung, eingesetzt werden. Der Ausgleich kann mit Silk Mica erfolgen.

Phase A wurde gemischt, anschließend wurde Phase B zu Phase A zugegeben und anschließend in ein geeignetes Gefäß abgefüllt.

**h. Lipgloss**

Tabelle A4-8: Anwendungsbeispiel - Lipgloss

| INCI Name | Produktname | Gew.-% | Hersteller/ Lieferant |
|---|---|---|---|
| *Phase A* | | | |
| Hydrogenated Polyisobutene (and) Ethylene/ Propylene/Styrene Copolymer (and) Butylene/ Ethylene/Styrene Copolymer | Versagel ME 750 | 79,00 | Calumet Penreco |
| Simmondsia Chinensis (Jojoba) Seed Oil | Jojoba Oil - Natural/Golden | 2,00 | BioChemica |
| Caprylyl Trimethicone | Silcare Silicone 31M50 | 7,00 | Clariant |
| Stearyl Dimethicone | Silcare Silicone 41M65 | 3,20 | Clariant |
| Hydrogenated Polydecene | Nexbase 2002 | 4,00 | Jan Dekker |
| Isopropyl Myristate | Isopropylmyristat | 4,50 | VWR |

(fortgesetzt)

| | | | |
|---|---|---|---|
| *Phase B* | | | |
| | erfindungsgemäße Aluminiumeffektpigmente gemäß Beispiel 1b | 0,10 | |
| Propylparaben | Propyl-4-hydroxybenzoat | 0,20 | Sigma-Aldrich |

Das Pigment kann in einem Bereich von 0,10 bis 8,00 Gew.-%, bezogen auf das Gesamtgewicht der Formulierung, eingesetzt werden. Der Ausgleich kann mit Versagel ME 750 erfolgen.

Phase A wurde auf 85°C erhitzt, anschließend wurden die Inhaltsstoffe der Phase B einzeln zu Phase A hinzugegeben, gerührt bis eine gleichmäßige Konsistenz entstand und dann in ein Lip Gloss Gefäß abgefüllt.

**i. Lippenkonturenstift**

Tabelle A4-9: Anwendungsbeispiel - Lippenkonturenstift

| INCI Name | Produktname | Gew.-% | Hersteller/ Lieferant |
|---|---|---|---|
| *Phase A* | | | |
| Hydrogenated Coco-Glycerides | Softisan 100 | 12,35 | Sasol Wax |
| Candelilla Cera | Ewacera 42 | 14,00 | H. Erhard Wagner |
| Magnesium Stearate | Magnesiumstearat | 6,00 | Sigma-Aldrich |
| Stearic Acid | Kortacid 1895 | 8,50 | Akzo Nobel |
| Hydrogenated Coconut Oil | Lipex 401 | 8,00 | Aarhus Karlshamn |
| Cetyl Palmitate | Kahlwax 7157 | 7,00 | Kahl |
| Caprylic/ Capric Triglyceride | Liponate GC-K | 3,60 | Lipo Chemicals |
| Soybean Glycerides (and) Butyrospermum Parkii | Lipex L'sens | 15,00 | Aarhus Karlshamn |
| Tocopheryl Acetate | dl-alpha-Tocopherylacetat | 0,25 | Jan Dekker |
| Methylparaben; Propylparaben | Rokonsal SSH-1 | 0,30 | ISP Biochema |
| *Phase B* | | | |
| | erfindungsgemäße Aluminiumeffektpigmente gemäß Beispiel 1b | 25,00 | |

Das Pigment kann in einem Bereich von 15 bis 25 Gew.-%, bezogen auf das Gesamtgewicht der Formulierung, eingesetzt werden. Alternativ können zusätzlich zu dem Pigment weitere Farb- und/ oder Effektpigmente zugegeben werden. Die maximale Pigmentierungshöhe sollte jedoch nicht überschritten werden.

Phase A wurde auf 85°C erhitzt und anschließend die Phase B der Phase A unter Rühren hinzugefügt bis sich eine gleichförmige Masse ergab. Danach wurde die Mischung heiß in eine Stiftform eingefüllt.

**j. Lippenstift**

Tabelle A4-10: Anwendungsbeispiel - Lippenstift

| INCI Name | Produktname | Gew.-% | Hersteller/ Lieferant |
|---|---|---|---|
| *Phase A* | | | |
| Carnauba Wax | Ewacera 34 | 4,50 | H. Erhard Wagner |
| Cera Alba | Ewacera 12 | 3,50 | H. Erhard Wagner |
| Candelilla Cera Extract | Ewacera 42 | 4,00 | H. Erhard Wagner |
| Microcrystalline Wax | TeCero-Wax 1030 K | 7,20 | TH.C. Tromm |
| Cetyl Palmitate | Kahlwax 7157 | 2,00 | Kahl |
| Hydrogenated Coco-Glycerides | Softisan 100 | 5,00 | Sasol Wax |
| Petrolatum | Penreco Blond | 5,80 | Calumet Penreco |
| Cetearyl Ethylhexanoate | Luvitol EHO | 10,70 | BASF |
| Tocopheryl Acetate | dl-alpha-Tocopherylacetat | 0,50 | Jan Dekker |
| Castor Oil | Rizinusöl | 46,60 | Honeywell Riedel-de Haen |
| *Phase B* | | | |
| | erfindungsgemäße Aluminiumeffektpigmente gemäß Beispiel 1b | 10,00 | |
| Methylparaben, Propylparaben | Rokonsal SSH-1 | 0,20 | ISP Biochema |

Das Pigment kann in einem Bereich von 0,5 bis 21,0 Gew.-%, bezogen auf das Gesamtgewicht der Formulierung, eingesetzt werden. Der Ausgleich kann mit Castor Oil erfolgen.
Phase A wurde auf 85°C erhitzt, danach wurde Phase B zu Phase A gegeben und gemischt. Anschließend wurde diese Mischung bei einer Temperatur von 75 °C in einem Lippenstiftform abgefüllt.

**k. Flüssig Lidstrich**

Tabelle A4-11: Anwendungsbeispiel - Flüssig Lidstrich

| INCI Name | Produktname | Gew.-% | Hersteller/ Lieferant |
|---|---|---|---|
| *Phase A* | | | |
| Aqua | Wasser | 66,70 | |
| Water/ carbon black dispersion | MBD 201 | 3,00 | Geotech |
| Acrylates Copolymer | Covacryl E14 | 10,00 | LCW |
| Magnesium Aluminium Silicate | Veegum HV | 1,00 | C. H. Erbslöh |
| *Phase B* | | | |
| Propylene Glycol | 1,2 Propandiol | 3,00 | VWR |

(fortgesetzt)

| Phase B | | | |
|---|---|---|---|
| Triethanolamine | Triethanolamin | 1,40 | VWR |
| Phase C | | | |
| Xanthan Gum | Keltrol CG-T | 0,30 | CP Kelco |
| Phase D | | | |
| | erfindungsgemäße Aluminiumeffektpigmente gemäß Beispiel 1b | 3,00 | |
| Mica | Silk Mica | 2,00 | VWR |
| Phase E | | | |
| Stearic Acid | Kortacid 1895 | 2,80 | Akzo Nobel |
| Glyceryl Stearate | Aldo MS K FG | 0,80 | Lonza |
| Oleyl Alcohol | HD-Ocenol 90/95 V | 0,50 | Cognis |
| Phenoxyethanol (and) Methylparaben (and) Ethylparaben (and) Butylparaben (and) Probylparaben (and) Isobutylparaben | Uniphen P-23 | 0,50 | Induchem |
| Phase F | | | |
| Dimethicone (and) Trisiloxane | Xiameter PMX-1184 Silicone Fluid | 5,00 | Dow Corning |

Das Pigment kann in einem Bereich von 0,5 bis 8,0 Gew.-%, bezogen auf das Gesamtgewicht der Formulierung eingesetzt werden. Der Ausgleich kann mit Wasser erfolgen.

Veegum wurde in Phase A dispergiert und 15 Minuten gerührt, danach wurde Phase B zu Phase A hinzugefügt, anschließend Phase C zu Phase AB und erneut 10 Minuten verrührt. Anschließend wurde Phase D zu Phase ABC hinzugefügt und auf 75°C erhitzt. Nun wurde Phase E ebenfalls auf 75°C erwärmt und zu Phase ABCD hinzugefügt. Nach dem Abkühlen auf 60°C wurde Phase F zugefügt und in ein geeignetes Gefäß abgefüllt.

**I. Mousse**

Tabelle A4-12: Anwendungsbeispiel - Mousse

| INCI Name | Produktname | Gew.-% | Hersteller/ Lieferant |
|---|---|---|---|
| Phase A | | | |
| Cyclopentasiloxane | Xiameter PMX-0245 Cyclosiloxane | 8,60 | Dow Corning |
| Hydrogenated Polyisobutene | MC 30 | 4,00 | www.sophim.com |
| Dimethicone (and) Dimethicone Crosspolymer | Dow Corning 9041 Silicone Elastomer Blend | 37,14 | Dow Corning |
| Squalane | Squalane | 5,74 | Impag |
| Isononyl Isononanoate | Dermol 99 | 10,16 | Alzo International |
| Hydrogenated Jojoba Oil | Jojoba Butter LM | 2,15 | Desert Whale |
| Hydrogenated Jojaba Oi | Jojoba Butter HM | 1,00 | Desert Whale |
| C30-45 Alkyl Methicone (and) C30-45 Olefin | Dow Corning AMS-C30 Cosmetic Wax | 1,15 | Dow Corning |

(fortgesetzt)

| INCI Name | Produktname | Gew.-% | Hersteller/ Lieferant |
|---|---|---|---|
| *Phase A* | | | |
| Stearyl Dimethicone | Dow Corning 2503 Cosmetic Wax | 0,47 | Dow Corning |
| Cyclopentasiloxane (and) Polypropylsilsesquioxane | Dow Corning 670 Fluid | 5,00 | Dow Corning |
| *Phase B* | | | |
| Dimethicone/ Vinyl Dimethicone Crosspolymer | Dow Corning 9506 Powder | 16,02 | Dow Corning |
| Silica Dimethyl Silylate | Covasilic 15 | 0,17 | LCW |
| Talc | Talkumpuder | 5,00 | Sigma-Aldrich |
| | erfindungsgemäße Aluminiumeffektpigmente gemäß Beispiel 1b | 3,00 | |
| *Phase D* | | | |
| Propylene Glycol, Diazolidinyl Urea, Methylparaben, Propylparaben | Germaben II | 0,40 | International Speciality Products |

Das Pigment kann in einem Bereich von 0,1 bis 8,0 Gew.-%, bezogen auf das Gesamtgewicht der Formulierung, eingesetzt werden. Der Ausgleich kann mit Dow Corning 9041 Elastomer erfolgen.

Phase A wurde gemischt und solange erhitzt bis alles aufgeschmolzen war. Phase B wurden separat eingewogen und mit einem Hochgeschwindigkeitsmixer für 60s bei 2400 rpm gemischt. Die Hälfte der geschmolzenen Phase A wurde zur Phase B zugegeben und wieder im Mixer bei 2400 rpm für 30s gemischt. Anschließend wurde der übrige Teil der Phase B ebenfalls zur Phase A zugegeben und wieder bei 2400 rpm für 30s gemischt. Zuletzt wird Phase C zu Phase AB gegeben und wieder bei 2400 rpm für 30s im Hochgeschwindigkeitsmixer gemischt.

## m. Nagellack

Tabelle A4-13: Anwendungsbeispiel - Nagellack

| INCI Name | Produktname | Gew.-% | Hersteller/ Lieferant |
|---|---|---|---|
| *Phase A* | | | |
| | erfindungsgemäße Aluminiumeffektpigmente gemäß Beispiel 1b | 2,00 | |
| *Phase B* | | | |
| Butylacetat (und) Ethylacetat (und) Nitrocellulose (und) Isopropyl Alcohol | International Lacquers Nailpolish & Care Base 359 | 98,00 | International Lacquers |

Das Pigment kann in einem Bereich von 0,1 bis 10,0 Gew.-%, bezogen auf das Gesamtgewicht der Formulierung, eingesetzt werden. Der Ausgleich kann mit International Lacquers Nailpolish erfolgen.

Phase A und Phase B wurden gemischt und anschließend in ein angemessenes Behältnis abgefüllt.

**n. Nagellack mit "soft touch"-Effekt**

Tabelle A4-14: Anwendungsbeispiel - Nagellack mit "soft touch"-Effekt

| INCI Name | Produktname | Gew.-% | Hersteller/ Lieferant |
|---|---|---|---|
| *Phase A* | | | |
| | erfindungsgemäße Aluminiumeffektpigmente gemäß Beispiel 1b | 2,00 | |
| | Ceraflour 913 | 5,00 | Byk Chemie |
| *Phase B* | | | |
| Butylacetat (und) Ethylacetat (und) Nitrocellulose (und) Isopropyl Alcohol | International Lacquers Nailpolish & Care Base 359 | 93,00 | International Lacquers |

Das Pigment kann in einem Bereich von 0,1 bis 10,0 Gew.-%, bezogen auf das Gesamtgewicht der Formulierung, eingesetzt werden. Der Ausgleich kann mit International Lacquers Nailpolish erfolgen.

**Anwendungsbeispiel 31: Anwendungsbeispiel - wässriger Nagellack**

Das Pigment kann in einem wässrigen Nagellack gemäß WO 2007/115675 A2 Anwendungsbeispiel 1 eingesetzt werden. Die Pigmentierungshöhe beträgt hierbei 0,1 bis 10,0 Gew.-%, beispielsweise 1,5 Gew.-%, bezogen auf das Gesamtgewicht der Formulierung.

**o. Flüssig Lidschatten**

Tabelle A4-15: Anwendungsbeispiel - Flüssig Lidschatten

| INCI Name | Produktname | Gew.-% | Hersteller/ Lieferant |
|---|---|---|---|
| *Phase A* | | | |
| Wasser | Wasser | 70,10 | |
| Glycerin | Pricerine 9090 | 6,00 | Croda |
| *Phase B* | | | |
| PEG-800 | Polyglycol 35000 S | 0,60 | Clariant |
| Allantoin | Allantoin | 0,30 | 3V |
| Ammonium Acryloyldimehtyltaurate/ VP Copolymer | Aristoflex AVC | 0,80 | Clariant |
| Acrylates Copolymer | Worlee Micromer CEK 20/50 | 5,00 | Worlee |
| *Phase C* | | | |
| | erfindungsgemäße Aluminiumeffektpigmente gemäß Beispiel 1b | 10,00 | |
| Divinyldimethicone/ Dimethicone Copolymer C12-C13 Pareth-3, C12-C13 Pareth-23 | Dow Corning HMW 2220 Non-Ionic Emulsion | 6,00 | Dow Corning |

(fortgesetzt)

| Phase C | | | |
|---|---|---|---|
| Fragrance | Water Lily OA | 0,20 | Bell Flavors and Fragrances |
| Phenoxyethanol (und) Methylparaben (und) Butylparaben (und) Ethylparaben (und) Propylparaben (und) Isobutylparaben | Phenonip | 1,00 | Clariant |

Das Pigment kann in einem Bereich von 0,10 bis 17,00 Gew.-% eingesetzt werden. Der Ausgleich kann mit Wasser erfolgen.

Phase A wurde gerührt, anschließend wurden die Inhaltsstoffe der Phase B einzeln zu Phase A hinzugegeben und gerührt bis eine gleichmäßige Konsistenz entstand. Danach wurden die Inhaltsstoffe der Phase C einzeln zu Phase AB gegeben und gerührt bis wieder eine gleichmäßige Konsistenz entstand.

### Anwendungsbeispiel 5: Coil Coating

### Anwendungsbeispiel 5a: Chemikalientest

Gemäß Vergleichsbeispiel 3a nasschemisch oxidierte Aluminiumeffektpigmente ohne Siliziumoxidbeschichtung, Aluminiumeffektpigmente gemäß Vergleichsbeispiel 3a mit Siliziumoxidbeschichtung und die erfindungsgemäßen Aluminiumeffektpigmente gemäß Beispiel 3b wurden im Coil Coating-Verfahren eingesetzt. Es wurden 8,0 g Aluminiumeffektpigmentpaste und 8,0 g Solvesso 150 mit einem Spatel gut dispergiert bis die Mischung stippenfrei war. Anschließend wurden 84,0 g Polyester-Lack 42-00001 (PE-Lack) bzw. 84,0 g Polyvinylidenfluorid zugeben und gerührt, bevor mit 5,0 g Solvesso 150 verdünnt wurde. Anschließend wurde 3 Minuten mit dem Zahnkranzrührer bei 500 U/min gerührt. Die Viskosität betrug 100"± 10 im DIN4 - Becher.

Dieser Lackansatz wurde auf ein Alcan - Aluminiumblech DIN A4 (Nr. 11) mit einer Spiralrakel abgezogen. Das Blech wurde sofort für 55 sec. in einen 280° heißen Ofen überführt. Danach wurde das Blech im Wasserbad (RT) abgeschreckt. Nach frühestens 24 h wurde dann der Chemikalientest durchgeführt.

Das beschichtete Prüfblech wurde in eine waagrechte Lage gebracht. Es wurde je ein Tropfen Salzsäure (HCl) 5 %-ig und ein Tropfen Natronlauge (NaOH) 5 %-ig auf das Blech aufgebracht. Die Tropfengröße sollte 20 bis 25 mm im Durchmesser betragen. Anschließend wurden die Tropfen mit einem Uhrglas abgedeckt und für 24 h stehen gelassen. Danach wurden die Tropfen mit Wasser entfernt und die ehemals bedeckten Flächen mit den unbedeckten Flächen optisch verglichen. Hierbei wurde eine Bewertungsskala von 0-3 herangezogen (0 = kein Angriff, 3 maximaler Abbau von Pigmenten).

Tabelle A5-1: Anwendungsbeispiel Coil-Coating - Chemikalientest

| Beispiel | Pigment | | Chemikalientest |
|---|---|---|---|
| AVB 5.1 | gemäß VB 3a nasschemisch oxidierte Aluminiumeffektpigmente ohne Siliziumoxidbeschichtung | PE-Lack | 3 |
| AVB 5.2 | VB 3a | PE-Lack | 3 |
| AB 5.3 | Beispiel 3b | PE-Lack | 0 |
| AVB 5.4 | gemäß VB 3a nasschemisch oxidierte Aluminiumeffektpigmente ohne Siliziumoxidbeschichtung | Polyvinylidenfluorid | 2 |
| AVB 5.5 | VB 3a | Polyvinylidenfluorid | 1 |
| AB 5.6 | Beispiel 3b | Polyvinylidenfluorid | 0 |
| AB: Anwendungsbeispiel AVB: Anwendungsvergleichsbeispiel | | | |

*Anwendungsbeispiel 5b: Spektrometrie*

Gemäß Vergleichsbeispiel 3a nasschemisch oxidierte Aluminiumeffektpigmente ohne Siliziumoxidbeschichtung, Aluminiumeffektpigmente gemäß Vergleichsbeispiel 3a mit Siliziumoxidbeschichtung und die erfindungsgemäßen Aluminiumeffektpigmente gemäß Beispiel 3b wurden im Coil Coating-Verfahren eingesetzt. Es wurden 8,0 g Aluminiumeffektpigmentpaste und 8,0 g Solvesso 150 mit einem Spatel gut dispergiert bis die Mischung stippenfrei war. Anschließend wurden 84,0 g Polyvinylidenfluorid zugeben und gerührt, bevor mit 5,0 g Solvesso 150 verdünnt wurde. Anschließend wurde 3 Minuten mit dem Zahnkranzrührer bei 500 U/min gerührt. Die Viskosität betrug 100"± 10 im DIN4 - Becher.

Diese Lackansätze wurde auf Alcan - Aluminiumblechen DIN A4 (Nr. 11) mit einer Spiralrakel abgezogen. Die Bleche wurden sofort für 15 sec. bzw. 95 sec. in einen 280° heißen Ofen überführt. Danach wurde das Blech im Wasserbad (RT) abgeschreckt. Nach frühestens 24 h wurde die Differenz der Farbwerte des 15 sec. und des 95 sec. erhitzten Bleches mittels eines Minolta Spectrophotometer CM-700d ermittelt.

Tabelle A5-2: Anwendungsbeispiel Coil-Coating - Spektrometrie

| Beispiel | Pigment | Lack | Farbwerte | |
|---|---|---|---|---|
| | | | $\Delta$a | $\Delta$b |
| **AVB 5.7** | gemäß VB 3a nasschemisch oxidierte Aluminiumeffektpigmente ohne Siliziumoxidbeschichtung | PE-Lack | -0,48 | -0,87 |
| **AVB 5.8** | VB 3a | PE-Lack | -0,45 | -0,73 |
| **AB 5.9** | Beispiel 3b | PE-Lack | -0,18 | -0,50 |
| AB: Anwenaungsbeispiel AVB: Anwendungsvergleichsbeispiel | | | | |

Die nasschemisch oxidierten Aluminiumeffektpigmente mit und ohne Siliziumoxidbeschichtung zeigen somit eine deutliche Veränderung der Farbwerte in Richtung grün (negativer $\Delta$a-Wert) und blau (negativer $\Delta$b-Wert). Die erfindungsgemäßen Pigmente zeigen hingegen bei der großen thermischen Belastung eine deutlich höhere Farbstabilität.

**Patentansprüche**

1. Nasschemisch oxidierte Aluminiumeffektpigmente, wobei die nasschemisch oxidierten Aluminiumeffektpigmente wenigstens eine von Aluminiumoxid verschiedene Metalloxidschicht und wenigstens eine umhüllende nichtreaktive organische Polymerschicht aufweisen und wobei das Gewichtsverhältnis des als Metalloxidschicht aufgebrachten von Aluminiumoxid verschiedenen Metalloxids zu der nasschemisch erzeugten Aluminiumoxidschicht in einem Bereich von 1:1 bis 1:40, insbesondere in einem Bereich von 1:2 bis 1:25, liegt, wobei die Dicke der nasschemisch erzeugten Aluminiumoxidschicht in einem Bereich von 30 nm bis 300 nm liegt.

2. Nasschemisch oxidierte Aluminiumeffektpigmente gemäß Anspruch1, wobei Summe der Gehalte der wenigstens einen von Aluminiumoxid verschiedenen Metalloxidschicht und der wenigstens einen nichtreaktiven organischen Polymerschicht in einem Bereich von 10 bis 50 Gew.-%, bezogen auf das Gewicht des unbeschichteten, nasschemisch oxidierten Aluminiumeffektpigments, liegt und das Gewichtsverhältnis der wenigstens einen von Aluminiumoxid verschiedenen Metalloxidschicht zur wenigstens einen nichtreaktiven organischen Polymerschicht in einem Bereich von 1:2 bis 1:20 liegt.

3. Nasschemisch oxidierte Aluminiumeffektpigmente gemäß Anspruch1, wobei die Summe der Gehalte der wenigstens einen von Aluminiumoxid verschiedenen Metalloxidschicht und der wenigstens einen nichtreaktiven organischen Polymerschicht in einem Bereich von 13 bis 40 Gew.-%, bezogen auf das Gewicht des unbeschichteten, nasschemisch oxidierten Aluminiumeffektpigments, liegt und das Gewichtsverhältnis der wenigstens einen von Aluminiumoxid verschiedenen Metalloxidschicht zur wenigstens einen nichtreaktiven organischen Polymerschicht in einem Bereich von 1:2,2 bis 1:17, liegt.

4. Nasschemisch oxidierte Aluminiumeffektpigmente gemäß einem der vorhergehenden Ansprüche, wobei der Gehalt an elementarem Aluminium höchstens 87 Gew.-% beträgt, bezogen auf das Gewicht der unbeschichteten, nasschemisch oxidierten Aluminiumeffektpigmente.

5. Nasschemisch oxidierte Aluminiumeffektpigmente gemäß einem der vorhergehenden Ansprüche, wobei der Gewichtsanteil der wenigstens einen von Aluminiumoxid verschiedenen Metalloxidschicht in einem Bereich von 0,8 bis 20 Gew.-% liegt, bezogen auf das Gewicht der unbeschichteten, nasschemisch oxidierten Aluminiumeffektpigmente.

6. Nasschemisch oxidierte Aluminiumeffektpigmente gemäß einem der vorhergehenden Ansprüche, wobei die wenigstens eine von Aluminiumoxid verschiedene Metalloxidschicht im Wesentlichen aus Metalloxid besteht, das ausgewählt wird aus der Gruppe bestehend aus Siliziumoxid, Boroxid, Zirkoniumoxid, Ceroxid, Eisenoxid, Titanoxid, Chromoxid, Zinnoxid, Molybdänoxid, deren Oxidhydraten, deren Hydroxiden sowie Mischungen davon.

7. Nasschemisch oxidierte Aluminiumeffektpigmente gemäß einem der vorhergehenden Ansprüche, wobei die wenigstens eine von Aluminiumoxid verschiedene Metalloxidschicht im Wesentlichen aus Siliziumoxid besteht.

8. Nasschemisch oxidierte Aluminiumeffektpigmente gemäß einem der vorhergehenden Ansprüche, wobei der Gewichtsanteil der wenigstens einen nichtreaktiven organischen Polymerschicht in einem Bereich von 8 bis 40 Gew.-% liegt, bezogen auf das Gewicht des unbeschichteten, nasschemisch oxidierten Aluminiumeffektpigments.

9. Nasschemisch oxidierte Aluminiumeffektpigmente gemäß einem der vorhergehenden Ansprüche, wobei die wenigstens eine nichtreaktive organische Polymerschicht im Wesentlichen aus organischem Polymer besteht, das ausgewählt wird aus der Gruppe bestehend aus Polyacrylat, Polymethacrylat, Polyacrylamid, Polyacrylnitril, Polyvinylchlorid, Polyvinylacetat, Polyamid, Polyalken, Polydien, Polyalkin, Polyalkylenglykol, Epoxidharz, Polyester, Polyether, Polyol, Polyurethan, Polycarbonat, Polyethylenterephthalat und Mischungen davon.

10. Nasschemisch oxidierte Aluminiumeffektpigmente gemäß einem der vorhergehenden Ansprüche, wobei zusätzlich zur wenigstens einen von Aluminiumoxid verschiedenen Metalloxidschicht eine hochbrechende Metallchalkogenidschicht vorhanden ist.

11. Verfahren zur Herstellung von beschichteten naßchemisch oxidierten Aluminiumeffektpigmenten, wobei das Verfahren folgende Schritte umfasst:

    (1) Nasschemisches Oxidieren von Aluminiumeffektpigmenten,
    (2) Beschichten der in Schritt (1) erhaltenen nasschemisch oxidierten Aluminiumeffektpigmente mit von Aluminiumoxid verschiedenem Metalloxid, wobei das Gewichtsverhältnis des als wenigstens eine Metalloxidschicht aufgebrachten Metalloxids zu der in Schritt (1) nasschemisch erzeugten Aluminiumoxidschicht in einem Bereich von 1:1 bis 1:40 liegt, wobei die Dicke der nasschemisch erzeugten Aluminiumoxidschicht in einem Bereich von 30 nm bis 300 nm liegt,
    (3) Beschichten der in Schritt (2) erhaltenen metalloxidbeschichteten nasschemisch oxidierten Aluminiumeffektpigmente mit wenigstens einer umhüllenden nichtreaktiven organischen Polymerschicht.

12. Verfahren gemäß Anspruch 11, wobei in Schritt (2) das Metalloxid mit einem Sol-Gel-Prozess aufgebracht wird.

13. Verfahren gemäß Anspruch 11 oder 12, wobei das nasschemische Oxidieren bei pH 7 bis 12 mit einem Gemisch von Wasser und einem oder mehreren wassermischbaren Lösungsmitteln durchgeführt wird.

14. Pigmente gemäß einem der Ansprüche 1 bis 10, wobei diese nach einem Verfahren gemäß einem der Ansprüche 11 bis 14 hergestellt worden sind.

15. Verwendung der Pigmente gemäß einem der Ansprüche 1 bis 10 oder 14 in einem Kunststoff, in einer Kosmetik oder in einem Beschichtungsmittel, insbesondere einer Farbe, einem Lack, einem Pulverlack oder einer Druckfarbe.

16. Beschichtungsmittel, das Pigmente gemäß einem der Ansprüche 1 bis 10 oder 14 enthält.

**Claims**

1.  Wet-chemically oxidised aluminium effect pigments, wherein the wet-chemically oxidised aluminium effect pigments comprise at least one metal oxide layer that is different from aluminium oxide and at least one enveloping non-reactive organic polymer layer and wherein the weight ratio of the metal oxide different from aluminium oxide applied as a metal oxide layer to the wet-chemically produced aluminium oxide layer is within a range of from 1:1 to 1:40, in particular within a range of from 1:2 to 1:25, and the thickness of the wet-chemically produced aluminium oxide layer is within in a range of from 30 nm to 300 nm.

2.  Wet-chemically oxidised aluminium effect pigments as claimed in claim 1, wherein the sum of the contents of the at least one metal oxide layer that is different from aluminium oxide and the at least one non-reactive organic polymer layer is within a range of 10 to 50% by weight relative to the weight of the uncoated wet-chemically oxidised aluminium effect pigment and the weight ratio of the at least one metal oxide layer that is different from aluminium oxide to the at least one non-reactive organic polymer layer is within a range of from 1:2 to 1:20.

3.  Wet-chemically oxidised aluminium effect pigments as claimed in claim 1, wherein the sum of the contents of the at least one metal oxide layer that is different from aluminium oxide and the at least one non-reactive organic polymer layer is within a range of from 13 to 40% by weight relative to the weight of the uncoated wet-chemically oxidised aluminium effect pigment and the weight ratio of the at least one metal oxide layer that is different from aluminium oxide to the at least one non-reactive organic polymer layer is within a range of from 1:2.2 to 1:17.

4.  Wet-chemically oxidised aluminium effect pigments as claimed in one of the preceding claims, wherein the content of elemental aluminium is at most 87% by weight relative to the weight of the uncoated wet-chemically oxidised aluminium effect pigments.

5.  Wet-chemically oxidised aluminium effect pigments as claimed in one of the preceding claims, wherein the proportion by weight of the at least one metal oxide layer that is different from aluminium oxide is within a range of from 0.8 to 20% by weight relative to the weight of the uncoated wet-chemically oxidised aluminium effect pigments.

6.  Wet-chemically oxidised aluminium effect pigments as claimed in one of the preceding claims, wherein the at least one metal oxide layer that is different from aluminium oxide substantially comprises metal oxide selected from the group consisting of silicon oxide, boron oxide, zirconium oxide, cerium oxide, iron oxide, titanium oxide, chromium oxide, tin oxide, molybdenum oxide, oxide hydrates thereof, hydroxides thereof and mixtures thereof.

7.  Wet-chemically oxidised aluminium effect pigments as claimed in one of the preceding claims, wherein the at least one metal oxide layer that is different from aluminium oxide substantially comprises silicon oxide.

8.  Wet-chemically oxidised aluminium effect pigments as claimed in one of the preceding claims, wherein the proportion by weight of the at least one non-reactive organic polymer layer is within a range of from 8 to 40 % by weight relative to the weight of the uncoated wet-chemically oxidised aluminium effect pigment.

9.  Wet-chemically oxidised aluminium effect pigments as claimed in one of the preceding claims, wherein the at least one non-reactive organic polymer layer substantially comprises organic polymer selected from the group consisting of polyacrylate, polymethacrylate, polyacrylamide, polyacrylonitrile, polyvinyl chloride, polyvinyl acetate, polyamide, polyalkene, polydiene, polyalkyne, polyalkylene glycol, epoxy resin, polyester, polyether, polyol, polyurethane, polycarbonate, polyethylene terephthalate and mixtures thereof.

10. Wet-chemically oxidised aluminium effect pigments as claimed in one of the preceding claims, wherein in addition to the at least one metal oxide layer that is different from aluminium oxide, a high refractive index metal chalcogenide layer is provided.

11. Method for producing coated, wet-chemically oxidised aluminium effect pigments, the method comprising the following steps:

    (1) wet-chemically oxidising aluminium effect pigments,
    (2) coating the wet-chemically oxidised aluminium effect pigments obtained in step (1) with metal oxide that is different from aluminium oxide, the weight ratio of the metal oxide applied as at least one metal oxide layer to the wet-chemically produced aluminium oxide layer from step (1) being within a range of from 1:1 to 1:40 and the thickness of the wet-chemically produced aluminium oxide layer is within a range of from 30 nm to 300 nm,
(3) coating the metal oxide coated, wet-chemically oxidised aluminium effect pigments obtained in step (2) with at least one enveloping non-reactive organic polymer layer.

12. Method as claimed in claim 11, wherein the metal oxide is applied by a sol-gel process in step (2).

13. Method as claimed in claim 11 or 12, wherein the wet-chemical oxidation is implemented at pH 7 to 12 with a mixture of water and one or more water-miscible solvents.

14. Pigments as claimed in one of claims 1 to 10, these being produced by a method as claimed in one of claims 11 to 14.

15. Use of the pigments as claimed in one of claims 1 to 10 or 14 in a plastic, in a cosmetic product or in a coating compound, in particular a paint, a varnish, a powder coating or a printing ink.

16. Coating compound which contains pigments as claimed in one of claims 1 to 10 or 14.


**Revendications**

1. Pigments à effets d'aluminium chimiquement oxydés par voie humide, dans lesquels les pigments à effets d'aluminium chimiquement oxydés par voie humide présentent au moins une couche d'un oxyde métallique différent de l'oxyde d'aluminium et au moins une couche polymère organique non réactive enveloppante, et le rapport en poids de l'oxyde métallique différent de l'oxyde d'aluminium, appliqué sous forme d'une couche d'un oxyde métallique, à la couche d'oxyde d'aluminium chimiquement produite par vois humide, étant compris dans la plage de 1:1 à 1:40, en particulier dans la plage de 1:2 à 1:25, l'épaisseur de la couche d'oxyde d'aluminium chimiquement produite par voie humide étant comprise dans la plage de 30 nm à 300 nm.

2. Pigments à effets d'aluminium chimiquement oxydés par voie humide selon la revendication 1, dans lesquels la somme des teneurs en l'au moins une couche d'un oxyde métallique différent de l'oxyde d'aluminium et en l'au moins une couche polymère organique non réactive est comprise dans la plage de 10 à 50 % en poids, par rapport au poids du pigment à effets d'aluminium chimiquement oxydé chimiquement oxydé par voie humide et non revêtu et le rapport en poids de l'au moins une couche d'un oxyde métallique différent de l'oxyde d'aluminium à l'au moins une couche polymère organique non réactive est compris dans la plage de 1:2 à 1:20.

3. Pigments à effets d'aluminium chimiquement oxydés par voie humide selon la revendication 1, dans lesquels la somme des teneurs en l'au moins une couche d'un oxyde métallique différent de l'oxyde d'aluminium et en l'au moins une couche polymère organique non réactive est comprise dans la plage de 13 à 40 % en poids par rapport au poids du pigment à effets d'aluminium chimiquement oxydés par voie humide et non revêtus, et le rapport en poids de l'au moins une couche d'un oxyde métallique différent de l'oxyde d'aluminium à l'au moins une couche polymère organique non réactive est compris dans la plage de 1:2,2 à 1:17.

4. Pigments à effets d'aluminium chimiquement oxydés par voie humide selon l'une des revendications précédentes, dans lesquels la teneur en oxyde d'aluminium est d'au plus 87 % en poids par rapport au poids des pigments à effets d'aluminium chimiquement oxydés par voie humide et non revêtus.

5. Pigments à effets d'aluminium chimiquement oxydés par voie humide selon l'une des revendications précédentes, dans lesquels la proportion pondérale de l'au moins une couche d'un oxyde métallique différent de l'oxyde d'aluminium est comprise dans la plage de 0,8 à 20 % en poids par rapport au poids des pigments à effets d'aluminium chimiquement oxydés par voie humide et non revêtus.

6. Pigments à effets d'aluminium chimiquement oxydés par voie humide selon l'une des revendications précédentes, dans lesquels l'au moins une couche d'un oxyde métallique différent de l'oxyde d'aluminium est pour l'essentiel constituée d'un oxyde métallique qui est choisi dans le groupe consistant en l'oxyde de silicium, l'oxyde de bore, l'oxyde de zirconium, l'oxyde de cérium, l'oxyde de fer, l'oxyde de titane, l'oxyde de chrome, l'oxyde d'étain, l'oxyde de molybdène, les oxyhydrates de ceux-ci, les hydroxydes de ceux-ci et les mélanges de ceux-ci.

7. Pigments à effets d'aluminium chimiquement oxydés par voie humide selon l'une des revendications précédentes, dans lesquels l'au moins une couche d'un oxyde métallique différent de l'oxyde d'aluminium est pour l'essentiel constituée d'oxyde de silicium.

8. Pigments à effets d'aluminium chimiquement oxydés par voie humide selon l'une des revendications précédentes, dans lesquels la proportion pondérale de l'au moins une couche polymère organique non réactive est comprise dans la plage de 8 à 40 % en poids par rapport au poids du pigment à effets d'aluminium chimiquement oxydés par voie humide et non revêtu.

9. Pigments à effets d'aluminium chimiquement oxydés par voie humide selon l'une des revendications précédentes, dans lesquels l'au moins une couche polymère organique non réactive est pour l'essentiel constituée d'un polymère organique qui est choisi dans le groupe consistant en un polyacrylate, un polyméthacrylate, un polyacrylamide, un polyacrylonitrile, un poly(chlorure de vinyle), un poly(acétate de vinyle), un polyamide, un polyalcène, un polydiène, un polyalcyne, un polyalkylèneglycol, une résine époxyde, un polyester, un polyéther, un polyol, un polyuréthanne, un polycarbonate, un poly(téréphtalate d'éthylène) et les mélanges de ceux-ci.

10. Pigments à effets d'aluminium chimiquement oxydés par voie humide selon l'une des revendications précédentes, dans lesquels une couche d'un chalcogénure métallique à grand indice de réfraction est présente en plus de l'au moins une couche d'un oxyde métallique différent de l'oxyde d'aluminium.

11. Procédé de fabrication de pigments à effets d'aluminium chimiquement oxydés par voie humide et revêtus, le procédé comprenant les étapes suivantes :

(1) oxydation chimique par voie humide des pigments à effets d'aluminium,
(2) revêtement des pigments à effets d'aluminium chimiquement oxydés par voie humide, obtenus dans l'étape (1), avec un oxyde métallique différent de l'oxyde d'aluminium, le rapport en poids de l'au moins un oxyde métallique appliqué en tant qu'au moins une couche d'un oxyde métallique à la couche d'oxyde d'aluminium chimiquement produite par voie humide dans l'étape (1) étant compris dans la plage de 1:1 à 1:40, l'épaisseur de la couche d'oxyde d'aluminium chimiquement produite par voie humide étant comprise dans la plage de 30 nm à 300 nm,
(3) revêtement des pigments à effets d'aluminium chimiquement oxydés par voie humide, revêtus d'un oxyde métallique, obtenus dans l'étape (2), avec au moins une couche polymère organique non réactive enveloppante.

12. Procédé selon la revendication 11, dans lequel l'oxyde métallique est dans l'étape (2) appliqué par un procédé sol-gel.

13. Procédé selon la revendication 11 ou 12, dans lequel l'oxydation chimique par voie humide est mise en œuvre à pH 7 à 12 avec un mélange d'eau et d'un ou plusieurs solvants miscibles à l'eau.

14. Pigments selon l'une des revendications 1 à 10, ces derniers ayant été fabriqués par un procédé selon l'une des revendications 11 à 14.

15. Utilisation des pigments selon l'une des revendications 1 à 10 ou 14 dans un plastique, dans un produit cosmétique ou dans une composition de revêtement, en particulier une peinture, un vernis, une peinture en poudre ou une encre d'impression.

16. Composition de revêtement qui contient des pigments selon l'une des revendications 1 à 10 ou 14.

Abbildung 1:

Abbildung 2:

Abbildung 3:

Abbildung 4:

Abbildung 5:

Abbildung 6:

Abbildung 7:

Abbildung 8:

Abbildung 9:

Abbildung 10:

1µm

Abbildung 11:

1µm

Abbildung 12:

1µm

Abbildung 13:

1µm

Abbildung 14:

1µm

Abbildung 15:

1µm

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5964936 A
- US 5931996 A
- US 20070104663 A1
- US 20080249209 A1
- US 20090264575 A1
- US 6761762 B1
- US 5332767 A

- US 20090117281 A1
- US 20100152355 A1
- US 20030209169 A1
- WO 2005063897 A2
- WO 2008095697 A1
- WO 2009152941 A2
- WO 2007115675 A2